# EUROPEAN PATENT APPLICATION

(11) **EP 4 706 646 A1**
(43) Date of publication of application: **11.03.2026**
(21) Application number: 24814575.7
(22) Date of filing: 31.05.2024
(51) Int. Cl.: A61K 31/00, A61K 31/7088, A61K 31/70, A61K 45/00, C07D 487/04, C07F 9/6561, A61P 31/14, A61K 31/53

(54) **NOVEL DOUBLE-STRANDED SIRNA, CONJUGATE THEREOF, AND USE THEREOF**

(30) Priority: 01.06.2023 CN 202310641202
(71) Applicant: Sunshine Lake Pharma Co., Ltd., Dongguan, Guangdong 523000 (CN)
(72) Inventor: REN, Qingyun, Dongguan, Guangdong 523871 (CN); ZOU, Zhifu, Dongguan, Guangdong 523871 (CN); CHEN, Yunfu, Dongguan, Guangdong 523871 (CN); CHEN, Huimeng, Dongguan, Guangdong 523871 (CN); WANG, Pu, Dongguan, Guangdong 523871 (CN); ZHANG, Yingjun, Dongguan, Guangdong 523871 (CN)
(74) Representative: Becker Kurig & Partner Patentanwälte mbB
(86) International application number: PCT/CN2024/096557
(87) International publication number: WO 2024/245381

(57) **Abstract**

The present invention provides a novel double-stranded siRNA, conjugate thereof, and use thereof. The present invention provides a double-stranded siRNA, a conjugate thereof or a salt thereof, and also relates to use of the double-stranded siRNA and the conjugate thereof in preparing a medicament for treating and/or preventing hepatitis B. In addition, the present invention also relates to a novel compound, and a use of the compound in raw materials for solid-phase synthesis of DNA nucleotides, raw materials for synthesis of siRNA drugs, siRNA drug research, gene function research and/or screening of whole gene libraries, especially the use of the compound as a raw material for synthesis of the double-stranded siRNA drug described in the present invention. Furthermore, the present invention also relates to a novel nucleotide residue and its use in siRNA drug research, as a raw material for the synthesis of gene function research, and/or as a raw material for screening of whole gene libraries, as well as as an embedding group in oligonucleotides.

## Description

### FIELD OF THE INVENTION

The present invention belongs to the field of small nucleic acid drugs. The purpose of the present invention is to provide a novel double-stranded siRNA, a conjugate thereof and a use thereof. The present invention also provides use of the double-stranded siRNA and conjugate thereof in preparing a drug for treating and/or preventing hepatitis B. In addition, the present invention also relates to a novel compound, and a use of the compound in raw materials for solid-phase synthesis of DNA nucleotides, raw materials for synthesis of oligonucleotide drugs, raw materials for synthesis of siRNA drugs, siRNA drug research, gene function research and/or screening of whole gene libraries, especially the use of the compound as a raw material for synthesis of the double-stranded siRNA drug described in the present invention. Furthermore, the present invention also relates to a novel nucleotide residue and its use in siRNA drug research, as a raw material for the synthesis of gene function research, and/or as a raw material for screening of whole gene libraries, as well as as an embedding group in oligonucleotides.

### BACKGROUND ART

Viral hepatitis B (abbreviated as hepatitis type B or hepatitis B) is a serious infectious disease that threatens the world, especially China. Currently, the two major types of hepatitis B treatment drugs recognized worldwide are interferon and nucleoside analogs. However, these two types of drugs have many disadvantages, such as easy development of drug resistance or limited use after use. For example, interferon is prone to adverse reactions, and nucleoside drugs have drug resistance and recurrence after discontinuation of the drug. Therefore, if the gene expression of the virus can be silenced at the genetic level, blocking the generation and replication of HBV, thereby fundamentally reducing viral replication and infection of liver cells, it will undoubtedly be the most ideal treatment for hepatitis B. Small interfering ribonucleic acid, i.e., small interfering RNA (siRNA) or short interfering RNA, can based on the RNA interference (RNAi) mechanism, inhibit or block the expression of any target gene of interest (such as genes that cause diseases like cancer) in a sequence-specific manner, thereby achieving therapeutic purposes.

RNA interference (RNAi) is a phenomenon in which double-stranded RNA (dsRNA) molecules shut down or silence the expression of homologous genes at the mRNA level. RNA interference technology, also known as gene knock-down or gene silencing, is a typical post-transcriptional gene regulation method, also known as post-transcriptional gene silencing (PTGS). The earliest report on RNA interference appeared in 1990, when two different research groups simultaneously reported the phenomenon of RNA interference in transgenic plants. Subsequently, RNA interference was observed in almost all eukaryotic organisms, including nematodes, fruit flies, zebrafish and mice. In 1999, Hamilton and Baulcombe detected RNA fragments of 21-25 nucleotides in length in plants undergoing RNA interference. These RNA fragments were shown to be necessary for RNA interference and were called small interfering nucleic acids (siRNAs). Double-stranded siRNA forms an RNA-induced silencing complex (RISC) with cell-derived related enzymes and proteins. During RNA interference, the sense strand in the double-stranded siRNA is excluded from the complex, and the antisense strand guides RISC to bind to the homologous site of the target mRNA. The target mRNA is then degraded by RNase III in the complex, thereby shutting down the expression of the target gene.

### SUMMARY

The present invention aims to provide a novel double-stranded siRNA, a conjugate thereof, a salt thereof and a use thereof, and specifically relates to the use of the double-stranded siRNA and conjugate thereof in preparing a drug for treating and/or preventing hepatitis B. In addition, the present invention also relates to a novel compound, and a use of the compound in raw materials for solid-phase synthesis of DNA nucleotides, raw materials for synthesis of siRNA drugs, siRNA drug research, gene function research and/or screening of whole gene libraries, especially the use of the compound as a raw material for synthesis of the double-stranded siRNA drug described in the present invention. Furthermore, the present invention also relates to a novel nucleotide residue and its use in siRNA drug research, as a raw material for the synthesis of gene function research, and/or as a raw material for screening of whole gene libraries, as well as as an embedding group in oligonucleotides. The double-stranded siRNA and conjugate thereof of the present invention have higher gene expression inhibition activity and/or lower toxicity against HBV.

In one aspect, the present invention relates to a double-stranded siRNA, a conjugate thereof, or a salt thereof, wherein, the double-stranded siRNA comprises a sense strand and an antisense strand, and the double-stranded siRNA comprisesone or more Y, the Y is 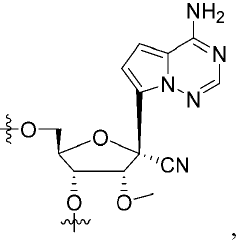 wherein each of nucleotides in the double-stranded siRNA and Y is independently modified or unmodified. Optionally, the Y can be at any position in the double-stranded siRNA.

In some embodiments of the double-stranded siRNA, conjugate or salt thereof of the present invention, wherein, the double-stranded siRNA comprises 1, 2, 3, 4, 5, 6, 7, 8, 9 or 10 Y.

In some embodiments of the double-stranded siRNA, conjugate or salt thereof of the present invention, wherein, the double-stranded siRNA comprises 1, 2, 3, 4 or 5 Y.

In some embodiments of the double-stranded siRNA, conjugate or salt thereof of the present invention, wherein, the antisense strand comprises a sequence of 5'-UGUGAAGCGAAGUGCACACUU-3' (SEQ ID NO:40), or a sequence in which one or more nucleotide residues are replaced by Y in the sequence of SEQ ID NO:40; wherein each of nucleotides and Y in the antisense strand is independently modified or unmodified, and the length of the antisense strand is not more than 23 nucleotides, wherein Y is as defined herein. Optionally, the Y substitution occurs at any position in the sequence of SEQ ID NO:40.

In some embodiments of the double-stranded siRNA, conjugate or salt thereof of the present invention, wherein, the sense strand comprises a sequence of 5'-GUGUGCACUUCGCUUCACA-3' (SEQ ID NO:20), or a sequence in which one or more nucleotide residues are replaced by Y in the sequence of SEQ ID NO:20, wherein each of nucleotides and Y in the sense strand is independently modified or unmodified, and the length of the sense strand is not more than 23 nucleotides. Optionally, the Y substitution occurs at any position in the sequence of SEQ ID NO:20.

In some embodiments of the double-stranded siRNA, conjugate or salt thereof of the present invention, more than 70%, 75%, 80%, 85%, 90% or 95% of the nucleotides and Y in the sense strand or the antisense strand of the double-stranded siRNA are modified.

In some embodiments of the double-stranded siRNA, conjugate or salt thereof of the present invention, more than 70%, 75%, 80%, 85%, 90% or 95% of the nucleotides and Y in the sense strand and the antisense strand of the double-stranded siRNA are modified.

In some embodiments of the double-stranded siRNA, conjugate or salt thereof of the present invention, all the nucleotides and Y in the double-stranded siRNA are modified.

In some embodiments of the double-stranded siRNA, conjugate or salt thereof of the present invention, the modification is selected from at least one of the following: 2'-methoxy modification, 2'-methoxyethyl modification, 2'-fluoro modification, phosphorothioate linkage, 2'-deoxy modification, 2'-amino modification, locked nucleic acid modification, unlocked nucleic acid modification, ethylene glycol nucleic acid modification and 5'-vinyl phosphate ester modification.

In some embodiments of the double-stranded siRNA, conjugate or salt thereof of the present invention, wherein, the sense strand comprises a sequence in which 0, 1, 2, 3, 4 or 5 nucleotide residues are replaced with Y in the sequence of SEQ ID NO:20, wherein each of nucleotides and Y in the sense strand are independently modified or unmodified.

In some embodiments of the double-stranded siRNA, conjugate or salt thereof of the present invention, wherein, the sense strand comprises a sequence in which one nucleotide residue is replaced with Y in the sequence of SEQ ID NO:20.

In some embodiments of the double-stranded siRNA, conjugate or salt thereof of the present invention, wherein, the antisense strand comprises a sequence in which 0, 1, 2, 3, 4 or 5 nucleotide residues are replaced with Y in the sequence of SEQ ID NO:40, wherein each ofnucleotides and Y in the antisense strand are independently modified or unmodified. In some embodiments of the double-stranded siRNA, conjugate or salt thereof of the present invention, wherein, the antisense strand comprises a sequence in which one nucleotide residue is replaced with Y in the sequence of SEQ ID NO:40.

In some embodiments of the double-stranded siRNA, conjugate or salt thereof of the present invention, wherein, the Y substitution occurs at positions 1 to 19 from the 5' end of the sequence of SEQ ID NO: 20 (i.e., the Y substitution optionally occurs at position 1, 2, 3, 4, 5, 6, 7, 8, 9, 10, 11, 12, 13, 14, 15, 16, 17, 18 or 19 from the 5' end of the sequence of SEQ ID NO: 20).

In some embodiments of the double-stranded siRNA, conjugate or salt thereof of the present invention, wherein, the Y substitution occurs at positions 1 to 21 from the 5' end of the sequence of SEQ ID NO: 40 (i.e., the Y substitution optionally occurs at position 1, 2, 3, 4, 5, 6, 7, 8, 9, 10, 11, 12, 13, 14, 15, 16, 17, 18, 19, 20 or 21 from the 5' end of the sequence of SEQ ID NO: 40).

In some embodiments of the double-stranded siRNA, conjugate or salt thereof of the present invention, wherein, the sense strand comprises a sequence in which 0, 1, 2, 3, 4 or 5 nucleotide residues are substituted with Y in the sequence of 5'-g•u•guGcACUucgcuucaca-3' (SEQ ID NO: 19), wherein each of nucleotides and Y in the sense strand is independently modified or unmodified, and the Y substitution occurs at positions 1 to 19 from the 5' end of the sequence of SEQ ID NO: 19 (i.e., the Y substitution optionally occurs at position 1, 2, 3, 4, 5, 6, 7, 8, 9, 10, 11, 12, 13, 14, 15, 16, 17, 18 or 19 form the 5' end of the sequence of SEQ ID NO: 19), and the length of the sense strand is not more than 23 nucleotides.

In some embodiments of the double-stranded siRNA, conjugate or salt thereof of the present invention, wherein, the antisense strand comprises a sequence in which 0, 1, 2, 3, 4 or 5 nucleotide residues are substituted with Y in the sequence of 5'-u•G•ugaAgCGaaguGcAcac•u•u-3' (SEQ ID NO: 36), wherein each of nucleotides and Y in the antisense strand is independently modified or unmodified, and the Y substitution occurs at positions 1 to 21 from the 5' end of the antisense strand of SEQ ID NO: 36 (i.e., the Y substitution optionally occurs at position 1, 2, 3, 4, 5, 6, 7, 8, 9, 10, 11, 12, 13, 14, 15, 16, 17, 18 or 19 from the 5' end of the sequence of SEQ ID NO: 36), and the length of the antisense strand is not more than 23 nucleotides.

In some embodiments of the double-stranded siRNA, conjugate or salt thereof of the present invention, wherein, the antisense strand comprises a sequence in which 0, 1, 2, 3, 4 or 5 nucleotide residues are substituted with Y in the sequence of 5'-u•G•uga(Agn)gCGaaguGcAcac•u•u-3' (SEQ ID NO: 33), wherein each of nucleotides and Y in the antisense strand is independently modified or unmodified, and the Y substitution occurs at positions 1 to 21 from the 5' end of the sequence of SEQ ID NO: 33 (i.e., the Y substitution optionally occurs at position 1, 2, 3, 4, 5, 6, 7, 8, 9, 10, 11, 12, 13, 14, 15, 16, 17, 18 or 19 from the 5' end of the sequence of SEQ ID NO: 33), and the length of the antisense strand is not more than 23 nucleotides.

Unless otherwise specified, in the context of the present invention, capital letters C, G, U and A represent the base composition of natural nucleotides; lowercase letters c, g, u and a respectively represent that the 2-position of the ribose on the nucleotide represented by the corresponding capital letter is modified by methoxy; an underline under a capital letter represents that the 2-position of the ribose on the nucleotide represented by the letter is modified by fluorine; Agn is adenosine-ethylene glycol nucleic acid; the middle dot "•" represents that there is phosphorothioate linkage between two nucleotide residues adjacent to the left and right sides of the middle dot "•"; Y is

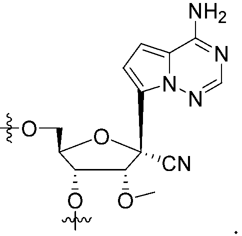

In some embodiments of the double-stranded siRNA, conjugate or salt thereof of the present invention, the sense strand comprises one of the following nucleotide sequences:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19 and SEQ ID NO:21;
the length of the sense strand is not more than 23 nucleotides.

In some embodiments of the double-stranded siRNA, conjugate or salt thereof of the present invention, the antisense strand comprises one of the following nucleotide sequences:
SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38 and SEQ ID NO:39;
the length of the antisense strand is not more than 23 nucleotides.

In some embodiments of the double-stranded siRNA, conjugate or salt thereof of the present invention, wherein, the double-stranded siRNA comprises one of the following:
A sense strand comprising the nucleotide sequence of SEQ ID NO: 15 and an antisense strand comprising the nucleotide sequence of SEQ ID NO: 25, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 25, wherein the sense strand and antisense strand are each independently not more than 23 nucleotides;
A sense strand comprising the nucleotide sequence of SEQ ID NO: 15 and an antisense strand comprising the nucleotide sequence of SEQ ID NO: 26, wherein the sense strand and antisense strand are each independently not more than 23 nucleotides;
A sense strand comprising the nucleotide sequence of SEQ ID NO: 16 and an antisense strand comprising the nucleotide sequence of SEQ ID NO: 26, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 26, wherein the sense strand and antisense strand are each independently not more than 23 nucleotides;
A sense strand comprising the nucleotide sequence of SEQ ID NO: 15 and an antisense strand comprising the nucleotide sequence of SEQ ID NO: 27, wherein the sense strand and antisense strand are each independently not more than 23 nucleotides;
A sense strand comprising the nucleotide sequence of SEQ ID NO: 16 and an antisense strand comprising the nucleotide sequence of SEQ ID NO: 27, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 27, wherein the sense strand and antisense strand are each independently not more than 23 nucleotides;
A sense strand comprising the nucleotide sequence of SEQ ID NO: 16 and an antisense strand comprising the nucleotide sequence of SEQ ID NO: 28, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 28, wherein the sense strand and antisense strand are each independently not more than 23 nucleotides; or
A sense strand comprising the nucleotide sequence of SEQ ID NO: 18 and an antisense strand comprising the nucleotide sequence of SEQ ID NO: 28, wherein the sense strand and antisense strand are each independently not more than 23 nucleotides.

In some embodiments of the double-stranded siRNA, conjugate or salt thereof of the present invention, the double-stranded siRNA or conjugate thereof is selected from one of S1 to S143, S146, S147 and S150 in Table 1 of the present specification. In some embodiments of the double-stranded siRNA, conjugate or salt thereof of the present invention, the double-stranded siRNA conjugate comprises the double-stranded siRNA and a conjugation group, that is, the double-stranded siRNA conjugate is a conjugate of the double-stranded siRNA conjugated to the conjugation group, wherein the conjugation group includes but is not limited to L-96 or DAW40007-4, and the structures of L-96 and DAW40007-4 are respectively:

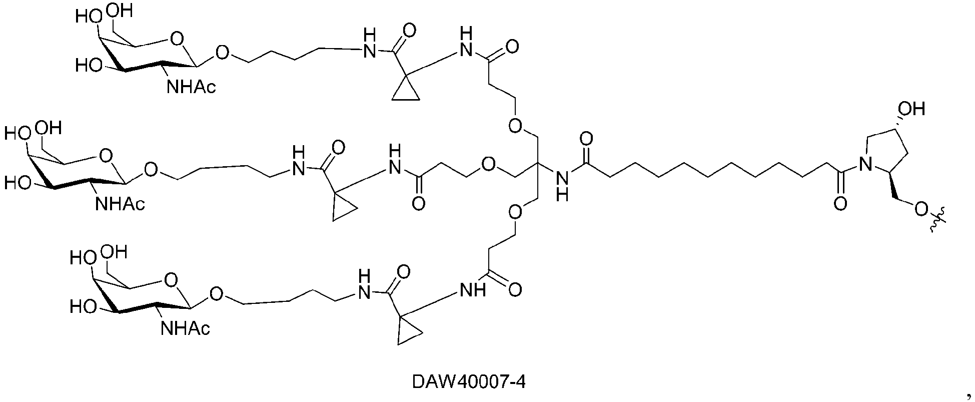

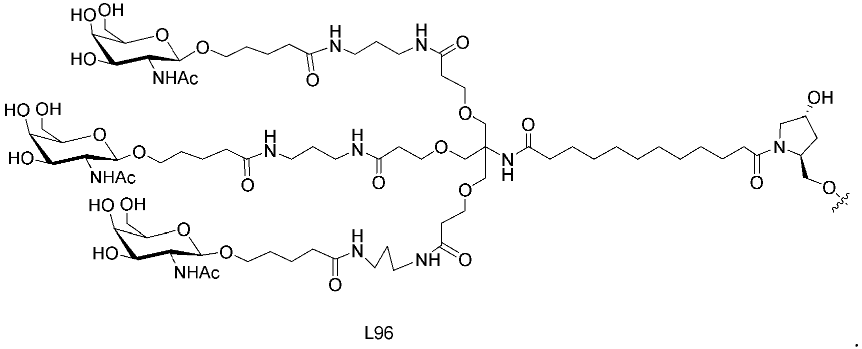

In some embodiments of the double-stranded siRNA, conjugate or salt thereof of the present invention, the conjugated group is conjugated to the 3' end or the 5' end of the sense strand of the double-stranded siRNA.

In some embodiments of the double-stranded siRNA, conjugate or salt thereof of the present invention, the conjugated group is conjugated to the 3' end of the sense strand of the double-stranded siRNA.

In some embodiments of the double-stranded siRNA, conjugate or salt thereof of the present invention, the double-stranded siRNA is linked to the conjugated group through a phosphate eater group, phosphorothioate group or a phosphate group.

In another aspect, the present invention also relates to a nucleotide residue Y having structure

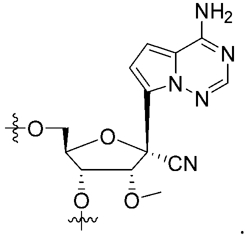

In another aspect, the present invention also relates to a compound having Formula (I) or a stereoisomer, a tautomer or an acceptable salt of the compound having Formula (I), 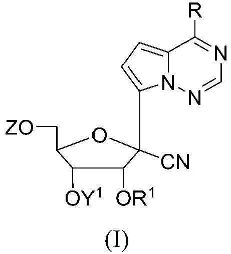
R¹ is methyl, ethyl, *n*-propyl, *i*-propyl, *n*-butyl, *i*-butyl or *tert*-butyl*,* each of the methyl, ethyl, *n*-propyl, *i*-propyl, *n*-butyl, *i*-butyl or *tert*-butyl is independently optionally substituted with 1, 2, 3 or 4 substituents selected from deuterium, hydroxyl, cyano, F, Cl, Br, I, methoxy, ethoxy, 1-propoxy and 2-propoxy;
Z and Y¹ are each independently H, deuterium, a hydroxyl protecting group, HOC(=O)(CH₂)ⱼC(=O)-, M-C(=O)(CH₂)jC(=O)-, a phosphate ester group, a phosphorothioate group, a phosphoramidite group or a hydrogen phosphate group;
M is a solid support, preferably a hydroxyl- or amino-functionalized solid support, more preferably a resin or CPG, further preferably a macroporous resin or CPG, further preferably an aminomethyl resin, a hydroxyl resin or -NHCPG;
R is -NHR² or -N=CH-NR^{a}R^{b};
R² is an amino protecting group;
each of R^{a} and R^{b} is independently H or an amino protecting group, or R^{a}, R^{b} and the nitrogen atom to which they are attached form a heterocyclic group consisting of 5-6 ring atoms, each of heterocyclic group consisting of 5-6 ring atoms is independently optionally substituted with 1, 2, 3 or 4 substituents selected from deuterium, hydroxyl, cyano, F, Cl, Br, I, methyl, ethyl, n-propyl, isopropyl, methoxy, ethoxy, 1-propoxy and 2-propoxy;
wherein, each j is independently 1, 2, 3, 4 or 5.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₁₂ alkyl, C₁₋₁₂ alkylC(=O)-, C₁₋₁₂ alkylsilyl, C₆₋₁₀ arylC₁₋₆ alkyl, HOC(=O)(CH₂)ⱼC(=O)-, M-C(=O)(CH₂)jC(=O)-, triphenylmethyl, MMTr, DMTr, 4',4',4'-trimethoxytrityl, 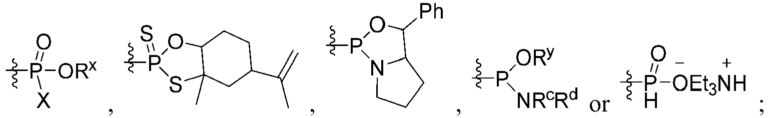 wherein, each j, M, X, R^{x}, R^{y}, R^{c} and R^{d} are as defined herein.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₁₀ alkyl, C₁₋₁₀ alkylC(=O)-, C₁₋₁₀ alkylsilyl, C₆₋₁₀ arylC₁₋₄ alkyl, HOC(=O)(CH₂)ⱼC(=O)-, M-C(=O)(CH₂)jC(=O)-, triphenylmethyl, MMTr, DMTr, 4',4',4'-trimethoxytrityl, 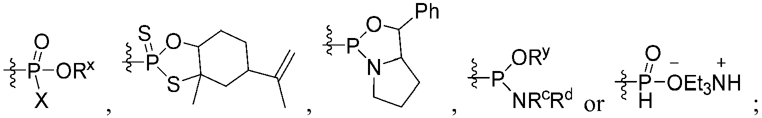 wherein, each j, M, X, R^{x}, R^{y}, R^{c} and R^{d} are as defined herein.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₈ alkyl, C₁₋₈ alkylC(=O)-, C₁₋₈ alkylsilyl, phenylC₁₋₃ alkyl, HOC(=O)(CH₂)ⱼC(=O)-, M-C(=O)(CH₂)ⱼC(=O)-, triphenylmethyl, MMTr, DMTr, 4',4',4'-trimethoxytrityl, 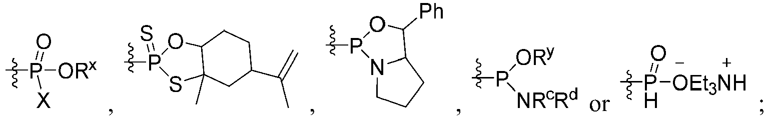 wherein, each j, M, X, R^{x}, R^{y}, R^{c} and R^{d} are as defined herein.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, C₁₋₆ alkyl, C₁₋₆ alkylC(=O)-, C₁₋₆ alkylsilyl, benzyl, phenethyl, HOC(=O)(CH₂)ⱼC(=O)-, M-C(=O)(CH₂)ⱼC(=O)-, triphenylmethyl, MMTr, DMTr, 4',4',4'-trimethoxytrityl, 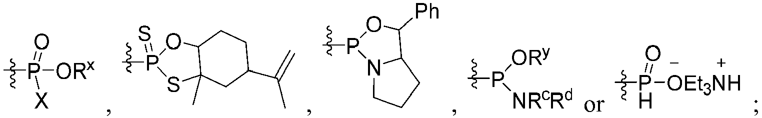 wherein, each j, M, X, R^{x}, R^{y}, R^{c} and R^{d} are as defined herein.

In some embodiments of the compounds described herein, X is Cl or Br.

In some embodiments of the compounds described herein, each of R^{x} and R^{y} is independently a hydroxy protecting group.

In some embodiments of the compounds described herein, each of R^{c} and R^{d} is independently H or an amino protecting group; or R^{c}, R^{d} and the nitrogen atom to which they are attached form a heterocyclic group consisting of 5-6 ring atoms, the heterocyclic group consisting of 5-6 ring atoms is independently optionally substituted with 1, 2, 3 or 4 substituents selected from deuterium, hydroxyl, cyano, F, Cl, Br, I, methyl, ethyl, propyl, methoxy, ethoxy, 1-propoxy and 2-propoxy.

In some embodiments of the compounds described herein, R² is 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, *tert-*butyl, 2-trimethylsilylethyl, 1,1-dimethyl-2-propenyl, 3-methyl-3-butenyl, allyl, benzyl, *p*-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl, C₁₋₆ alkoxy or R³C(=O)-, wherein, R³ is as defined herein.

In some embodiments of the compounds described herein, each of R^{a} and R^{b} is independently H, methyl, ethyl, *n*-propyl, *i*-propyl, *n*-butyl, *i*-butyl, *tert*-butyl, methoxy, ethoxy, 1-propoxy, 2-propoxy, 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2-trimethylsilylethyl, 1,1-dimethyl-2-propenyl, 3-methyl-3-butenyl, allyl, benzyl, *p*-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl or R⁴C(=O)-, or R^{a}, R^{b} and the N atom to which they are attached form a pyrrolidinyl, morpholinyl, piperidinyl or piperazinyl, each of pyrrolidinyl, morpholinyl, piperidinyl and piperazinyl is independently optionally substituted with 1, 2, 3 or 4 substituents selected from deuterium, hydroxyl, cyano, F, Cl, Br, I, methoxy, ethoxy, 1-propoxy and 2-propoxy, wherein, R⁴ is as defined herein.

In some embodiments of the compounds described herein, each of R³ and R⁴ is independently C₁₋₆ alkyl, halo C₁₋₆ alkyl, C₁₋₆ alkoxy, phenyl, halo-substituted phenyl, C₁₋₆ alkylphenyl or benzyl.

In some embodiments of the compounds described herein, each of R³ and R⁴ is independently C₁₋₄ alkyl, halo C₁₋₄ alkyl, C₁₋₄ alkoxy, phenyl, halo-substituted phenyl, C₁₋₄ alkylphenyl or benzyl.

In some embodiments of the compounds described herein, each of R³ and R⁴ is independently methyl, ethyl, *n*-propyl, isopropyl, trifluoromethyl, difluoromethyl, monofluoromethyl, trichloromethyl, dichloromethyl, monochloromethyl, 2,2,2-trichloroethyl, methoxy, ethoxy, 1-propoxy, 2-propoxy, 1-butoxy, 2-methyl-1-propoxy, 2-butoxy, *tert*-butoxy, phenyl, halogen-substituted phenyl, C₁₋₃ alkylphenyl or benzyl.

In some embodiments of the compounds described herein, each of R^{x} and R^{y} is independently benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, cyano C₁₋₆ alkyl, C₁₋₆ alkyl, C₁₋₆ alkyl C(=O)-, C₁₋₆ alkylsilyl, phenyl, halogen-substituted phenyl, benzyl or phenethyl.

In some embodiments of the compounds described herein, each of R^{x} and R^{y} is independently benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, CNCH₂-, CN(CH₂)₂-, CNCH(CH₃)₂CH₂-, CN(CH₂)₃-, CNCH(CH₃)₂CH₂CH₂-, methyl, ethyl, *n*-propyl, *i*-propyl, *n*-butyl, *i*-butyl, *tert*-butyl, C₁₋₄ alkyl C(=O)-, C₁₋₄ alkylsilyl, phenyl, halogen-substituted phenyl, benzyl or phenethyl.

In some embodiments of the compounds described herein, each of R^{c} and R^{d} is independently methyl, ethyl, *n*-propyl, *i*-propyl, *n*-butyl, *i*-butyl, *tert*-butyl, methoxy, ethoxy, 1-propoxy, 2-propoxy, 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2-trimethylsilylethyl, 1,1-dimethyl-2-propenyl, 3-methyl-3-butenyl, allyl, benzyl, *p*-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl or R⁴C(=O)-, or R^{c}, R^{d} and the N atom to which they are attached form a pyrrolidinyl, morpholinyl, piperidinyl or piperazinyl, each of pyrrolidinyl, morpholinyl, piperidinyl and piperazinyl is independently optionally substituted with 1, 2, 3 or 4 substituents selected from deuterium, hydroxyl, cyano, F, Cl, Br, I, methoxy, ethoxy, 1-propoxy and 2-propoxy.

In some embodiments of the compounds described herein, the compound of the present invention has a structure as shown in Formula (II),

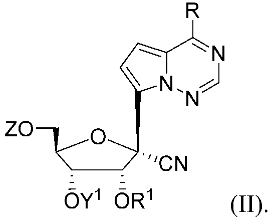

In another aspect of the present invention, the present invention also relates to a pharmaceutical composition comprising the double-stranded siRNA, conjugate or salt thereof according to the present invention, and a pharmaceutically acceptable carrier.

In another aspect of the present invention, the present invention also relates to the use of the double-stranded siRNA conjugate, conjugate thereof and the pharmaceutical composition of the present invention in the preparation of drugs for treating and/or preventing hepatitis B disease.

In another aspect of the present invention, the present invention also relates to the use of the nucleotide residues of the present invention in drug research, gene function research and/or screening of whole gene libraries for siRNA, and the structure of the nucleotide residues is:

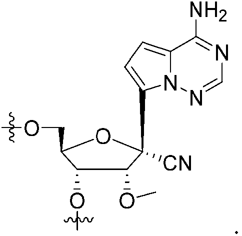

In some embodiments of the application of the nucleotide residues described in the present invention, the siRNA drug is the double-stranded siRNA drug described in the present invention.

In another aspect of the present invention, the present invention also relates to a use of the compound in raw materials for solid-phase synthesis of DNA nucleotides, raw materials for synthesis of oligonucleotide drugs, raw materials for synthesis of siRNA drugs, siRNA drug research, gene function research and/or screening of whole gene libraries.

In another aspect of the present invention, the present invention also relates to the use of the nucleotide residues described in the present invention as oligonucleotide embedding groups, wherein the oligonucleotide is a nucleotide sequence containing 10 to 50 nucleotides or nucleotide base pairs, and the oligonucleotide can inhibit or block gene expression, and the structure of the nucleotide residue is:

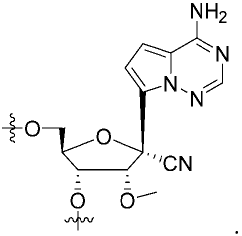

In some embodiments of the use of the nucleotide residue as an oligonucleotide embedding group in the present invention, wherein, the gene is the HBV gene.

In some embodiments of the use of the nucleotide residue as an oligonucleotide embedding group in the present invention, wherein, the oligonucleotide is siRNA.

In some embodiments of the use of the nucleotide residue as an oligonucleotide embedding group in the present invention, wherein, the siRNA comprises the sense strand and the antisense strand.

In some embodiments of the use of the nucleotide residue as an oligonucleotide embedding group in the present invention, wherein, the nucleotide residue is embedded only into the sense strand of the siRNA.

In some embodiments of the use of the nucleotide residue as an oligonucleotide embedding group in the present invention, wherein, the nucleotide residue is embedded only into the antisense strand of the siRNA.

In some embodiments of the use of the nucleotide residue as an oligonucleotide embedding group in the present invention, wherein, the nucleotide residue is embedded into the sense strand and the antisense strand of the siRNA.

In some embodiments of the use of the nucleotide residue as an oligonucleotide embedding group in the present invention, wherein, the sense strand of the siRNA comprises the sequence of SEQ ID NO: 19.

In some embodiments of the use of the nucleotide residue as an oligonucleotide embedding group in the present invention, wherein, the antisense strand of the siRNA comprises the sequence of SEQ ID NO: 36 or SEQ ID NO: 33.

### DETAILED DESCRIPTION OF THE INVENTION

### DEFINITIONS AND GENERAL TERMINOLOGY

In the present invention, the term "comprise" or "include" is an open expression, it means comprising the contents disclosed herein, but don't exclude other contents.

In the present invention, the term "small interfering RNA" or "siRNA" refers to a type of double-stranded RNA molecule with a length of 17 to 30 nucleotides, comprising a sense strand and an antisense strand. siRNA mediates targeted cleavage of RNA transcripts through the RISC pathway by forming the RNA-induced silencing complex (RISC). Specifically, siRNA directs the specific degradation of mRNA sequences through a process known as RNA interference (RNAi), inhibiting the translation of mRNA into amino acids and protein. For example, siRNA can modulate (e.g., inhibit) the expression of hepatitis B virus in cells.

In the present invention, the term "antisense strand (or guide strand)" refers to the siRNA strand that includes a region that is substantially complementary to a target sequence (eg, a sequence in hepatitis B virus mRNA). The term "sense strand (or follower strand)" refers to the siRNA strand that contains a region that is substantially complementary to the antisense strand. The antisense strand or the sense strand in the double-stranded siRNA of the present invention contains one or more (eg, 2, 3, 4 or 5) nucleotide residues Y, and the Y has the structure of the present invention.

The term "substantially complementary" refers to complete complementarity or at least partial complementarity, for example, the antisense strand is completely complementary or at least partially complementary to the target sequence. In the case of partial complementarity, mismatches can occur within internal or terminal regions of the molecule, with the most tolerated mismatches occurring within the terminal regions, e.g., within 5, 4, 3, or 2 nucleotides of the 5'- and/or 3'-end of either strand of the siRNA.

It should be noted that "at least a portion of the antisense strand is substantially complementary to the mRNA" means that the antisense strand has a polynucleotide that is substantially complementary to a continuous portion of the mRNA of interest (eg, mRNA encoding hepatitis B virus). Alternatively, if a polynucleotide is substantially non-interruptedly complementary to a portion of an mRNA encoding a hepatitis B virus, then the antisense strand is complementary to at least a portion of the mRNA encoding the hepatitis B virus.

In the present invention, the term "target sequence" refers to a continuous portion of the nucleotide sequence of an mRNA molecule formed during transcription of a gene encoding hepatitis B virus, including mRNA that is an RNA processing product of a primary transcription product.

In the present disclosure, a double-stranded siRNA analogue refers to a complex of ribonucleic acid molecules. It has a double-stranded structure, comprises two antiparallel and substantially complementary nucleic acid strands, and has "sense" and "antisense" orientations relative to a target RNA. In the present disclosure, "complementary" has the meaning well known to those skilled in the art. That is, in a double-stranded nucleic acid molecule, bases of one strand pair with bases on the other strand in a complementary manner. A purine base adenine (A) is always paired with a pyrimidine base uracil (U); a pyrimidine base cytosine (G) is always paired with a purine base guanine (C). Each base pair comprises a purine and a pyrimidine. When adenines on one strand are always paired with uracils on the other strand, and guanines are always paired with cytosines, the two strands are considered complementary to each other, and the sequence of the strand can be deduced from the sequence of its complementary strand.

In the present invention, the term "inhibiting the expression of hepatitis B virus genes" includes any level of inhibition of hepatitis B virus (HBV) genes, for example, at least partial inhibition of HBV gene expression, such as inhibition of at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98% or at least about 99%. The expression of the HBV gene can be evaluated based on the level of any variable related to the expression of the HBV gene, for example, the level of HBV mRNA or the level of HBV protein. Inhibition can be assessed by a decrease in the absolute or relative level of one or more of these variables compared to a control level. The control level can be any type of control level utilized in the art, e.g., a baseline level prior to administration, or a level determined from a similar subject, cell, or sample that has not been treated or has been treated with a control (e.g., a buffer-only control or a no active agent control).

In the present invention, "pharmaceutical composition" may refer to a composition used for treating a disease or for in vitro cell culture experiments. When used in the treatment of disease, the term "pharmaceutical composition" generally refers to unit dosage form and can be prepared by any of the methods well known in the art of pharmacy. All methods include the step of bringing the active ingredient into association with the excipient which constitutes one or more accessory ingredients. Typically, the composition is prepared by uniformly and thoroughly combining active double-stranded siRNA with a liquid excipient, a finely divided solid excipient, or both.

The siRNA of the present invention contains a nucleotide group as a basic structural unit. It is well known to those skilled in the art that the nucleotide group contains a phosphate group, a ribose group and a base, which will not be described in detail here. Each nucleotide in the siRNA is independently a modified or unmodified nucleotide, preferably an unmodified nucleotide. The double-stranded siRNA of the present invention contains a sense strand and an antisense strand.

The term "embed" as used herein means that an embedding group is linked to at least one nucleotide residue in the sequence, including replacing a nucleotide residue in the sequence with an embedding group (such as Y).

The "embedding group" described in the present invention is a residue of an analog of a natural nucleotide base, which is different from any natural nucleotide base disclosed in any patent. After being introduced into a nucleic acid sequence, it can give the sequence certain functions (such as bringing unexpected activities). As described in the present invention, Y, when embedded in an oligonucleotide sequence as a embedding group, can inhibit gene expression and thereby produce unexpected activities.

The "oligonucleotide embedde group" of the present invention refers to the embedding group connected to at least one nucleotide residue in the oligonucleotide, including replacing a nucleotide residue in the oligonucleotide with an embedding group (such as Y).

The Y-embedded sequence of the present invention refers to a sequence in which at least one nucleotide residue is connected to Y, including a sequence in which a nucleotide residue is replaced by Y. The Y-embedded sequence of the present invention may also be optionally modified, such as methoxy modification, fluorination modification, and phosphorothioate linkage. The Y-embedded sequence of the present invention includes but is not limited to: Y-embedded siRNA, Y-embedded positive strand and Y-embedded antisense strand.

The conjugate group of the present invention includes a pharmaceutically acceptable conjugate group. Generally speaking, the pharmaceutically acceptable conjugate group comprises a pharmaceutically acceptable targeting molecule and an optional linker. Exemplary conjugate groups, linkers, and targeting molecules can be found in WO2015006740A2 and CN114555188A. Exemplary conjugated groups include, but are not limited to, L96, NAG37, or DAW40007-4 described herein.

Unless otherwise indicated, "conjugation" refers to the covalent linkage of two or more chemical moieties, each with a specific function, to each other; accordingly, "conjugate" refers to a compound formed by covalent linkage of the chemical moieties.

The double-stranded siRNA conjugate of the present invention is a compound formed by connecting the double-stranded siRNA and a pharmaceutically acceptable conjugated group, and the double-stranded siRNA and the pharmaceutically acceptable conjugated group are covalently linked.

In the present invention, the term "pharmaceutically acceptable" means that the substance or composition must be chemically and/or toxicologically compatible with the other ingredients comprising the formulation and/or the mammal to be treated therewith. Preferably, the term "pharmaceutically acceptable" as used herein means approved by a regulatory agency of the Federal or a state government or listed in the U.S. Pharmacopeia or other generally recognized pharmacopeia for use in animals, and more particularly in humans.

In the present invention, the term "pharmaceutically acceptable excipients" may include any solvents, solid excipients, diluents or other liquid excipients, etc., which are suitable for a specific target dosage form. Except to the extent that any conventional excipients are incompatible with the double-stranded siRNA or its conjugate or salt thereof of the present invention, such as any adverse biological effects produced or interactions with any other components of the pharmaceutically acceptable composition in a deleterious manner, their use is also contemplated by the present invention.

As used herein, the term "treat" refers to administering a drug for the purpose of obtaining a desired pharmacological and/or physiological effect. The effect may be prophylactic in terms of complete or partial prevention of the disease or its symptoms, and/or therapeutic in terms of partial or complete cure of the disease and/or its adverse effects. As used herein, "treat" encompasses diseases in mammals, particularly humans, and includes: (a) preventing the occurrence of a disease or condition in an individual who is susceptible to the disease but has not yet been diagnosed with the disease; (b) inhibiting the disease, such as arresting the progression of the disease; or (c) alleviating the disease, such as alleviating the symptoms associated with the disease. As used herein, "treat" encompasses any administration of a drug, RNAi agent, or siRNA to a subject to treat, cure, alleviate, ameliorate, mitigate, or inhibit a disease in the subject, including but not limited to administering a drug containing an RNAi agent, siRNA, or siRNA conjugate described herein to a subject in need thereof.

Unless otherwise specified, in the context of the present invention, capital letters C, G, U and A represent the base composition of natural nucleotides; lowercase letters c, g, u and a respectively represent that the 2-position of the ribose on the nucleotide represented by the corresponding capital letter is modified by methoxy; an underline under a capital letter represents that the 2-position of the ribose on the nucleotide represented by the letter is modified by fluorine; the middle dot **"•"** represents that there is phosphorothioate linkage ( 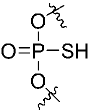 ) between two nucleotide residues adjacent to the left and right sides of the middle dot "•", If there is no **"•"** between nucleotide residues, it means that the nucleotide residues are linked by phosphorothioate ( 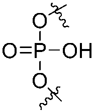 ), for example, "g•u" means that g and u residues are linked by phosphorothioate, and "gu" means that g and u residues are linked by phosphate groups; Agn is adenosine-glycol nucleic acid (Glycol nucleic acid, GNA, 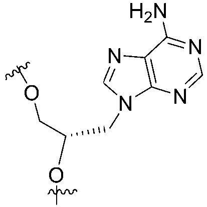 ); Y in double-stranded siRNA represents 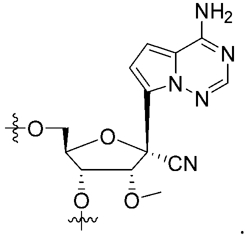 Wherein, the fluorine modification of the ribose position 2 in the present invention means that the hydroxyl group at the ribose position 2 is replaced by F; the methoxy modification of the ribose position 2 means that the hydroxyl group at the ribose position 2 is replaced by a methoxy group.

In the context of the present invention, Bz represents benzoyl; MMTr represents 4'-methoxytrityl; DMTr represents 4',4'-dimethoxytriphenylmethyl.

In the present invention, "phosphate group", "phosphate group" and "phosphate bond" are used interchangeably and include phosphate monoester group, phosphate diester group or phosphate triester group. The "phosphate group" in the "phosphorothioate group" has the same meaning. Unless otherwise specified, natural internucleotide phosphate groups are phosphodiester groups. Unless otherwise specified, the sequence in which Y replaces one or more nucleotide residues in the present invention means that one Y replaces one nucleotide residue, rather than one Y replacing two or more nucleotide residues. If two nucleotide residues are replaced, two Ys should replace two nucleotide residues respectively. For example, if the antisense strand comprises a sequence in which Y replaces zero, one, two, three, four, or five nucleotide residues in the sequence set forth in SEQ ID NO:40, then it refers to a sequence in which 0, 1, 2, 3, 4 or 5 nucleotide residues in the sequence set forth in SEQ ID NO:40 are replaced by 0, 1, 2, 3, 4 or 5 Y, respectively.

The term "oxo" refers to a =O group. For example, a carbon atom is linked to an oxygen atom via a double bond, whereby a ketone or an aldehyde is formed.

As used herein, "chemically modified" or "modification" means a structure that is chemically different when compared to a naturally occurring counterpart, including all changes by chemical means, such as the addition or removal of a chemical moiety, or the substitution of one chemical moiety for another.

The compounds of the present invention may be asymmetric, ie, have one or more stereoisomers. Unless otherwise indicated, all stereoisomers are included, as are enantiomers and diastereomers. The compounds of the present invention containing an asymmetric carbon atom can be isolated in optically pure or racemic forms. Optically pure forms can be resolved from racemic mixtures or synthesized by using chiral starting materials or reagents.

Optically active (*R*)- and (*S*)-isomers, as well as *D* and *L* isomers, can be prepared by chiral synthesis or chiral reagents or other conventional techniques. An enantiomer of a certain compound of the present disclosure can be prepared by asymmetric synthesis or derivatization using a chiral auxiliary, wherein the resulting diastereoisomeric mixture is separated and the auxiliary group is cleaved so as to provide the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art followed by recovery to give the pure enantiomer. Furthermore, the enantiomer and the diastereoisomer are generally isolated through chromatography using a chiral stationary phase, optionally in combination with chemical derivatization (e.g., carbamate generated from amines).

The present invention also includes isotopically-labeled compounds of the invention which are identical to those recited herein, except that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorus, sulfur, fluorine, iodine, and chlorine, such as ²H, ³H, ¹¹C, ¹³C, ^{14C}, ¹³N, ¹⁵N, ^{15O}, ¹⁷O, ¹⁸O, ³¹P, ³²P, ³⁵S, ¹⁸F, ¹²³I, ¹²⁵I and ³⁶Cl, respectively.

Unless otherwise indicated, when a position is specifically designated as deuterium (D), that position is understood to have an abundance of deuterium at least 1000 times greater than the natural abundance of deuterium (which is 0.015%) (i.e., at least 10% deuterium incorporation). Example compounds having an abundance greater than the natural abundance of deuterium can include at least 1000 times greater deuterium, at least 2000 times greater deuterium, at least 3000 times greater deuterium, at least 4000 times greater deuterium, at least 5000 times greater deuterium, at least 6000 times greater deuterium, or even greater abundances of deuterium. The present invention also encompasses the compounds of formula (I) in various deuterated forms. Each available hydrogen atom attached to a carbon atom can independently be replaced with a deuterium atom. Those skilled in the art can synthesize the deuterated form of the compound of formula (I) by referring to relevant literature. In preparing deuterated forms of compounds of formula (I), commercially available deuterated starting materials may be used, or they may be synthesized using conventional techniques using deuterated reagents, including but not limited to deuterated borane, trideuterated borane in tetrahydrofuran, deuterated lithium aluminum hydride, deuterated iodoethane, deuterated iodomethane, and the like.

The conjugated groups described herein can enhance the delivery of therapeutic agents to specific target locations (eg, specific organs or tissues) within a subject, such as a human or animal. In some embodiments of the present invention, the conjugated group can enhance the targeted delivery of expression-inhibitory double-stranded siRNA. In some embodiments of the present invention, the conjugated group can enhance the targeted delivery of expression-inhibitory double-stranded siRNA to the liver.

The conjugate groups described herein can be linked directly or indirectly to a compound, such as a therapeutic agent, e.g., an expression inhibitory oligonucleotide, e.g., to the 3' or 5' end of an expression inhibitory oligonucleotide. In some embodiments of the invention, the expression inhibitory oligonucleotide comprises one or more modified nucleotides. In some embodiments of the invention, the expression-inhibiting oligonucleotide is an RNAi agent, such as a double-stranded siRNA agent comprising a sense strand and an antisense strand. In some embodiments of the present invention, the conjugated group disclosed herein is attached to the 3' end of the sense strand of a double-stranded siRNA agent. In some embodiments, the conjugate group disclosed herein is linked to the expression inhibitory oligonucleotide agent at the 3' end of the sense strand of the double-stranded siRNA agent via a phosphate, phosphorothioate, or phosphonate group.

Stereochemical definitions and conventions used herein generally follow S. P. Parker Ed. , McGraw-Hill Dictionary of Chemical Terms (1984) McGraw-Hill Book Company, New York and Eliel et al., "Stereochemistry of Organic Compounds", John Wiley & Sons, Inc., New York, 1994. The compounds disclosed herein may contain asymmetric or chiral centers, and therefore exist in different stereoisomeric forms. It is intended that all stereoisomeric forms of the compounds disclosed herein, including, but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes *D* and *L,* or R and *S*, are used to denote the absolute configuration of the molecule about its chiral center(s). The prefixes *d* and *1* or (+) and (-) are employed to designate the sign of rotation of plane-polarized light by the compound, with (-) or *l* meaning that the compound is levorotatory. A compound prefixed with (+) or *d* is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The term "racemic mixture" or "racemate" refers to an equimolar mixture of two enantiomeric species, devoid of optical activity.

The term "tautomer" or "tautomeric form" refers to structural isomers of different energies which are interconvertible via a low energy barrier. Some non-limiting examples of proton tautomers (also known as prototropic tautomers) include interconversions via migration of a proton, such as keto-enol and imine-enamine isomerizations.

The term "composition" refers to a mixture of a drug containing one or more compounds described herein, or a physiologically acceptable salt or precursor thereof, and other chemical components, as well as other components such as physiologically acceptable carriers and excipients. The purpose of the composition is to facilitate administration to an organism, facilitate the absorption of the active ingredient and thus exert biological activity.

The term "pharmaceutically acceptable excipient" or "pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. Food and Drug Administration as acceptable for use in humans or domestic animals.

Unless otherwise specified, the "compound", "ligand", "nucleic acid conjugate", "double-stranded siRNA conjugate", "double-stranded siRNA" and "nucleic acid" of the present invention can independently exist in the form of salt, mixed salt or non-salt (such as free acid or free base). When present in the form of a salt or mixed salt, it may be a pharmaceutically acceptable salt.

The term "acceptable salts" includes acceptable acid addition salts and pharmaceutically acceptable base addition salts. "Acceptable acid addition salts" refer to salts formed with inorganic or organic acids that retain the biological effectiveness of the free base without other adverse effects. Inorganic acid salts include, but are not limited to, hydrochloride, hydrobromide, sulfate, nitrate, phosphate, and the like; organic acid salts include, but are not limited to, formate, acetate, 2,2-dichloroacetate, trifluoroacetate, propionate, caproate, octanoate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalene disulfonate, and the like. These salts can be prepared by methods known in the art.

"Pharmaceutically acceptable base addition salt" refers to a salt formed with an inorganic base or an organic base that retains the biological effectiveness of the free acid without other side effects. Salts derived from inorganic bases include, but are not limited to, sodium, potassium, lithium, ammonium, calcium, magnesium, iron, zinc, copper, manganese, aluminum salts, and the like. Preferred inorganic salts are ammonium salts, sodium salts, potassium salts, calcium salts and magnesium salts, preferably sodium salts. Salts derived from organic bases include, but are not limited to, salts of primary, secondary, and tertiary amines, substituted amines, including naturally substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purines, piperazine, piperidine, N-ethylpiperidine, polyamine resins, and the like. Preferred organic bases include isopropylamine, diethylamine, ethanolamine, trimethylamine, dicyclohexylamine, choline and caffeine. These salts can be prepared by methods known in the art.

As described herein, compounds disclosed herein may optionally be substituted with one or more substituents, such as are illustrated generally below, or as exemplified by particular classes, subclasses, and species of the invention. In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position.

Furthermore, unless otherwise stated, the phrase "each...is independently" is used interchangeably with the phrase "each (of)...and...is independently". It should be understood broadly that the specific options expressed by the same symbol are independently of each other in different radicals; or the specific options expressed by the same symbol are independently of each other in same radicals.

In the present invention, the terms "optionally" or "optional" generally mean that the subsequently described event or circumstance can but need not occur, and that the description includes instances where the event or circumstance occurs and instances where it does not occur.

At various places in the present specification, substituents of compounds disclosed herein are disclosed in groups or in ranges. It is specifically intended that the invention include each and every individual subcombination of the members of such groups and ranges. For example, the term "C₁₋₆ alkyl" is specifically intended to individually disclose methyl, ethyl, C₃ alkyl, C₄ alkyl, C₅ alkyl and C₆ alkyl.

The term "alkyl" or "alkyl group" refers to a saturated linear or branched-chain monovalent hydrocarbon radical of 1 to 20 carbon atoms, wherein the alkyl radical may be optionally and independently substituted with one or more substituents described herein. In some embodiments, the alkyl group contains 1-12 carbon atoms. In other embodiments, the alkyl group contains 1-10 carbon atoms. In other embodiments, the alkyl group contains 1-8 carbon atoms. In still other embodiments, the alkyl group contains 1-6 carbon atoms. In yet other embodiments, the alkyl group contains 1-4 carbon atoms and in still yet other embodiments, the alkyl group contains 1-3 carbon atoms. Some non-limiting examples of the alkyl group include, methyl (Me, -CH₃), ethyl (Et, -CH₂CH₃), *n*-propyl (*n*-Pr, -CH₂CH₂CH₃), isopropyl (*i*-Pr, -CH(CH₃)₂), *n*-butyl (*n*-Bu, -CH₂CH₂CH₂CH₃), 2-methylpropyl or isobutyl (*i*-Bu, -CH₂CH(CH₃)₂), 1-methylpropyl or sec-butyl (s-Bu, -CH(CH₃)CH₂CH₃), *tert*-butyl (*t*-Bu, -C(CH₃)₃), *n*-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), *n*-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, *n*-heptyl, *n*-octyl, *n*-nonyl and *n*-decyl, etc.

The term "alkoxy" refers to an alkyl group, as previously defined, attached to the parent molecular moiety via an oxygen atom. Unless otherwise specified, the alkoxy group contains 1-12 carbon atoms. In some embodiments, the alkoxy group contains 1-8 carbon atoms; in other embodiments, the alkoxy group contains 1-6 carbon atoms; in still other embodiments, the alkoxy group contains 1-4 carbon atoms; in yet other embodiments, the alkoxy group contains 1-3 carbon atoms. The alkoxy group may be optionally substituted with one or more substituents disclosed herein.

Some non-limiting examples of the alkoxy group include, but are not limited to, methoxy (MeO, -OCH₃), ethoxy (EtO, -OCH₂CH₃), 1-propoxy (n-PrO, n-propoxy, -OCH₂CH₂CH₃), 2-propoxy (*i*-PrO, *i*-propoxy, -OCH(CH₃)₂), 1-butoxy (*n*-BuO, *n*-butoxy, -OCH₂CH₂CH₂CH₃), 2-methyl-l-propoxy (*i*-BuO, *i*-butoxy, -OCH₂CH(CH₃)₂), 2-butoxy (*s*-BuO, *s*-butoxy, -OCH(CH₃)CH₂CH₃), 2-methyl-2-propoxy (*t*-BuO, *t*-butoxy, -OC(CH₃)₃), 1-pentoxy (*n*-pentoxy, -OCH₂CH₂CH₂CH₂CH₃), 2-pentoxy (-OCH(CH₃)CH₂CH₂CH₃), 3-pentoxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-1-butoxy (-OCH₂CH₂CH(CH₃)₂), 2-methyl-1-butoxy (-OCH₂CH(CH₃)CH₂CH₃), and the like.

The term "alkenyl" refers to a linear or branched monovalent, divalent or polyvalent hydrocarbon group containing 2-12 carbon atoms, or 2-8 carbon atoms, or 2-6 carbon atoms, or 2-4 carbon atoms, wherein at least one position of the C-C is an sp² double bond, wherein the alkenyl group can be independently unsubstituted or substituted with one or more substituents described in the present invention, including "*cis*", "*trans*" or "*Z*", "*E*" isomers, specific examples of which include, but are not limited to, vinyl (-CH=CH₂), propenyl (-CH=CHCH₃), allyl (-CH₂CH=CH₂), etc., wherein the alkenyl group can be independently unsubstituted or substituted with one or more substituents described in the present invention.

The term "consisting of M-M₁ ring atoms" means that the cyclic group is composed of M-M₁ ring atoms, and the ring atoms include carbon atoms and/or heteroatoms such as O, N, S and P. For example, "heterocyclyl consisting of 3-6 ring atoms" means a monocyclic heterocyclyl consisting of 3, 4, 5 or 6 ring atoms.

The term "heterocyclyl" refers to a saturated or unsaturation, nonaromatic, monocyclic, bicyclic or tricyclic ring system in which at least one ring member is selected from nitrogen, sulfur or oxygen. Wherein, the heterocyclyl group may be optionally substituted with one or more substituents disclosed herein. Unless otherwise specified, the heterocyclyl group may be carbon or nitrogen linked, and a -CH₂- group can be optionally replaced by -C(=O)- or -C(=S)-. The sulfur can be optionally oxygenized to S-oxide The nitrogen can be optionally oxygenized to N-oxide. In some embodiments, the heterocyclyl is a heterocyclyl consisting of 3-12 ring atoms, in some embodiments, the heterocyclyl is a heterocyclyl consisting of 5-10 ring atoms, in some embodiments, the heterocyclyl is a heterocyclyl consisting of 3-6 ring atoms, in some embodiments, the heterocyclyl is a heterocyclyl consisting of 4-6 ring atoms, in some embodiments, the heterocyclyl is a heterocyclyl consisting of 5-6 ring atoms, in other embodiments, the heterocyclyl is a heterocyclyl consisting of 4 atoms, which refers to a monovalent or polyvalent, saturated or partially unsaturated, non-aromatic monocyclic ring containing 4 ring atoms, wherein at least one ring atom is selected from nitrogen, sulfur and oxygen atoms. In other embodiments, the heterocyclyl is a heterocyclyl consisting of 5 atoms, which refers to a monovalent or multivalent, saturated or partially unsaturated, non-aromatic monocyclic ring containing 5 ring atoms, wherein at least one ring atom is selected from nitrogen, sulfur and oxygen atoms. In other embodiments, the heterocyclyl is a heterocyclyl consisting of 6 atoms, which refers to a monovalent or multivalent, saturated or partially unsaturated, non-aromatic monocyclic ring containing 6 ring atoms, wherein at least one ring atom is selected from nitrogen, sulfur and oxygen atoms. The "heterocyclyl" also includes a group in which the heterocyclyl is fused with a saturated or partially unsaturated ring or a heterocyclic ring. Some non-limiting examples of the heterocyclyl include pyrrolidinyl, tetrahydrofuranyl, dihydrofuranyl, tetrahydrothienyl, tetrahydropyranyl, dihydropyranyl, tetrahydrothiopyranyl, piperidinyl, morpholinyl, thiomorpholinyl, thioxanyl, piperazinyl, homopiperazinyl, azetidinyl, oxetanyl, thietanyl, homopiperidinyl, epoxypropyl, azepanyl, oxepanyl, thiepanyl, oxazepinyl, diazepinyl, thiazepinyl, 2-pyrrolinyl, 3-pyrrolinyl, dihydroindolyl, 2*H*-pyranyl, 4*H*-pyranyl, dioxanyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3*H*-indolylquinolizinyl and *N*-pyridylurea. Examples of heterocyclic groups also include 1,1-dioxothiomorpholinyl, examples wherein the carbon atoms on the ring are substituted with oxo (=O) include, but are not limited to, pyrimidinedionyl, 1,2,4-thiadiazole-5(4*H*)-onyl, 1,2,4-oxadiazole-5(4*H*)-onyl, 1*H*-1,2,4-triazole-5(4*H*)-onyl, etc., examples wherein the carbon atoms on the ring are substituted with a group =S include, but are not limited to, 1,2,4-oxadiazole-5(4*H*)-thionyl, 1,3,4-oxadiazole-2(3*H*)-thionyl, etc.

The term "heteroatom" refers to one or more of O, S, N, P and Si, including N, S and P in any oxidation state; in the form of primary, secondary, tertiary amines and quaternary ammonium salts; or in the form of a nitrogen atom in a heterocyclic ring in which the hydrogen is substituted, for example, N (as N in 3,4-dihydro-2*H*-pyrrolyl), NH (as NH in pyrrolidinyl) or NR (as NH in *N*-substituted pyrrolidinyl), wherein R represents a substituent as described herein.

The terms "alkylsilyl" and "alkylsilyl" refer to silyl (-SiH₃) groups in which the hydrogen atoms are independently replaced by 1, 2 or 3 alkyl groups, respectively. wherein, in some embodiments, the alkylsilyl group is a lower alkylsilyl group formed by 1, 2 or 3 C₁₋₁₂ alkyl groups attached to a silicon atom. In other embodiments, the alkylsilyl group is a lower alkylsilyl group formed by 1, 2 or 3 C₁₋₉ alkyl groups attached to a silicon atom. In other embodiments, the alkylsilyl group is a lower alkylsilyl group formed by 1, 2 or 3 C₁₋₆ alkyl groups attached to a silicon atom. In other embodiments, the alkylsilyl group is a lower alkylsilyl group formed by 1, 2 or 3 C₁₋₄ alkyl groups attached to a silicon atom. In another embodiments, the alkylsilyl group is a lower alkylsilyl group formed by 1, 2 or 3 C₁₋₃ alkyl groups attached to a silicon atom. Suitable alkylsilyl groups can be monoalkylsilyl, dialkylsilyl or trialkylsilyl. Examples of alkylsilyl groups include, but are not limited to, trimethylsilyl (-Si(CH₃)₃), triethylsilyl (-Si(CH₂CH₃)₃), tri-n-propylsilyl (-Si(CH₂CH₂CH₃)₃), and the like.

The term "hydroxy protecting group" refers to a labile chemical moiety that protects a hydroxy group against undesirable reactions during one or more synthetic procedures. After the one or more synthetic procedures, the hydroxy protecting group can be selectively removed. Hydroxyl protecting groups known in the art are generally described in T. H. Greene and P. G. M. Wuts, Protective Groups in Organic Synthesis, 3rd ed., John Wiley & Sons, New York (1999). Examples of hydroxy protecting groups of the present invention include, but are not limited to, C₁₋₁₂ alkyl, benzyloxycarbonyl, 4-nitrobenzyloxycarbonyl, 4-bromobenzyloxycarbonyl, 4-methoxybenzyloxycarbonyl, methoxycarbonyl, tert-butoxycarbonyl, isopropyloxycarbonyl, biphenylmethoxycarbonyl, 2,2,2-trichloroethoxycarbonyl, 2-(trimethylsilyl)ethoxycarbonyl, 2-furfuryloxycarbonyl, allyloxycarbonyl, acetyl (Ac or -C(O)CH₃), formyl, chloroacetyl, trifluoroacetyl, methoxyacetyl, phenoxyacetyl, benzoyl (Bz or -C(O)C₆H₅), C₁₋₁₀ alkyl (methyl, tert-butyl, etc.), 2,2,2-trichloroethyl, 2-trimethylsilylethyl, 1,1-dimethyl-2-propenyl, 3-methyl-3-butenyl, allyl, C₆₋₁₀ aryl C₁₋₄ alkyl (such as benzyl, phenethyl, etc.), *p*-methoxybenzyldiphenylmethyl, triphenylmethyl (triphenylmethyl or trityl), tetrahydrofuranyl, methoxymethyl, methylthiomethyl, benzyloxymethyl, 2,2,2-trichloroethoxymethyl, 2-(trimethylsilyl)ethoxymethyl, methylsulfonyl, *p*-toluenesulfonyl, C₁₋₁₀ alkylsilyl (such as trimethylsilyl (TMS or -Si(CH₃)₃)), triethylsilyl, triisopropylsilyl, MMTr, DMTr or 4',4',4'-trimethoxytrityl, etc.

The term "amino protecting group" refers to a labile chemical moiety that protects an amino group against undesirable reactions during synthetic procedures. After the one or more synthetic procedures, the amino protecting group can be selectively removed as described herein. Amino protecting groups known in the art are generally described in T. H. Greene and P. G. M. Wuts, Protective Groups in Organic Synthesis, 3rd ed., John Wiley & Sons, New York (1999). Examples of amino protecting groups include, but are not limited to, acetyl, *tert*-butoxycarbonyl, 9-fluorenylmethoxycarbonyl, benzyloxycarbonyl, and the like.

The term "solid support" specifically denotes any particle, bead or surface on which oligonucleotide synthesis can take place. For example, both inorganic solid supports and organic solid supports can be selected for use in the embodiments of the present invention. The inorganic solid support is preferably selected from silica gel and controlled-pore glass beads (CPG). The organic solid support is a resin, preferably a macroporous resin, more preferably highly cross-linked polystyrene, Tentagel (a graft copolymer composed of a low-cross-linked polystyrene matrix with polyethylene glycol (PEG or POE) grafted thereon), polyvinyl acetate (PVA), Poros-polystyrene/divinylbenzene copolymer, aminopolyethylene glycol and cellulose, etc. Preferred embodiments of the present invention utilize CPG-based solid supports. Many other commercially available solid supports are contemplated by the present invention.

### Description of the drawings:

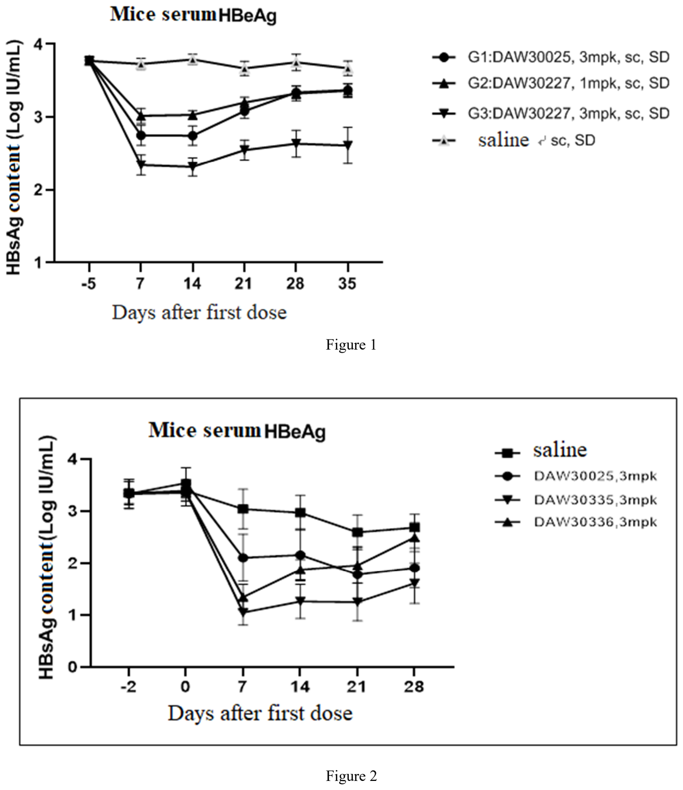

Figure 1 shows the inhibitory effects of the present compound DAW30227 (i.e., double-stranded siRNA conjugate S146), the control compound DAW30025 (i.e., double-stranded siRNA conjugate S149), and the vehicle control group (normal saline) on HBsAg in an HBV-Tg mouse model experiment. In the figures, SD represents single dose.
Figure 2 shows the inhibitory effects of the present compound DAW30335 (i.e., double-stranded siRNA conjugate S147), the control compound DAW30025 (i.e., double-stranded siRNA conjugate S149), DAW30336 (i.e., siRNA conjugate S151), and the vehicle control group (normal saline) on HBsAg in an AAV mouse model experiment.

### DETAILED DESCRIPTION OF COMPOUNDS OF THE INVENTION

The present invention provides a new targeting compound, which can be used to prepare a nucleic acid drug for delivering oligonucleotides to the liver in a targeted manner. The present invention also provides a nucleotide conjugate, a preparation method and use thereof, wherein the nucleotide conjugate has higher *in vivo* delivery efficiency, better stability, higher gene expression inhibition activity in hepatocytes and/or lower toxicity. On the other hand, the present invention further provides an siRNA reagent, a preparation method and application thereof, wherein the siRNA reagent has high in vivo delivery efficiency, good stability, high gene expression inhibition activity in HBV and/or low toxicity.

The present invention aims to provide a novel double-stranded siRNA, a conjugate thereof, a salt thereof and a use thereof, and specifically relates to the use of the double-stranded siRNA and conjugate thereof in preparing a drug for treating and/or preventing hepatitis B. In addition, the present invention also relates to a novel compound, and a use of the compound in raw materials for solid-phase synthesis of DNA nucleotides, raw materials for synthesis of siRNA drugs, siRNA drug research, gene function research and/or screening of whole gene libraries, especially the use of the compound as a raw material for synthesis of the double-stranded siRNA drug described in the present invention. Furthermore, the present invention also relates to a novel nucleotide residue and its use in siRNA drug research, as a raw material for the synthesis of gene function research, and/or as a raw material for screening of whole gene libraries, as well as as an embedding group in oligonucleotides. The double-stranded siRNA and conjugate thereof of the present invention have higher gene expression inhibition activity and/or lower toxicity against HBV.

In one aspect, the present invention relates to a double-stranded siRNA, a conjugate thereof, or a salt thereof, the double-stranded siRNA comprises a sense strand and an antisense strand, wherein, the double-stranded siRNA comprisesone or more Y (i.e., the double-stranded siRNA of the present invention comprises at least one Y), the Y is 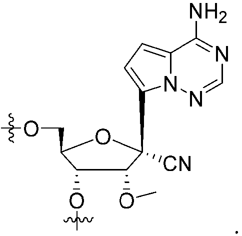 Unless otherwise specified, each of nucleotides and Y in the double-stranded siRNA of the present invention is independently modified or unmodified. Optionally, the Y can be at any position in the double-stranded siRNA.

In some embodiments of the double-stranded siRNA, conjugate or salt thereof of the present invention, wherein, the double-stranded siRNA comprises 1, 2, 3, 4, 5, 6, 7, 8, 9 or 10 Y.

In some embodiments of the double-stranded siRNA, conjugate or salt thereof of the present invention, wherein, the double-stranded siRNA comprises 1, 2, 3, 4 or 5 Y. Optionally, the Y can be at any position in the double-stranded siRNA.

In some embodiments of the double-stranded siRNA, conjugate or salt thereof of the present invention, the sense strand in the double-stranded siRNA comprises at least one (i.e., 1, 2, 3, 4 or 5) Y. Optionally, the Y can be at any position in the sense strand.

In some embodiments of the double-stranded siRNA, conjugate or salt thereof of the present invention, the antisense strand in the double-stranded siRNA comprises at least one (i.e., 1, 2, 3, 4 or 5) Y. Optionally, the Y can be at any position in the antisense strand.

In some embodiments of the double-stranded siRNA, conjugate or salt thereof of the present invention, in the double-stranded siRNA, the sense strand comprises 0 or at least one (e.g., 1, 2, 3, 4 or 5) Y, and the antisense strand comprises at least one (e.g., 1, 2, 3, 4 or 5) Y. Optionally, the Y can be at any position in the sense strand or the antisense strand.

In some embodiments of the double-stranded siRNA, conjugate or salt thereof of the present invention, the length of the antisense strand of the double-stranded siRNA of the present invention is not more than 30 nucleotides. Preferably, the length of the antisense strand of the double-stranded siRNA of the present invention is not more than 23 nucleotides. More preferably, the length of the antisense strand of the double-stranded siRNA of the present invention is not more than 21 nucleotides.

In some embodiments of the double-stranded siRNA, conjugate or salt thereof of the present invention, the length of the sense strand of the double-stranded siRNA of the present invention is not more than 30 nucleotides. Preferably, the length of the sense strand of the double-stranded siRNA of the present invention is not more than 23 nucleotides. More preferably, the length of the sense strand of the double-stranded siRNA of the present invention is not more than 19 or 21 nucleotides.

In some embodiments of the double-stranded siRNA, conjugate or salt thereof of the present invention, the antisense strand comprises a sequence of 5'-UGUGAAGCGAAGUGCACACUU-3' (SEQ ID NO:40), or a sequence in which one or more (i.e., 1, 2, 3, 4 or 5) nucleotide residues are replaced by Y in the sequence of SEQ ID NO:40; wherein each of nucleotides and Y in the antisense strand is independently modified or unmodified, and the length of the antisense strand is not more than 23 nucleotides. Optionally, the Y substitution occurs at any position in the sequence of SEQ ID NO:40,

In some embodiments of the double-stranded siRNA, conjugate or salt thereof of the present invention, the sense strand comprises a sequence of 5'-GUGUGCACUUCGCUUCACA-3' (SEQ ID NO:20), or a sequence in which one or more (i.e., 1, 2, 3, 4 or 5) nucleotide residues are replaced by Y in the sequence of SEQ ID NO:20; wherein each of nucleotides and Y in the sense strand is independently modified or unmodified, and the length of the sense strand is not more than 23 nucleotides. Optionally, the Y substitution occurs at any position in the sequence of SEQ ID NO:20.

In some embodiments of the double-stranded siRNA, conjugate or salt thereof of the present invention, the antisense strand comprises a sequence in which one or more (e.g., 1, 2, 3, 4 or 5) nucleotide residues are substituted with Y in the sequence of 5'-UGUGAAGCGAAGUGCACACUU-3' (SEQ ID NO:40); the sense strand comprises a sequence in which one or more (e.g., 1, 2, 3, 4 or 5) nucleotide residues are substituted with Y in the sequence of SEQ ID NO:20; wherein the Y substitution occurs at any position in the sequence of SEQ ID NO:40 or SEQ ID NO:20, and the lengths of the antisense strand and the sense strand are each independently no more than 23 nucleotides.

In some embodiments of the double-stranded siRNA, conjugate or salt thereof of the present invention, more than 70%, 75%, 80%, 85%, 90% or 95% of the nucleotides and Y in the sense strand or the antisense strand of the double-stranded siRNA are modified.

In some embodiments of the double-stranded siRNA, conjugate or salt thereof of the present invention, more than 70%, 75%, 80%, 85%, 90% or 95% of the nucleotides and Y in the sense strand and the antisense strand of the double-stranded siRNA are modified.

In some embodiments of the double-stranded siRNA, conjugate or salt thereof of the present invention, all the nucleotides and Y in the double-stranded siRNA are modified.

In some embodiments of the double-stranded siRNA, conjugate or salt thereof of the present invention, the modification is selected from at least one of the following: 2'-methoxy modification (i.e., the hydroxyl group at position 2 of ribose is replaced by a methoxy group), 2'-methoxyethyl modification (i.e., the hydroxyl group at position 2 of ribose is replaced by a methoxyethyl group), 2'-fluoro modification (i.e., the hydroxyl group at position 2 of ribose is replaced by an F group), phosphorothioate linkage, 2'-deoxy modification, 2'-amino modification (i.e., the hydroxyl group at position 2 of ribose is replaced by an amino group), locked nucleic acid modification, unlocked nucleic acid modification, ethylene glycol nucleic acid modification and 5'-vinyl phosphate ester modification.

In some embodiments of the double-stranded siRNA, conjugate or salt thereof of the present invention, wherein, the sense strand comprises a sequence in which 0, 1, 2, 3, 4 or 5 nucleotide residues are replaced with Y in the sequence of SEQ ID NO:20, wherein each of nucleotides and Y in the sense strand are independently modified or unmodified.

In some embodiments of the double-stranded siRNA, conjugate or salt thereof of the present invention, wherein, the sense strand comprises a sequence in which one nucleotide residue is replaced with Y in the sequence of SEQ ID NO:20.

In some embodiments of the double-stranded siRNA, conjugate or salt thereof of the present invention, wherein, the antisense strand comprises a sequence in which 0, 1, 2, 3, 4 or 5 nucleotide residues are replaced with Y in the sequence of SEQ ID NO:40, wherein each of nucleotides and Y in the antisense strand are independently modified or unmodified.

In some embodiments of the double-stranded siRNA, conjugate or salt thereof of the present invention, wherein, the antisense strand comprises a sequence in which one nucleotide residue is replaced with Y in the sequence of SEQ ID NO:40.

In some embodiments of the double-stranded siRNA, conjugate or salt thereof of the present invention, wherein, the Y substitution occurs at positions 1 to 19 from the 5' end of the sequence of SEQ ID NO: 20 (i.e., the Y substitution optionally occurs at position 1, 2, 3, 4, 5, 6, 7, 8, 9, 10, 11, 12, 13, 14, 15, 16, 17, 18 or 19 from the 5' end of the sequence of SEQ ID NO: 20).

In some embodiments of the double-stranded siRNA, conjugate or salt thereof of the present invention, wherein, the Y substitution occurs at positions 1 to 21 from the 5' end of the sequence of SEQ ID NO: 40 (i.e., the Y substitution optionally occurs at position 1, 2, 3, 4, 5, 6, 7, 8, 9, 10, 11, 12, 13, 14, 15, 16, 17, 18, 19, 20 or 21 from the 5' end of the sequence of SEQ ID NO: 40).

In some embodiments of the double-stranded siRNA, conjugate or salt thereof of the present invention, wherein, the sense strand comprises a sequence in which 0, 1, 2, 3, 4 or 5 nucleotide residues are substituted with Y in the sequence of 5'-g•u•guGcACUucgcuucaca-3' (SEQ ID NO: 19), wherein each of nucleotides and Y in the sense strand is independently modified or unmodified, and the Y substitution occurs at positions 1 to 19 from the 5' end of the sequence of SEQ ID NO: 19 (i.e., the Y substitution optionally occurs at position 1, 2, 3, 4, 5, 6, 7, 8, 9, 10, 11, 12, 13, 14, 15, 16, 17, 18 or 19 from the 5' end of the sequence of SEQ ID NO: 19), and the length of the sense strand is not more than 23 nucleotides.

In some embodiments of the double-stranded siRNA, conjugate or salt thereof of the present invention, wherein, the antisense strand comprises a sequence in which 0, 1, 2, 3, 4 or 5 nucleotide residues are substituted with Y in the sequence of 5'-u•G•ugaAgCGaaguGcAcac•u•u-3' (SEQ ID NO: 36), wherein each of nucleotides and Y in the antisense strand is independently modified or unmodified, and the Y substitution occurs at positions 1 to 21 from the 5' end of the antisense strand SEQ ID NO: 36 (i.e., the Y substitution optionally occurs at position 1, 2, 3, 4, 5, 6, 7, 8, 9, 10, 11, 12, 13, 14, 15, 16, 17, 18 or 19 from the 5' end of the sequence of SEQ ID NO: 36), and the length of the antisense strand is not more than 23 nucleotides.

In some embodiments of the double-stranded siRNA, conjugate or salt thereof of the present invention, wherein, the antisense strand comprises a sequence in which 0, 1, 2, 3, 4 or 5 nucleotide residues are substituted with Y in the sequence of 5'-u•G•uga(Agn)gCGaaguGcAcac•u•u-3' (SEQ ID NO: 33), wherein each of nucleotides and Y in the antisense strand is independently modified or unmodified, and the Y substitution occurs at positions 1 to 21 from the 5' end of the sequence of SEQ ID NO: 33 (i.e., the Y substitution optionally occurs at position 1, 2, 3, 4, 5, 6, 7, 8, 9, 10, 11, 12, 13, 14, 15, 16, 17, 18 or 19 of the 5' end of the sequence of SEQ ID NO: 33), and the length of the antisense strand is not more than 23 nucleotides.

Unless otherwise specified, in the context of the present invention, capital letters C, G, U and A in oligonucleotides or siRNA represent the base composition of natural nucleotides; lowercase letters c, g, u and a respectively represent that the 2-position of the ribose on the nucleotide represented by the corresponding capital letter is modified by methoxy; an underline under a capital letter represents that the 2-position of the ribose on the nucleotide represented by the letter is modified by fluorine; Agn is adenosine-ethylene glycol nucleic acid; the middle dot "•" represents that there is phosphorothioate linkage between two nucleotide residues adjacent to the left and right sides of the middle dot "•".

In some embodiments of the double-stranded siRNA, conjugate or salt thereof of the present invention, the sense strand comprises one of the following nucleotide sequences:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19 and SEQ ID NO:21;
the length of the sense strand is not more than 23 nucleotides.

In some embodiments of the double-stranded siRNA, conjugate or salt thereof of the present invention, the antisense strand comprises one of the following nucleotide sequences:
SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38 and SEQ ID NO:39;
the length of the antisense strand is not more than 23 nucleotides.

In some embodiments of the double-stranded siRNA, conjugate or salt thereof of the present invention, wherein, the double-stranded siRNA comprises one of the following:
A sense strand comprising the nucleotide sequence of SEQ ID NO: 15 and an antisense strand comprising the nucleotide sequence of SEQ ID NO: 25, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 25, wherein the sense strand and antisense strand are each independently not more than 23 nucleotides;
A sense strand comprising the nucleotide sequence of SEQ ID NO: 15 and an antisense strand comprising the nucleotide sequence of SEQ ID NO: 26, wherein the sense strand and antisense strand are each independently not more than 23 nucleotides;
A sense strand comprising the nucleotide sequence of SEQ ID NO: 16 and an antisense strand comprising the nucleotide sequence of SEQ ID NO: 26, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 26, wherein the sense strand and antisense strand are each independently not more than 23 nucleotides;
A sense strand comprising the nucleotide sequence of SEQ ID NO: 15 and an antisense strand comprising the nucleotide sequence of SEQ ID NO: 27, wherein the sense strand and antisense strand are each independently not more than 23 nucleotides;
A sense strand comprising the nucleotide sequence of SEQ ID NO: 16 and an antisense strand comprising the nucleotide sequence of SEQ ID NO: 27, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 27, wherein the sense strand and antisense strand are each independently not more than 23 nucleotides;
A sense strand comprising the nucleotide sequence of SEQ ID NO: 16 and an antisense strand comprising the nucleotide sequence of SEQ ID NO: 28, wherein the sense strand and antisense strand are each independently not more than 23 nucleotides;
A sense strand comprising the nucleotide sequence of SEQ ID NO: 17 and an antisense strand comprising the nucleotide sequence of SEQ ID NO: 28, wherein the sense strand and antisense strand are each independently not more than 23 nucleotides; or
A sense strand comprising the nucleotide sequence of SEQ ID NO: 18 and an antisense strand comprising the nucleotide sequence of SEQ ID NO: 28, wherein the sense strand and antisense strand are each independently not more than 23 nucleotides.

In some embodiments of the double-stranded siRNA, conjugate or salt thereof of the present invention, the double-stranded siRNA is selected from one of S1 to S143 in Table 1. In some embodiments of the double-stranded siRNA, conjugate or salt thereof of the present invention, the conjugate of the double-stranded siRNA is selected from S146, S147 and S150 in Table 1.

**Table 1**

| **Numbers of the double-stranded siRNA** | **Sense strand sequence (5'-3')** | **SEQ ID NO** | **Antisense strand sequence (5'-3')** | **SEQ ID NO** |
|---|---|---|---|---|
| S1 | g•Y•guGcACUucgcuucaca | 1 | u•Y•ugaAgCGaaguGcAcac•u•u | 22 |
| S2 | g•u•YuGcACUucgcuucaca | 2 | u•Y•ugaAgCGaaguGcAcac•u•u | 22 |
| S3 | g•u•gYGcACUucgcuucaca | 3 | u•Y•ugaAgCGaaguGcAcac•u•u | 22 |
| S4 | g•u•guYcACUucgcuucaca | 4 | u•Y•ugaAgCGaaguGcAcac•u•u | 22 |
| S5 | g•u•guGYACUucgcuucaca | 5 | u•Y•ugaAgCGaaguGcAcac•u•u | 22 |
| S6 | g•u•guGcYCUucgcuucaca | 6 | u•Y•ugaAgCGaaguGcAcac•u•u | 22 |
| S7 | g•u•guGcAYUucgcuucaca | 7 | u•Y•ugaAgCGaaguGcAcac•u•u | 22 |
| S8 | g•u•guGcACYucgcuucaca | 8 | u•Y•ugaAgCGaaguGcAcac•u•u | 22 |
| S9 | g•u•guGcACUYcgcuucaca | 9 | u•Y•ugaAgCGaaguGcAcac•u•u | 22 |
| S10 | g•u•guGcACUuYgcuucaca | 10 | u•Y•ugaAgCGaaguGcAcac•u•u | 22 |
| S11 | g•u•guGcACUucYcuucaca | 11 | u•Y•ugaAgCGaaguGcAcac•u•u | 22 |
| S12 | g•u•guGcACUucgYuucaca | 12 | u•Y•ugaAgCGaaguGcAcac•u•u | 22 |
| S13 | g•u•guGcACUucgcYucaca | 13 | u•Y•ugaAgCGaaguGcAcac•u•u | 22 |
| S14 | g•u•guGcACUucgcuYcaca | 14 | u•Y•ugaAgCGaaguGcAcac•u•u | 22 |
| S15 | g•u•guGcACUucgcuuYaca | 15 | u•Y•ugaAgCGaaguGcAcac•u•u | 22 |
| S16 | g•u•guGcACUucgcuucYca | 16 | u•Y•ugaAgCGaaguGcAcac•u•u | 22 |
| S17 | g•u•guGcACUucgcuucaYa | 17 | u•Y•ugaAgCGaaguGcAcac•u•u | 22 |
| S18 | g•u•guGcACUucgcuucacY | 18 | u•Y•ugaAgCGaaguGcAcac•u•u | 22 |
| S19 | g•Y•guGcACUucgcuucaca | 1 | u•G•YgaAgCGaaguGcAcac•u•u | 23 |
| S20 | g•u•YuGeACUucgcuucaca | 2 | u•G•YgaAgCGaaguGcAcac•u•u | 23 |
| S21 | g•u•gYGcACUucgcuucaca | 3 | u•G•YgaAgCGaaguGcAcac•u•u | 23 |
| S22 | g•u•guYcACUucgcuucaca | 4 | u•G•YgaAgCGaaguGcAcac•u•u | 23 |
| S23 | g•u•guGYACUucgcuucaca | 5 | u•G•YgaAgCGaaguGcAcac•u•u | 23 |
| S24 | g•u•guGcYCUucgcuucaca | 6 | u•G•YgaAgCGaaguGcAcac•u•u | 23 |
| S25 | g•u•guGcAYUucgcuucaca | 7 | u•G•YgaAgCGaaguGcAcac•u•u | 23 |
| S26 | g•u•guGcACYucgcuucaca | 8 | u•G•YgaAgCGaaguGcAcac•u•u | 23 |
| S27 | g•u•guGcACUYcgcuucaca | 9 | u•G•YgaAgCGaaguGcAcac•u•u | 23 |
| S28 | g•u•guGcACUuYgcuucaca | 10 | u•G•YgaAgCGaaguGcAcac•u•u | 23 |
| S29 | g•u•guGcACUucYcuucaca | 11 | u•G•YgaAgCGaaguGcAcac•u•u | 23 |
| S30 | g•u•guGcACUucgYuucaca | 12 | u•G•YgaAgCGaaguGcAcac•u•u | 23 |
| S31 | g•u•guGcACUucgcYucaca | 13 | u•G•YgaAgCGaaguGcAcac•u•u | 23 |
| S32 | g•u•guGcACUucgcuYcaca | 14 | u•G•YgaAgCGaaguGcAcac•u•u | 23 |
| S33 | g•u•guGcACUucgcuuYaca | 15 | u•G•YgaAgCGaaguGcAcac•u•u | 23 |
| S34 | g•u•guGcACUucgcuucYca | 16 | u•G•YgaAgCGaaguGcAcac•u•u | 23 |
| S35 | g•u•guGcACUucgcuucaYa | 17 | u•G•YgaAgCGaaguGcAcac•u•u | 23 |
| S36 | g•u•guGcACUucgcuucacY | 18 | u•G•YgaAgCGaaguGcAcac•u•u | 23 |
| S37 | g•Y•guGcACUucgcuucaca | 1 | u•G•uYaAgCGaaguGcAcac•u•u | 24 |
| S38 | g•u•YuGcACUucgcuucaca | 2 | u•G•uYaAgCGaaguGcAcac•u•u | 24 |
| S39 | g•u•gYGcACUucgcuucaca | 3 | u•G•uYaAgCGaaguGcAcac•u•u | 24 |
| S40 | g•u•guYcACUucgcuucaca | 4 | u•G•uYaAgCGaaguGcAcac•u•u | 24 |
| S41 | g•u•guGYACUucgcuucaca | 5 | u•G•uYaAgCGaaguGcAcac•u•u | 24 |
| S42 | g•u•guGcYCUucgcuucaca | 6 | u•G•uYaAgCGaaguGcAcac•u•u | 24 |
| S43 | g•u•guGcAYUucgcuucaca | 7 | u•G•uYaAgCGaaguGcAcac•u•u | 24 |
| S44 | g•u•guGcACYucgcuucaca | 8 | u•G•uYaAgCGaaguGcAcac•u•u | 24 |
| S45 | g•u•guGcACUYcgcuucaca | 9 | u•G•uYaAgCGaaguGcAcac•u•u | 24 |
| S46 | g•u•guGcACUuYgcuucaca | 10 | u•G•uYaAgCGaaguGcAcac•u•u | 24 |
| S47 | g•u•guGcACUucYcuucaca | 11 | u•G•uYaAgCGaaguGcAcac•u•u | 24 |
| S48 | g•u•guGcACUucgYuucaca | 12 | u•G•uYaAgCGaaguGcAcac•u•u | 24 |
| S49 | g•u•guGcACUucgcYucaca | 13 | u•G•uYaAgCGaaguGcAcac•u•u | 24 |
| S50 | g•u•guGcACUucgcuYcaca | 14 | u•G•uYaAgCGaaguGcAcac•u•u | 24 |
| S51 | g•u•guGcACUucgcuucYca | 16 | u•G•uYaAgCGaaguGcAcac•u•u | 24 |
| S52 | g•u•guGcACUucgcuucaYa | 17 | u•G•uYaAgCGaaguGcAcac•u•u | 24 |
| S53 | g•u•guGcACUucgcuucacY | 18 | u•G•uYaAgCGaaguGcAcac•u•u | 24 |
| S54 | g•Y•guGcACUucgcuucaca | 1 | u•G•ugYAgCGaaguGcAcac•u•u | 25 |
| S55 | g•u•YuGcACUucgcuucaca | 2 | u•G•ugYAgCGaaguGcAcac•u•u | 25 |
| S56 | g•u•gYGcACUucgcuucaca | 3 | u•G•ugYAgCGaaguGcAcac•u•u | 25 |
| S57 | g•u•guYcACUucgcuucaca | 4 | u•G•ugYAgCGaaguGcAcac•u•u | 25 |
| S58 | g•u•guGYACUucgcuucaca | 5 | u•G•ugYAgCGaaguGcAcac•u•u | 25 |
| S59 | g•u•guGcYCUucgcuucaca | 6 | u•G•ugYAgCGaaguGcAcac•u•u | 25 |
| S60 | g•u•guGcAYUucgcuucaca | 7 | u•G•ugYAgCGaaguGcAcac•u•u | 25 |
| S61 | g•u•guGcACYucgcuucaca | 8 | u•G•ugYAgCGaaguGcAcac•u•u | 25 |
| S62 | g•u•guGcACUYcgcuucaca | 9 | u•G•ugYAgCGaaguGcAcac•u•u | 25 |
| S63 | g•u•guGcACUuYgcuucaca | 10 | u•G•ugYAgCGaaguGcAcac•u•u | 25 |
| S64 | g•u•guGcACUucYcuucaca | 11 | u•G•ugYAgCGaaguGcAcac•u•u | 25 |
| S65 | g•u•guGcACUucgYuucaca | 12 | u•G•ugYAgCGaaguGcAcac•u•u | 25 |
| S66 | g•u•guGcACUucgcYucaca | 13 | u•G•ugYAgCGaaguGcAcac•u•u | 25 |
| S67 | g•u•guGcACUucgcuuYaca | 15 | u•G•ugYAgCGaaguGcAcac•u•u | 25 |
| S68 | g•u•guGcACUucgcuucYca | 16 | u•G•ugYAgCGaaguGcAcac•u•u | 25 |
| S69 | g•u•guGcACUucgcuucaYa | 17 | u•G•ugYAgCGaaguGcAcac•u•u | 25 |
| S70 | g•u•guGcACUucgcuucacY | 18 | u•G•ugYAgCGaaguGcAcac•u•u | 25 |
| S71 | g•Y•guGcACUucgcuucaca | 1 | u•G•ugaYgCGaaguGcAcac•u•u | 26 |
| S72 | g•u•YuGcACUucgcuucaca | 2 | u•G•ugaYgCGaaguGcAcac•u•u | 26 |
| S73 | g•u•gYGcACUucgcuucaca | 3 | u•G•ugaYgCGaaguGcAcac•u•u | 26 |
| S74 | g•u•guYcACUucgcuucaca | 4 | u•G•ugaYgCGaaguGcAcac•u•u | 26 |
| S75 | g•u•guGYACUucgcuucaca | 5 | u•G•ugaYgCGaaguGcAcac•u•u | 26 |
| S76 | g•u•guGcYCUucgcuucaca | 6 | u•G•ugaYgCGaaguGcAcac•u•u | 26 |
| S77 | g•u•guGcAYUucgcuucaca | 7 | u•G•ugaYgCGaaguGcAcac•u•u | 26 |
| S78 | g•u•guGcACYucgcuucaca | 8 | u•G•ugaYgCGaaguGcAcac•u•u | 26 |
| S79 | g•u•guGcACUYcgcuucaca | 9 | u•G•ugaYgCGaaguGcAcac•u•u | 26 |
| S80 | g•u•guGcACUuYgcuucaca | 10 | u•G•ugaYgCGaaguGcAcac•u•u | 26 |
| S81 | g•u•guGcACUucYcuucaca | 11 | u•G•ugaYgCGaaguGcAcac•u•u | 26 |
| S82 | g•u•guGcACUucgYuucaca | 12 | u•G•ugaYgCGaaguGcAcac•u•u | 26 |
| S83 | g•u•guGcACUucgcYucaca | 13 | u•G•ugaYgCGaaguGcAcac•u•u | 26 |
| S84 | g•u•guGcACUucgcuYcaca | 14 | u•G•ugaYgCGaaguGcAcac•u•u | 26 |
| S85 | g•u•guGcACUucgcuuYaca | 15 | u•G•ugaYgCGaaguGcAcac•u•u | 26 |
| S86 | g•u•guGcACUucgcuucYca | 16 | u•G•ugaYgCGaaguGcAcac•u•u | 26 |
| S87 | g•u•guGcACUucgcuucaYa | 17 | u•G•ugaYgCGaaguGcAcac•u•u | 26 |
| S88 | g•u•guGcACUucgcuucacY | 18 | u•G•ugaYgCGaaguGcAcac•u•u | 26 |
| S89 | g•Y•guGcACUucgcuucaca | 1 | u•G•ugaAYCGaaguGcAcac•u•u | 27 |
| S90 | g•u•YuGcACUucgcuucaca | 2 | u•G•ugaAYCGaaguGcAcac•u•u | 27 |
| S91 | g•u•gYGcACUucgcuucaca | 3 | u•G•ugaAYCGaaguGcAcac•u•u | 27 |
| S92 | g•u•guYcACUucgcuucaca | 4 | u•G•ugaAYCGaaguGcAcac•u•u | 27 |
| S93 | g•u•guGYACUucgcuucaca | 5 | u•G•ugaAYCGaaguGcAcac•u•u | 27 |
| S94 | g•u•guGcYCUucgcuucaca | 6 | u•G•ugaAYCGaaguGcAcac•u•u | 27 |
| S95 | g•u•guGcAYUucgcuucaca | 7 | u•G•ugaAYCGaaguGcAcac•u•u | 27 |
| S96 | g•u•guGcACYucgcuucaca | 8 | u•G•ugaAYCGaaguGcAcac•u•u | 27 |
| S97 | g•u•guGcACUYcgcuucaca | 9 | u•G•ugaAYCGaaguGcAcac•u•u | 27 |
| S98 | g•u•guGcACUuYgcuucaca | 10 | u•G•ugaAYCGaaguGcAcac•u•u | 27 |
| S99 | g•u•guGcACUucYcuucaca | 11 | u•G•ugaAYCGaaguGcAcac•u•u | 27 |
| S100 | g•u•guGcACUucgYuucaca | 12 | u•G•ugaAYCGaaguGcAcac•u•u | 27 |
| S101 | g•u•guGcACUucgcYucaca | 13 | u•G•ugaAYCGaaguGcAcac•u•u | 27 |
| S102 | g•u•guGcACUucgcuYcaca | 14 | u•G•ugaAYCGaaguGcAcac•u•u | 27 |
| S103 | g•u•guGcACUucgcuuYaca | 15 | u•G•ugaAYCGaaguGcAcac•u•u | 27 |
| S104 | g•u•guGcACUucgcuucYca | 16 | u•G•ugaAYCGaaguGcAcac•u•u | 27 |
| S105 | g•u•guGcACUucgcuucaYa | 17 | u•G•ugaAYCGaaguGcAcac•u•u | 27 |
| S106 | g•u•guGcACUucgcuucacY | 18 | u•G•ugaAYCGaaguGcAcac•u•u | 27 |
| S107 | g•Y•guGcACUucgcuucaca | 1 | u•G•ugaAgYGaaguGcAcac•u•u | 28 |
| S108 | g•u•YuGcACUucgcuucaca | 2 | u•G•ugaAgYGaaguGcAcac•u•u | 28 |
| S109 | g•u•gYGcACUucgcuucaca | 3 | u•G•ugaAgYGaaguGcAcac•u•u | 28 |
| S110 | g•u•guYcACUucgcuucaca | 4 | u•G•ugaAgYGaaguGcAcac•u•u | 28 |
| S111 | g•u•guGYACUucgcuucaca | 5 | u•G•ugaAgYGaaguGcAcac•u•u | 28 |
| S112 | g•u•guGcYCUucgcuucaca | 6 | u•G•ugaAgYGaaguGcAcac•u•u | 28 |
| S113 | g•u•guGcAYUucgcuucaca | 7 | u•G•ugaAgYGaaguGcAcac•u•u | 28 |
| S114 | g•u•guGcACYucgcuucaca | 8 | u•G•ugaAgYGaaguGcAcac•u•u | 28 |
| S115 | g•u•guGcACUYcgcuucaca | 9 | u•G•ugaAgYGaaguGcAcac•u•u | 28 |
| S116 | g•u•guGcACUuYgcuucaca | 10 | u•G•ugaAgYGaaguGcAcac•u•u | 28 |
| S117 | g•u•guGcACUucgYuucaca | 12 | u•G•ugaAgYGaaguGcAcac•u•u | 28 |
| S118 | g•u•guGcACUucgcYucaca | 13 | u•G•ugaAgYGaaguGcAcac•u•u | 28 |
| S119 | g•u•guGcACUucgcuYcaca | 14 | u•G•ugaAgYGaaguGcAcac•u•u | 28 |
| S120 | g•u•guGcACUucgcuuYaca | 15 | u•G•ugaAgYGaaguGcAcac•u•u | 28 |
| S121 | g•u•guGcACUucgcuucYca | 16 | u•G•ugaAgYGaaguGcAcac•u•u | 28 |
| S122 | g•u•guGcACUucgcuucaYa | 17 | u•G•ugaAgYGaaguGcAcac•u•u | 28 |
| S123 | g•u•guGcACUucgcuucacY | 18 | u•G•ugaAgYGaaguGcAcac•u•u | 28 |
| S124 | g•u•guGcACUucgcuucaca | 19 | u•Y•ugaAgCGaaguGcAcac•u•u | 22 |
| S125 | g•u•guGcACUucgcuucaca | 19 | u•G•YgaAgCGaaguGcAcac•u•u | 23 |
| S126 | g•u•guGcACUucgcuucaca | 19 | u•G•uYaAgCGaaguGcAcac•u•u | 24 |
| S127 | g•u•guGcACUucgcuucaca | 19 | u•G•ugYAgCGaaguGcAcac•u•u | 25 |
| S128 | g•u•guGcACUucgcuucaca | 19 | u•G•ugaYgCGaaguGcAcac•u•u | 26 |
| S129 | g•u•guGcACUucgcuucaca | 19 | u•G•ugaAYCGaaguGcAcac•u•u | 27 |
| S130 | g•u•guGcACUucgcuucaca | 19 | u•G•ugaAgYGaaguGcAcac•u•u | 28 |
| S131 | g•u•guGcACUucgcuucaca | 19 | u•G•ugaAgCGYaguGcAcac•u•u | 29 |
| S132 | g•u•guGcACUucgcuucaca | 19 | u•G•ugaAgCGaYguGcAcac•u•u | 30 |
| S133 | g•u•guGcACUucgcuucaca | 19 | u•G•ugaAgCGaaguGcYcac•u•u | 31 |
| S134 | g•u•guGcACUucgcuucaca | 19 | u•G•ugaAgCGaaguGcAcYc•u•u | 32 |
| S135 | g•u•guGcACUucgcuucaca | 19 | u●G●uga(Agn)gCGYaguGcAcac●u ●u | 38 |
| S136 | g•u•guGcACUucgcuucaca | 19 | u●G●uga(Agn)gCGaYguGcAcac●u ●u | 39 |
| S137 | g•u•guGcACUucgcuucaca | 19 | u●G●uga(Agn)gCGaaguGcYcac●u ●u | 34 |
| S138 | g•u•guGcACUucgcuucaca | 19 | u•G•uga(Agn)gCGaaguGcAcYc•u •u | 35 |
| S139 | g•u•guGcYCUucgcuucaca | 6 | u•G•ugaAgCGaaguGcAcac•u•u | 36 |
| S140 | g•u•guGcACUucgcuucYca | 16 | u•G•ugaAgCGaaguGcAcac•u•u | 36 |
| S141 | g•u•guGcACUucgcuucacY | 18 | u•G•ugaAgCGaaguGcAcac•u•u | 36 |
| S142 | g•u•guGcACUucgcuucYca | 16 | u•G•ugaAYCGaaguGcAcac•u•u | 27 |
| S143 | g•u•guGcACUucgcuucYca | 16 | u•G•ugaYYCGaaguGcAcac•u•u | 37 |

| **Numbers of the double-stranded siRNA** | **Sense strand sequence (5'-3')-conjugation group** | **SEQ ID NO** | **Antisense strand sequence (5'-3')** | **SEQ ID NO** |
|---|---|---|---|---|
| S146 | g●u●guGcACUucgcuucYca-D AW40007-4 | 21 | u•G•ugaAYCGaaguGcAcac•u•u | 27 |
| S147 | g●u●guGcACUucgcuuYaca-D AW40007-4 | 41 | u•G•ugaAYCGaaguGcAcac•u•u | 27 |
| S150 | g●u●guGcACUucgcuucaYa-D AW40007-4 | 44 | u•G•ugaYgCGaaguGcAcac•u•u | 26 |

Wherein, capital letters C, G, U and A represent the base composition of natural nucleotides; lowercase letters c, g, u and a respectively represent that the 2-position of the ribose on the nucleotide represented by the corresponding capital letter is modified by methoxy; an underline under a capital letter represents that the 2-position of the ribose on the nucleotide represented by the letter is modified by fluorine; the middle dot **"•"** represents that there is phosphorothioate linkage between two nucleotide residues adjacent to the left and right sides of the middle dot "•"; Agn is adenosine-glycol nucleic acid (Glycol nucleic acid, GNA,); Y in double-stranded siRNA represents

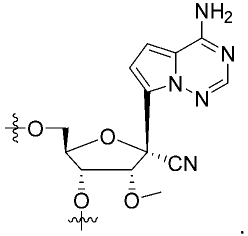

In some embodiments of the double-stranded siRNA, conjugate or salt thereof of the present invention, the double-stranded siRNA conjugate comprises the double-stranded siRNA and a conjugation group, that is, the double-stranded siRNA conjugate is a conjugate of the double-stranded siRNA conjugated to the conjugation group, wherein the conjugation group includes but is not limited to L-96 or DAW40007-4, and the structures of L-96 and DAW40007-4 are respectively:

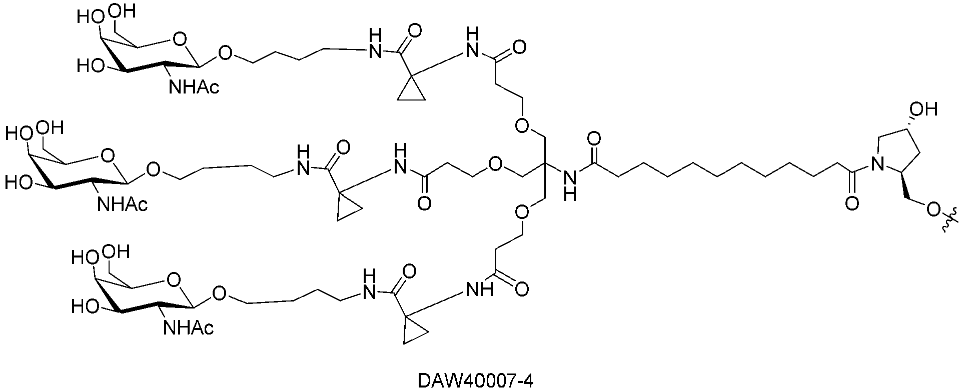

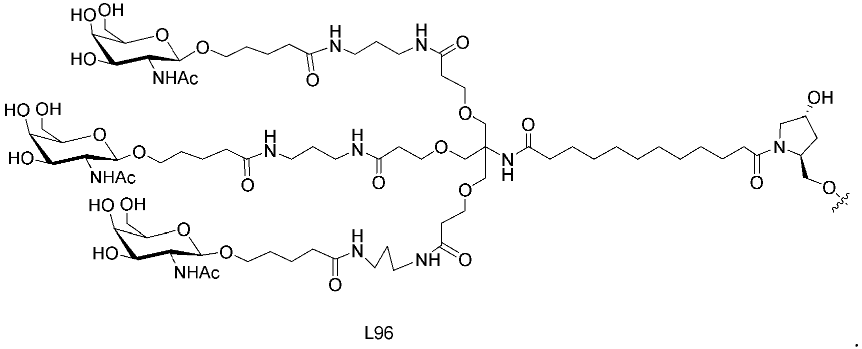

In some embodiments of the double-stranded siRNA, conjugate or salt thereof of the present invention, the conjugated group is conjugated to the 3' end or the 5' end of the sense strand of the double-stranded siRNA.

In some embodiments of the double-stranded siRNA, conjugate or salt thereof of the present invention, the conjugated group is conjugated to the 3' end of the sense strand of the double-stranded siRNA.

In some embodiments of the double-stranded siRNA, conjugate or salt thereof of the present invention, the double-stranded siRNA is linked to the conjugated group through a phosphate eater group, phosphorothioate group or a phosphate group.

In some embodiments of the double-stranded siRNA, conjugate or salt thereof of the present invention, the 3' end or 5' end of the sense strand of the double-stranded siRNA is linked to the conjugated group through a phosphate eater group, phosphorothioate group or a phosphate group.

In another aspect, the present invention also relates to a nucleotide residue Y having structure

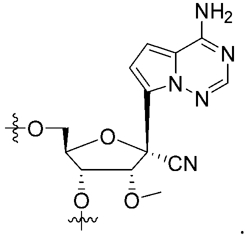

In another aspect, the present invention also relates to a compound having Formula (I) or a stereoisomer, a tautomer or an acceptable salt of the compound having Formula (I), 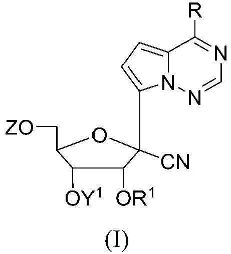 wherein, each of R, Z, Y¹ and R¹ is as defined herein.

In some embodiments of the compounds described herein, R¹ is methyl, ethyl, *n*-propyl, *i*-propyl, *n*-butyl, *i*-butyl or *tert*-butyl, each of the methyl, ethyl, *n*-propyl, *i*-propyl, *n*-butyl, *i*-butyl or *tert*-butyl is independently optionally substituted with 1, 2, 3 or 4 substituents selected from deuterium, hydroxyl, cyano, F, Cl, Br, I, methoxy, ethoxy, 1-propoxy and 2-propoxy.

In some embodiments of the compounds described herein, Z and Y¹ are each independently H, deuterium, a hydroxyl protecting group, HOC(=O)(CH₂)ⱼC(=O)-, M-C(=O)(CH₂)ⱼC(=O)-, a phosphate ester group, a phosphorothioate group, a phosphoramidite group or a hydrogen phosphate group, wherein, each of j and M is as defined herein.

Any Y¹ group that can enable the compounds described in the present invention to participate in oligonucleotide synthesis belongs to the present invention, such as active phosphate groups such as phosphate ester groups, phosphorothioate groups, phosphoramidite groups or hydrogen phosphate groups. They all belong to the content of the present invention. They can be obtained by the compounds described in the present invention through the preparation methods disclosed in the art, such as the phosphodiester method, phosphotriester method, and hydrogen phosphate method described in Section 3 of the master's thesis "Chemical Synthesis Research of Nucleic Acids and Synthesis and Interference Activity Test of 4'-C-Hydroxy Modified siRNA" to obtain the compounds of the present application, or by the method described in the document Kyle W. Knouse et al. "Unlocking P(V): Reagents for chiral phosphorothioate synthesis" 2018. pages 6-9.

The phosphate ester described in the present invention include phosphate diester and phosphate triester; the phosphorothioate described include phosphate diester and phosphate triester; and the hydrogen phosphate group refers to the group formed by the salt formed by the phosphoric acid group and the base.

In some embodiments of the compounds described herein, M is a solid support, preferably a hydroxyl- or amino-functionalized solid support, more preferably a resin or CPG, further preferably a macroporous resin or CPG, further preferably an aminomethyl resin, a hydroxyl resin or -NHCPG;

In some embodiments of the compounds described herein, R is -NHR² or -N=CH-NR^{a}R^{b}; wherein each of R², R^{a} and R^{b} is as defined herein.

In some embodiments of the compounds described herein, R² is an amino protecting group.

In some embodiments of the compounds described herein, each of R^{a} and R^{b} is independently H or an amino protecting group, or R^{a}, R^{b} and the nitrogen atom to which they are attached form a heterocyclic group consisting of 5-6 ring atoms, each of heterocyclic group consisting of 5-6 ring atoms is independently optionally substituted with 1, 2, 3 or 4 substituents selected from deuterium, hydroxyl, cyano, F, Cl, Br, I, methyl, ethyl, *n*-propyl, isopropyl, methoxy, ethoxy, 1-propoxy and 2-propoxy.

In some embodiments of the compounds described herein, each j is independently 1, 2, 3, 4 or 5.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, C₁₋₁₂ alkyl, C₁₋₁₂ alkylC(=O)-, C₁₋₁₂ alkylsilyl, C₆₋₁₀ arylC₁₋₆ alkyl, HOC(=O)(CH₂)ⱼC(=O)-, M-C(=O)(CH₂)ⱼC(=O)-, triphenylmethyl, MMTr, DMTr, 4',4',4'-trimethoxytrityl, 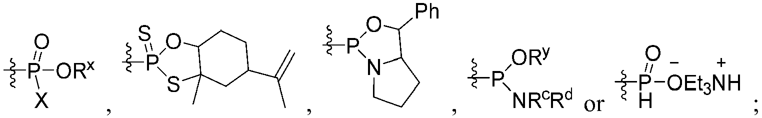 wherein, each j, M, X, R^{x}, R^{y}, R^{c} and R^{d} are as defined herein.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, C₁₋₁₀ alkyl, C₁₋₁₀ alkylC(=O)-, C₁₋₁₀ alkylsilyl, C₆₋₁₀ arylC₁₋₄ alkyl, HOC(=O)(CH₂)ⱼC(=O)-, M-C(=O)(CH₂)jC(=O)-, triphenylmethyl, MMTr, DMTr, 4',4',4'-trimethoxytrityl, 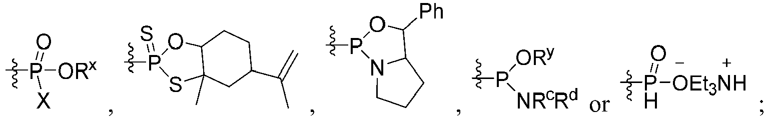 wherein, each j, M, X, R^{x}, R^{y}, R^{c} and R^{d} are as defined herein.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₈ alkyl, C₁₋₈ alkylC(=O)-, C₁₋₈ alkylsilyl, phenylC₁₋₃ alkyl, HOC(=O)(CH₂)ⱼC(=O)-, M-C(=O)(CH₂)ⱼC(=O)-, triphenylmethyl, MMTr, DMTr, 4',4',4'-trimethoxytrityl, 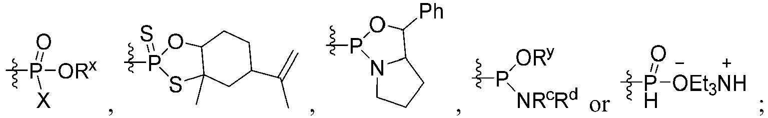 wherein, each j, M, X, R^{x}, R^{y}, R^{c} and R^{d} are as defined herein.

In some embodiments of the compounds described herein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, C₁₋₆ alkyl, C₁₋₆ alkylC(=O)-, C₁₋₆ alkylsilyl, benzyl, phenethyl, HOC(=O)(CH₂)ⱼC(=O)-, M-C(=O)(CH₂)jC(=O)-, triphenylmethyl, MMTr, DMTr, 4',4',4'-trimethoxytrityl, 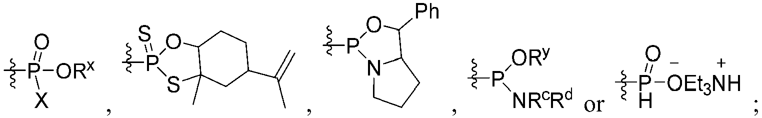 wherein, each j, M, X, R^{x}, R^{y}, R^{c} and R^{d} are as defined herein.

In some embodiments of the compounds described herein, X is Cl or Br.

In some embodiments of the compounds described herein, each of R^{x} and R^{y} is independently a hydroxy protecting group.

In some embodiments of the compounds described herein, each of R^{c} and R^{d} is independently H or an amino protecting group; or R^{c}, R^{d} and the nitrogen atom to which they are attached form a heterocyclic group consisting of 5-6 ring atoms, the heterocyclic group consisting of 5-6 ring atoms is independently optionally substituted with 1, 2, 3 or 4 substituents selected from deuterium, hydroxyl, cyano, F, Cl, Br, I, methyl, ethyl, propyl, methoxy, ethoxy, 1-propoxy and 2-propoxy.

In some embodiments of the compounds described herein, R² is 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, *tert*-butyl*,* 2-trimethylsilylethyl, 1,1-dimethyl-2-propenyl, 3-methyl-3-butenyl, allyl, benzyl, *p*-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl, C₁₋₆ alkoxy or R³C(=O)-, wherein, R³ is as defined herein.

In some embodiments of the compounds described herein, each of R^{a} and R^{b} is independently H, methyl, ethyl, *n*-propyl, *i*-propyl, *n*-butyl, *i*-butyl, *tert*-butyl*,* methoxy, ethoxy, 1-propoxy, 2-propoxy, 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl or R⁴C(=O)-, or R^{a}, R^{b} and the N atom to which they are attached form a pyrrolidinyl, morpholinyl, piperidinyl and piperazinyl, each of pyrrolidinyl, morpholinyl, piperidinyl or piperazinyl is independently optionally substituted with 1, 2, 3 or 4 substituents selected from deuterium, hydroxyl, cyano, F, Cl, Br, I, methoxy, ethoxy, 1-propoxy and 2-propoxy, wherein, R⁴ is as defined herein.

In some embodiments of the compounds described herein, each of R³ and R⁴ is independently C₁₋₆ alkyl, halo C₁₋₆ alkyl, C₁₋₆ alkoxy, phenyl, halo-substituted phenyl, C₁₋₆ alkylphenyl or benzyl.

In some embodiments of the compounds described herein, each of R³ and R⁴ is independently C₁₋₄ alkyl, halo C₁₋₄ alkyl, C₁₋₄ alkoxy, phenyl, halo-substituted phenyl, C₁₋₄ alkylphenyl or benzyl.

In some embodiments of the compounds described herein, each of R³ and R⁴ is independently methyl, ethyl, *n*-propyl, isopropyl, trifluoromethyl, difluoromethyl, monofluoromethyl, trichloromethyl, dichloromethyl, monochloromethyl, 2,2,2-trichloroethyl, methoxy, ethoxy, 1-propoxy, 2-propoxy, 1-butoxy, 2-methyl-1-propoxy, 2-butoxy, *tert*-butoxy, phenyl, halogen-substituted phenyl, C₁₋₃ alkylphenyl or benzyl.

In some embodiments of the compounds described herein, each of R^{x} and R^{y} is independently benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, cyano C₁₋₆ alkyl, C₁₋₆ alkyl, C₁₋₆ alkyl C(=O)-, C₁₋₆ alkylsilyl, phenyl, halogen-substituted phenyl, benzyl or phenethyl.

In some embodiments of the compounds described herein, each of R^{x} and R^{y} is independently benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, *p*-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, CNCH₂-, CN(CH₂)₂-, CNCH(CH₃)₂CH₂-, CN(CH₂)₃-, CNCH(CH₃)₂CH₂CH₂-, methyl, ethyl, *n*-propyl, *i*-propyl, *n*-butyl, *i*-butyl, *tert*-butyl, C₁₋₄ alkyl C(=O)-, C₁₋₄ alkylsilyl, phenyl, halogen-substituted phenyl, benzyl or phenethyl.

In some embodiments of the compounds described herein, each of R^{c} and R^{d} is independently methyl, ethyl, *n*-propyl, *i*-propyl, *n*-butyl, *i*-butyl, *tert*-butyl, methoxy, ethoxy, 1-propoxy, 2-propoxy, 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2-trimethylsilylethyl, 1,1-dimethyl-2-propenyl, 3-methyl-3-butenyl, allyl, benzyl, *p*-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl or R⁴C(=O)-, or R^{c}, R^{d} and the N atom to which they are attached form a pyrrolidinyl, morpholinyl, piperidinyl or piperazinyl, each of pyrrolidinyl, morpholinyl, piperidinyl and piperazinyl is independently optionally substituted with 1, 2, 3 or 4 substituents selected from deuterium, hydroxyl, cyano, F, Cl, Br, I, methoxy, ethoxy, 1-propoxy and 2-propoxy.

In some embodiments of the compounds described herein, the compound of the present invention has a structure as shown in Formula (II),

In another aspect of the present invention, the present invention also relates to a pharmaceutical composition comprising the double-stranded siRNA, conjugate or salt thereof according to the present invention, and a pharmaceutically acceptable carrier.

In another aspect of the present invention, the present invention also relates to the use of the siRNA conjugate, conjugate thereof and the pharmaceutical composition of the present invention in the preparation of drugs for treating and/or preventing hepatitis B disease.

In another aspect of the present invention, the present invention also relates to the use of the nucleotide residues of the present invention in drug research, gene function research and/or screening of whole gene libraries for siRNA, and the structure of the nucleotide residues is:

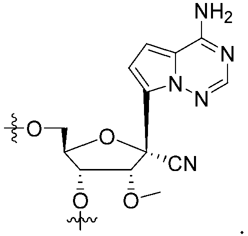

In some embodiments of the application of the nucleotide residues described in the present invention, the siRNA drug is the double-stranded siRNA drug described in the present invention.

In another aspect of the present invention, the present invention also relates to a use of the compound in raw materials for solid-phase synthesis of DNA nucleotides, raw materials for synthesis of oligonucleotide drugs, raw materials for synthesis of siRNA drugs, siRNA drug research, gene function research and/or screening of whole gene libraries.

In another aspect of the present invention, the present invention also relates to the use of the nucleotide residues described in the present invention as oligonucleotide embedding groups, wherein the oligonucleotide is a nucleotide sequence containing 10~50 nucleotides or nucleotide base pairs, and the oligonucleotide can inhibit or block gene expression, and the structure of the nucleotide residues is:

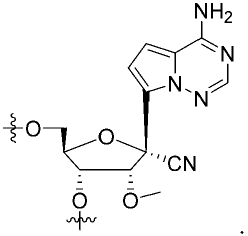

In some embodiments of the use of the nucleotide residue as an oligonucleotide embedding group in the present invention, wherein, the gene is the HBV gene.

In some embodiments of the use of the nucleotide residue as an oligonucleotide embedding group in the present invention, wherein, the oligonucleotide is siRNA.

In some embodiments of the use of the nucleotide residue as an oligonucleotide embedding group in the present invention, wherein, the siRNA comprises the sense strand and the antisense strand.

In some embodiments of the use of the nucleotide residue as an oligonucleotide embedding group in the present invention, wherein, the nucleotide residue is embedded only into the sense strand of the siRNA.

In some embodiments of the use of the nucleotide residue as an oligonucleotide embedding group in the present invention, wherein, the nucleotide residue is embedded only into the antisense strand of the siRNA.

In some embodiments of the use of the nucleotide residue as an oligonucleotide embedding group in the present invention, wherein, the nucleotide residue is embedded into the sense strand and the antisense strand of the siRNA.

In some embodiments of the use of the nucleotide residue as an oligonucleotide embedding group in the present invention, wherein, the sense strand of the siRNA comprises the sequence of SEQ ID NO: 19.

In some embodiments of the use of the nucleotide residue as an oligonucleotide embedding group in the present invention, wherein, the antisense strand of the siRNA comprises the sequence of SEQ ID NO: 36 or SEQ ID NO: 33.

In another aspect, the present invention also provides a method for inhibiting the expression of a specific gene in liver cells in a patient, which comprises administering to the patient the double-stranded siRNA and double-stranded siRNA conjugate of the present invention or a composition thereof, wherein the nucleic acid ligand conjugate or the composition thereof may be a therapeutically effective amount.

In another aspect, the double-stranded siRNA conjugate, conjugate thereof and salt thereof, or the pharmaceutical composition of the present invention is used for treating and/or preventing hepatitis B disease.

In another aspect, the compounds of the present invention are used as raw materials for solid-phase synthesis of DNA nucleotides, raw materials for synthesis of oligonucleotide drugs, raw materials for synthesis of siRNA drugs, siRNA drug research, gene function research and/or screening of whole gene libraries.

In another aspect, the nucleotide residue of the present invention is used as an oligonucleotide embedding group, wherein the oligonucleotide is a nucleotide sequence containing 10 to 50 nucleotides or nucleotide base pairs, and the oligonucleotide can inhibit or block gene expression.

In some embodiments, the gene of the present invention is the HBV gene.

In some embodiments, the oligonucleotide is siRNA.

In some embodiments, the siRNA comprises the sense strand and the antisense strand.

In some embo diments, the nucleotide residues are embedded only into the sense strand of the siRNA.

In some embodiments, the nucleotide residues are embedded only into the antisense strand of the siRNA.

In some embodiments, the nucleotide residues are embedded into the sense strand and the antisense strand of the siRNA.

In some embodiments, the sense strand of the siRNA comprises the sequence of SEQ ID NO: 19.

In some embodiments, the antisense strand of the siRNA comprises the sequence of SEQ ID NO: 36 or SEQ ID NO: 33.

In another aspect, the present invention relates to a method for treating and/or preventing hepatitis B, comprising using the double-stranded siRNA conjugate, its conjugate and salt thereof, or the pharmaceutical composition of the present invention.

In another aspect, the present invention relates to a method for solid-phase synthesis of DNA nucleotides, synthesis of oligonucleotide drugs, synthesis of siRNA drugs, siRNA drug research, gene function research and/or screening of a whole gene library, which method comprises using the compound described in the present invention.

In another aspect, the present invention relates to a method for inhibiting or blocking gene expression, comprising using a nucleotide sequence comprising 10~50 nucleotides or nucleotide base pairs, wherein the nucleotide sequence has embedded the nucleotide residues of the present inventiontherein.

In some embodiments, the gene is the HBV gene.

In some embodiments, the oligonucleotide is siRNA.

In some embodiments, the siRNA comprises the sense strand and the antisense strand.

In some embo diments, the nucleotide residues are embedded only into the sense strand of the siRNA.

In some embodiments, the nucleotide residues are embedded only into the antisense strand of the siRNA.

In some embodiments, the nucleotide residues are embedded into the sense strand and the antisense strand of the siRNA.

In some embodiments, the sense strand of the siRNA comprises the sequence of SEQ ID NO: 19.

In some embodiments, the antisense strand of the siRNA comprises the sequence of SEQ ID NO: 36 or SEQ ID NO: 33.

### Nucleic acid conjugates, preparations, administration and disease treatment methods of the present invention

The effective amount of the nucleic acid conjugate (eg, siRNA conjugate or pharmaceutical composition) of the present invention may vary depending on the mode of administration and the severity of the disease to be treated. The selection of the preferred effective amount can be determined by those skilled in the art based on various factors (eg, through clinical trials). The factors include, but are not limited to: pharmacokinetic parameters of the active ingredient such as bioavailability, metabolism, half-life, etc.; severity of the disease to be treated, weight of the patient, immune status of the patient, route of administration, etc. For example, several divided doses may be administered daily, such as four times a day, three times a day, twice a day, once a day, or every other day, or the several divided doses administered daily may be proportionally reduced, as required by the exigencies of the therapeutic situation.

The drug can be administered to a subject by any suitable route known in the art, including but not limited to oral or parenteral routes, including intravenous administration, intramuscular administration, subcutaneous administration, transdermal administration, airway administration (aerosol), pulmonary administration, nasal administration, rectal administration and topical administration (including buccal administration and sublingual administration), preferably intravenous administration.

The pharmaceutical compositions disclosed herein include formulations suitable for parenteral administration. The formulations may conveniently be presented in unit dosage form and may be prepared by any methods well known in the art of pharmacy. The amount of active ingredient that can be combined with auxiliary material to produce a single dosage form will generally be that amount of the siRNA that produces a therapeutic effect. Generally, out of one hundred percent, this amount will range from about 1% to about 99% of active ingredient, preferably from about 5% to about 70%, most preferably from about 10% to about 30%.

In another aspect of the present invention, a method for inhibiting the expression or activity of hepatitis B virus genes is provided. According to an embodiment of the present invention, the method includes: contacting the aforementioned siRNA, the aforementioned siRNA conjugate, or the aforementioned pharmaceutical composition with cells. As mentioned above, the aforementioned siRNA and siRNA conjugates can degrade hepatitis B virus mRNA and inhibit the expression and replication of HBV. Therefore, the method of the present invention can be used to inhibit the expression or activity of hepatitis B virus genes. For example, the expression or activity of the hepatitis B virus gene can be inhibited in an animal body; or the expression or activity of the hepatitis B virus gene can be inhibited for non-disease treatment purposes, such as inhibiting the expression or activity of the hepatitis B virus gene in vitro for subsequent research.

In another aspect of the present invention, the present invention provides a method for preventing and/or treating hepatitis B virus-induced hepatitis. According to an embodiment of the present invention, the method comprises: administering a pharmaceutically acceptable amount of the aforementioned siRNA, the aforementioned siRNA conjugate, or the aforementioned pharmaceutical composition to a subject. According to an embodiment of the present invention, the method can effectively prevent and/or treat related diseases caused by hepatitis B virus.

### General synthesis methods of the compounds, double-stranded siRNA, and double-stranded siRNA conjugates of the present invention

Generally, the compounds and nucleic acid conjugates disclosed herein may be prepared by methods described herein, wherein the substituents are as defined for Formula (I) and Formula (II), except where further noted. The following non-limiting schemes and examples are presented to further exemplify the invention.

In the examples described below, unless otherwise indicated all temperatures are set forth in degrees Celsius (°C). Column chromatography was conducted using a silica gel column. Silica gel (200 - 300 mesh) was purchased from Qingdao Ocean Chemical Factory, and NH₂CPG was purchased from Hebei Dinaxingke. 1H NMR spectra were obtained by using CDCl₃, DMSO-*d₆*, CD₃OD or acetone-*d₆* solutions (in ppm), with TMS (0 ppm) or chloroform (7.25 ppm) as the reference standard. When peak multiplicities were reported, the following abbreviations were used: s (singlet), d (doublet), t (triplet), m (multiplet), br (broadened), dd (doublet of doublets), dt (doublet of triplets), br.s (broadened singlet), q (quartet). Coupling constant *J*, when given, were reported in Hertz (Hz).

Low-resolution mass spectral (MS) data were also determined on an Agilent 6320 series LC-MS spectrometer equipped with G1312A binary pumps, a G1316A TCC (Temperature Control of Column, maintained at 30 °C), a G1329A autosampler and a G1315B DAD detector were used in the analysis. An ESI source was used on the LC-MS spectrometer.

High-resolution mass spectral (MS) data were also determined on an Agilent 6130 series LC-MS spectrometer equipped with G1311A quaternary pumps, a G1316A TCC (Temperature Control of Column, maintained at 30 °C), a G1329A autosampler and a G1315D DAD detector were used in the analysis. An ESI source was used on the HR-MS spectrometer.

The following abbreviations are used throughout the specification:

| | | | |
|---|---|---|---|
| EDCI | 1-ethyl-3(3-dimethylpropylamine)carbodiimide | EDCI | 1-(3-dimethylaminopropyl)-3-ethylcarbodiimide |
| HOBT | 1-hydroxybenzotriazole | EA | ethyl acetate |
| TFA | trifluoroacetic acid | HOBT | 1-hydroxybenzotriazole |
| Py | Pyridine | DMAP | 4-Dimethylaminopyridine |
| PE | petroleum ether | MsCl | Methanesulfonyl chloride |
| ACN | acetonitrile | TMSOTf | Trimethylsilyl trifluoromethanesulfonate |
| Ac₂O | Acetic anhydride | | |
| Boc | tert-butoxycarbonyl | THF | tetrahydrofuran |
| MeOH | methanol | mL | milliliter |
| DMSO | dimethylsulfoxide | min | minute, minutes |
| DMF | *N,N-dimethylformamide* | M, N, | mol/L mole/liter |
| DCM | dichloromethane | h | hour, hours |
| DIPEA, | *N,N*-diisopropylethylamine | RT, rt | room temperature |
| HBTU | Benzotriazole-*N,N,N',N'*-tetramethyluronium hexafluorophosphate | | |

### EXAMPLES

The solutions of the present invention will be explained below with reference to the embodiments. Those skilled in the art will understand that the following embodiments are only used to illustrate the present invention and should not be considered to limit the scope of the present invention. In particular, the synthesis of small nucleic acids and nucleic acid conjugates can be synthesized according to the embodiments of the present invention or conventional adjustments in the art. If no specific techniques or conditions are specified in the examples, the techniques or conditions described in the literature in the art or the product instructions shall be followed. All reagents and instruments used without manufacturer indication are commercially available conventional products.

### Synthesis method of the compound of the present invention

The following synthesis scheme lists the general experimental steps for preparing the compounds disclosed in the present invention. Those skilled in the art can make appropriate modifications to the method or adjustment of the raw materials according to actual conditions to prepare the compounds of the present invention. wherein, each of R¹ and R is as defined herein; wherein, Z is a hydroxyl protecting group, Y² is M-C(=O)(CH₂)ⱼC(=O)-, a phosphate ester group, a phosphorothioate group, a phosphoramidite group or a hydrogen phosphate group, wherein, M is as defined herein. Unless otherwise specified, the compounds of the present invention can be prepared by the methods described in the following synthetic schemes.

### Synthesis Scheme 1:

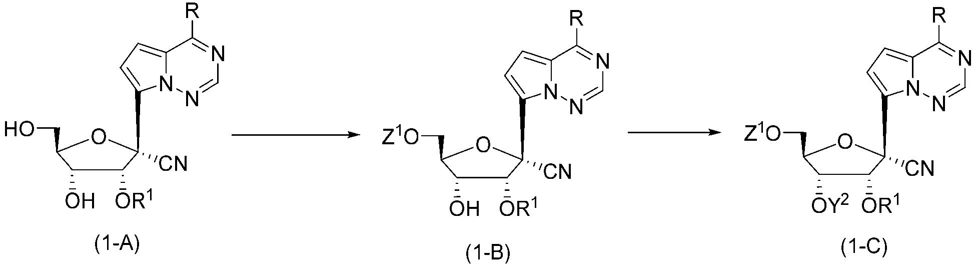

Compound (1-C) can be prepared by referring to the preparation method of Synthesis Scheme 1. First, compound (1-A) is protected with a hydroxyl protecting group to obtain compound (1-B). Compound (1-B) is then reacted with a suitable phosphate reagent or solid support to obtain compound (1-C) containing an active phosphate group or solid support. The stereoisomers of compound (1-C) can also be obtained by referring to the preparation method of Synthesis Scheme 1.

### Preparation Example

In the following preparation examples, the inventors take some of the compounds of the present invention as examples to describe in detail the preparation process of the compounds of the present invention, wherein, 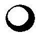 is CPG.

### Example 1: Synthesis of Monomer

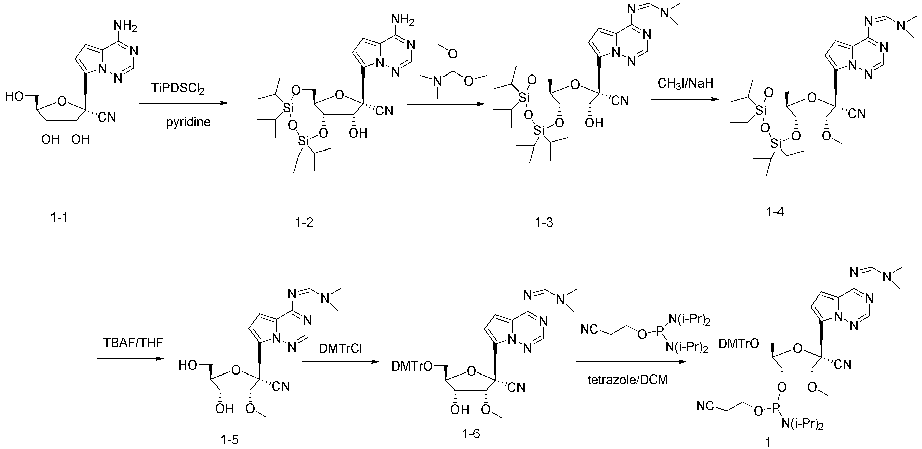

### Step 1: Synthesis of compound 1-2

(2*R*,3*R*,5*R*)-2-(4-Aminopyrrolo[2,1-*f*][1,2,4]triazin-7-yl)-3,4-dihydroxy-5-(hydroxymethyl)tetr ahydrofuran-2-carbonitrile (i.e., compound 1-1) (1.0 g, 3.43 mmol) was added to pyridine (15 mL). The mixture was protected by nitrogen and cooled to -5 °C. 1,3-Dichloro-1,1,3,3-tetraisopropyldisiloxane (1.13 mL, 3.53 mmol) was then added dropwise. After the addition was complete, the reaction mixture was heated to 25 °C and stirred for 20 h. The reaction solution was concentrated under reduced pressure to remove the solvent, and the residue was purified by silica gel column chromatography (eluent: PE/EA (v/v) = 2/3) to obtain white solid compound 1-2 (1.38 g, 75.3%). MS (ESI, pos. ion) m/z: 534.3 [M+H]⁺.

### Step 2: Synthesis of compound 1-3

Compound 1-2 (1.08 g, 2.02 mmol) and N,N-dimethylformamide dimethyl acetal (0.48 g, 4.04 mmol) were added to toluene (15 mL), and the mixture was heated to 50 °C and stirred for 4 h. After the reaction was completed, the mixture was concentrated under reduced pressure to obtain compound 1-3 as a white foamy solid (1.19 g, 100%). MS (ESI, pos. ion) m/z: 589.3 [M+H]⁺; ¹H NMR (599 MHz, DMSO-*d*₆) δ 8.95 (s, 1H), 8.15 (s, 1H), 6.90 (d, *J =* 4.4 Hz, 1H), 6.80 (d, *J=* 4.5 Hz, 1H), 6.49 (d, *J =* 5.7 Hz, 1H), 4.58 (dd, *J =* 5.7, 4.4 Hz, 1H), 4.23 - 4.12 (m, 3H), 3.92 (dd, *J =* 13.2, 2.5 Hz, 1H), 3.25 (s, 3H), 3.19 (s, 3H), 1.06 - 1.04 (m, 7H), 1.01 (dd, *J =* 7.2, 2.0 Hz, 6H), 0.96 (dd, *J =* 8.8, 7.0 Hz, 8H), 0.89 (t, *J=* 7.6 Hz, 7H).

### Step 3: Synthesis of compound 1-4

Compound 1-3 (0.84 g, 1.43 mmol) was added to N,N-dimethylformamide (10 mL), and the temperature was lowered to 0 °C. Then, iodomethane (0.41 g, 2.86 mmol) and sodium hydride (0.11 g, 2.86 mmol, 60%) were added in sequence, and the reaction was stirred at 0 °C for 20 min. After the reaction, the reaction solution was poured into a saturated NH₄Cl solution and extracted with EA. The organic phase was concentrated and the residue was purified by silica gel column chromatography (EA/PE (v/v) = 1/1) to obtain compound 1-4 as a white solid (0.71 g, 83%). MS (ESI, pos. ion) m/z: 603.40 [M+H]⁺.

### Step 4: Synthesis of compound 1-5

Compound 1-4 (5.1 g, 8.46 mmol), tetrahydrofuran (50 mL) and tetrabutylammonium fluoride solution in tetrahydrofuran (9.48 mL, 9.48 mmol, 1 M) were mixed, and the resulting mixture was stirred at room temperature. After completion of the reaction, the mixture was concentrated under reduced pressure. The resulting residue was purified by silica gel column chromatography (MeOH/DCM (v/v) = 1:20) to obtain compound 1-5 (2.4 g, 79%) as a white solid. MS (ESI, pos. ion) m/z: 361.3 [M+H]⁺.

### Step 5: Synthesis of compound 1-6

Compound 1-5 (2.4 g, 6.66 mmol), dichloromethane (50 mL), triethylamine (2.02 g, 19.98 mmol) and 4-dimethylaminopyridine (0.081 g, 0.67 mmol) were mixed. and then resulting mixture was cooled to 0 °C, then was added 4,4'-bismethoxytrityl chloride (2.71 g, 7.99 mmol) portionwise. The resulting reaction mixture was warmed to room temperature and stirred for 3 h. Then the temperature was lowered to 0 °C and 4,4'-bismethoxytrityl chloride (1.35 g) was added in two portions. The reaction mixture was stirred at 0 °C for 2 h. The reaction system was diluted with MeOH (1 mL) and DCM (100 mL), washed sequentially with saturated sodium bicarbonate solution and saturated sodium chloride solution. The organic phase was dried over anhydrous sodium sulfate and concentrated under reduced pressure. The resulting residue was purified on a silica gel column (EA/PE (v/v) = 2/1) to obtain compound 1-6 (4.0 g, 91%) as a light yellow solid. MS (ESI, pos. ion) m/z: 663.7 [M+H]⁺.

### Step 6: Synthesis of compound 1

Compound 1-6 (2.0 g, 3.02 mmol) and 1H-tetrazole (0.25 g, 3.62 mmol) were added to dichloromethane (40 mL) under nitrogen. Bis(diisopropylamino)(2-cyanoethoxy)phosphine (1.92 mL, 6.04 mmol) was then added dropwise. The mixture was stirred at room temperature for 4 h. After the reaction, DCM (20 mL) and saturated sodium bicarbonate solution (10 mL) were added to the reaction mixture. The organic phase was washed with saturated brine, dried over anhydrous sodium sulfate, and concentrated under reduced pressure. The resulting residue was purified by silica gel column chromatography (EA/PE (v/v) = 4/1) to obtain compound 1 (2.13 g, 82%).
MS (ESI, pos. ion) m/z: 864.9 [M+H]⁺;
¹H NMR (400 MHz, CDCl₃) δ 8.84 (s, 1H), 8.00 (d, *J =* 12.0 Hz, 1H), 7.45 - 7.39 (m, 2H), 7.34 - 7.28 (m, 4H), 7.22 (qd, *J =* 7.6, 6.4, 3.5 Hz, 3H), 7.02 (dd, *J =* 6.8, 4.5 Hz, 1H), 6.92 (dd, *J =* 4.6, 3.1 Hz, 1H), 6.78 (td, *J* = 8.5, 4.4 Hz, 4H), 4.93 (dd, *J =* 25.0, 5.0 Hz, 1H), 4.64 - 4.43 (m, 2H), 4.05 - 3.88 (m, 2H), 3.79 (dd, *J = 3.7,* 2.4 Hz, 6H), 3.60 (d, *J =* 6.3 Hz, 4H), 3.48 (dd, *J =* 10.5, 3.6 Hz, 1H), 3.27 (s, 3H), 3.24 (s, 3H), 2.66 (d, *J =* 6.4 Hz, 1H), 2.06 (s, 1H), 1.28 (t, *J =* 7.1 Hz, 2H), 1.19 (dd, *J =* 6.8, 4.1 Hz, 8H), 1.04 (d, *J =* 6.8 Hz, 4H).
³¹P NMR (162 MHz, CDCl₃) δ 150.01, 149.80.

### Example 2: Synthesis of carrier (compound DAW40007-3)

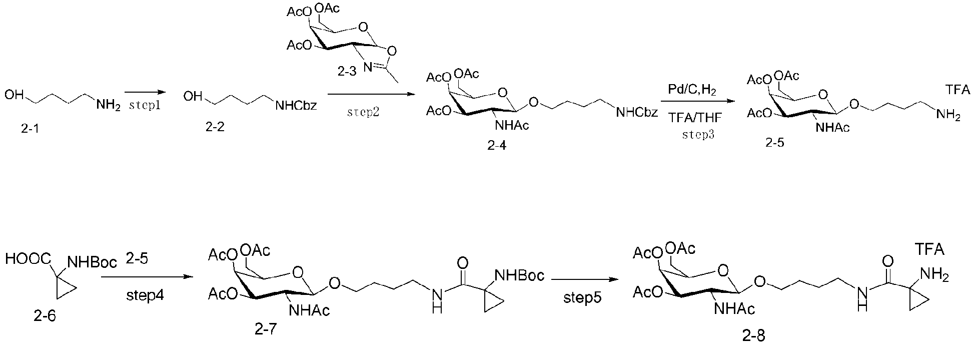

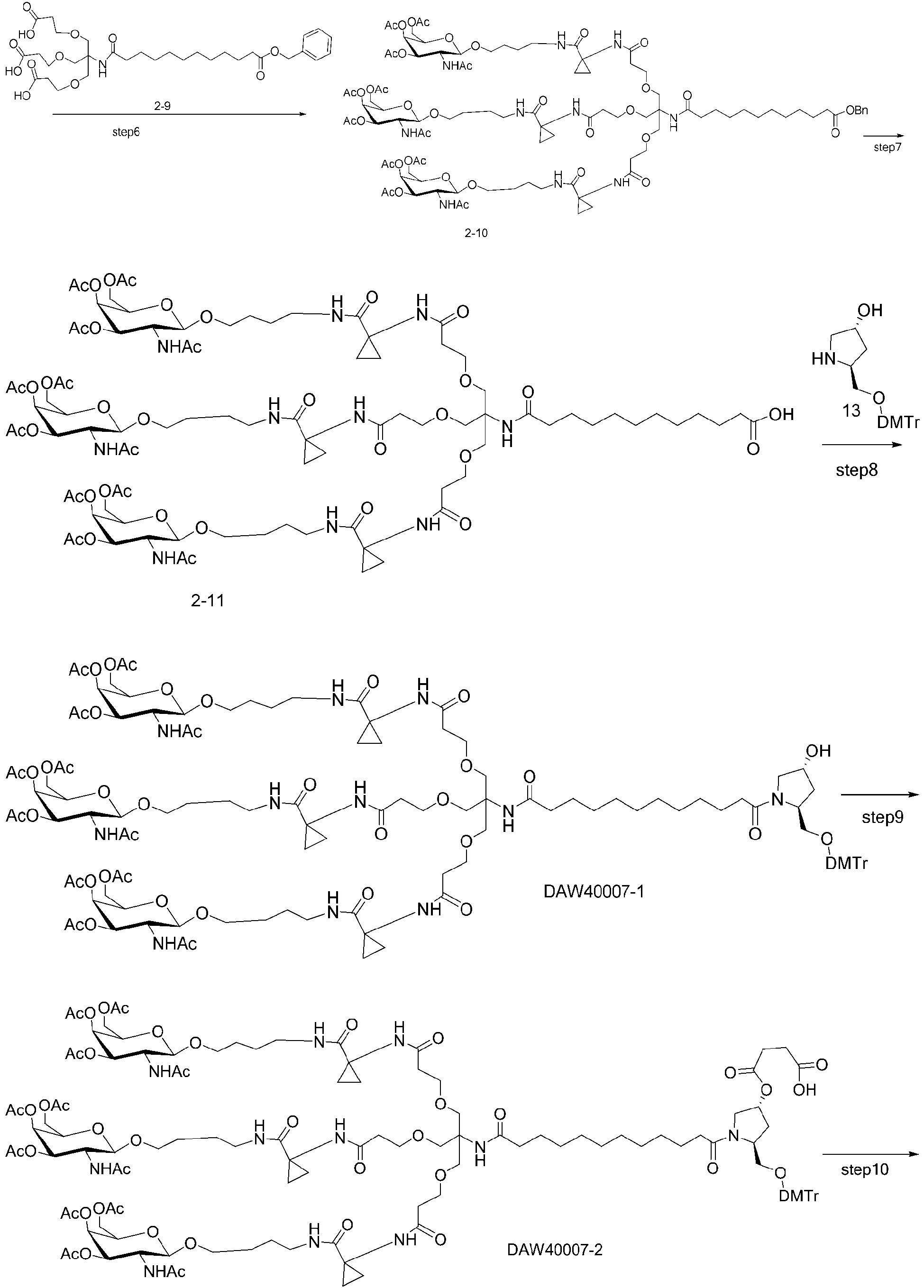

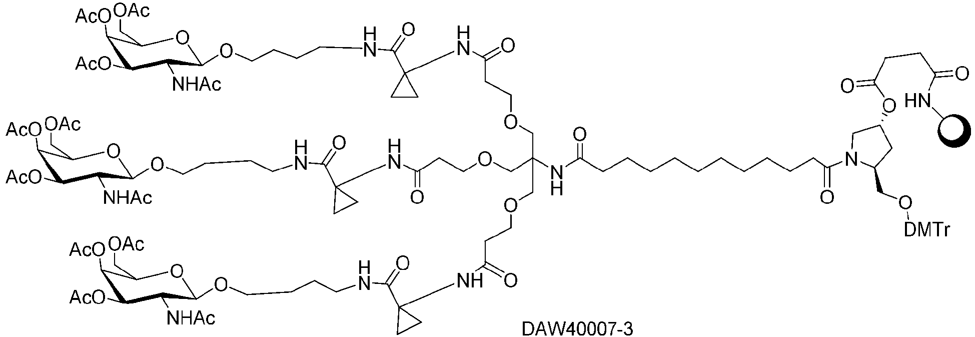

### Step 1: Synthesis of compound 2-2

Compound 2-1 (2.50 g, 28.05 mmol) and triethylamine (7.8 mL, 56.1 mmol) were dissolved in DCM (120 mL), then Benzyl chloroformate (9.57 g, 56.1 mmol) was added dropwise at 0 °C. After the addition, the reaction mixture was warmed to room temperature and stirred for 20 h. Saturated ammonium chloride solution (50 mL) was then added for dilution. The layers were separated, the aqueous phase was discarded, and the organic phase was concentrated. The resulting residue was purified by silica gel column chromatography (MeOH/DCM (v/v) = 1/30) to obtain compound 2-2 (2.96 g, 47.2%) as a white solid.
MS (ESI, pos. ion) m/z:224.2 [M+H]⁺;
¹H NMR (400 MHz, CDCl₃) δ 7.37 (d, *J =* 4.2 Hz, 5H), 5.11 (s, 2H), 5.00 (s, 1H), 4.19 - 4.14 (m, 1H), 3.70 - 3.64 (m, 2H), 3.24 (t, *J =* 6.4 Hz, 2H), 3.15 (q, *J =* 4.6 Hz, 1H), 1.90 (dq, *J =* 14.2, 5.0, 4.3 Hz, 2H), 1.77 - 1.71 (m, 1H).

### Step 2: Synthesis of compound 2-4

Compound 2-3 (1.5 g, 4.56 mmol) and compound 2-2 (1.22 g, 5.47 mmol) were dissolved in 1,2-dichloroethane (30 mL), and 3A molecular sieves (2.0 g) were added and the resulting mixture was stirred at room temperature for 10 min. TMSOTF (0.51 g, 2.28 mmol) was then added, and the reaction mixture was stirred at room temperature for 18 h. The reaction mixture was poured into saturated sodium bicarbonate solution (100 mL), extracted with DCM (100 mL), washed with saturated brine (100 mL), dried over anhydrous sodium sulfate, and the solvent evaporated under reduced pressure. The resulting residue was purified by silica gel column chromatography (PE/EA (v/v) = 2/1 to 0/1) to obtain compound 2-4 (1.8 g, 71.51%) as a light brown oil. MS (ESI, pos. ion) m/z: 553.3 [M+H]⁺.

### Step 3: Synthesis of compound 2-5

Compound 2-4 (0.57 g, 1.03 mmol) and palladium carbon (0.11 g, 0.1 mmol, 10%) were added to THF (10 mL), and TFA (0.12 g, 1.03 mmol) was added. The atmosphere was then replaced with hydrogen three times, and then stirred at room temperature in a hydrogen atmosphere for 19 h. After the reaction was completed, the mixture was filtered through celite, and the solvent was evaporated under reduced pressure to obtain compound 2-5 (0.55 g, 100.32%) as a light brown oil. MS (ESI, pos. ion) m/z: 419.3 [M-TFA+H]⁺.

### Step 4: Synthesis of compound 2-7

Compound 2-6 (0.46 g, 2.29 mmol, purchased from Shanghai Bid Pharmaceutical Technology Co., Ltd.) and compound 2-5 (1.16 g, 2.18 mmol) were dissolved in DCM (30 mL), and HOBT (0.46 g, 3.44 mmol), HBTU (1.30 g, 3.44 mmol) and DIPEA (2.66 mL, 16.03 mmol) were added sequentially. The reaction mixture was reacted at room temperature for 16 h. After the reaction, water (20 mL) and DCM (50 mL × 2) were added sequentially. The organic phase was washed with saturated sodium bicarbonate solution (30 mL) and saturated brine (20 mL) in sequence. The solvent was evaporated under reduced pressure, and the resulting residue was purified by silica gel column chromatography (MeOH/EA (v/v) = 1/20) to obtain compound 2-7 (1.0 g, yield 72.7%) as a white solid.
MS (ESI, pos. ion) m/z: 602.3 [M+H]⁺;
¹H NMR (400 MHz, CD₃OD) δ 5.39 - 5.33 (m, 1H), 5.07 (dd, *J =* 11.2, 3.4 Hz, 1H), 4.58 (d, *J* = 8.4 Hz, 1H), 4.21 - 4.09 (m, 3H), 4.04 (t, *J =* 6.7 Hz, 1H), 3.89 (dt, *J =* 10.4, 5.1 Hz, 1H), 3.60 - 3.50 (m, 1H), 3.30 - 3.13 (m, 2H), 2.16 (s, 3H), 2.04 (s, 3H), 1.97 (s, 3H), 1.94 (s, 3H), 1.67 - 1.56 (m, 4H), 1.48 (s, 9H), 1.41 - 1.37 (m, 2H), 1.02 - 0.98 (m, 2H).

### Step 5: Synthesis of compound 2-8

Compound 2-7 (0.72 g, 1.17 mmol) was dissolved in DCM (8 mL), and then TFA (0.87 mL, 11.7 mmol) was added. The reaction mixture was stirred at 25 °C for 16 h, and the solvent was concentrated to obtain compound 2-8 (0.74 g, 103.1%) as a brown oil.
MS (ESI, pos. ion) m/z:502.2 [M+H]⁺;
¹H NMR (400 MHz, CD₃OD) δ 5.36 (d, *J =* 3.3 Hz, 1H), 5.08 (dd, *J =* 11.3, 3.3 Hz, 1H), 4.58 (d, *J* = 8.4 Hz, 1H), 4.16 - 4.10 (m, 3H), 4.08 - 4.02 (m, 1H), 3.92 - 3.85 (m, 1H), 3.58 - 3.50 (m, 1H), 3.26 - 3.20 (m, 2H), 2.16 (s, 3H), 2.04 (d, *J =* 5.2 Hz, 6H), 1.98 (s, 3H), 1.97 (s, 3H), 1.61 - 1.56 (m, 4H), 1.54 - 1.52 (m, 2H), 1.42 - 1.38 (m, 2H).

### Step 6: Synthesis of compound 2-10

Compound 2-9 (0.19 g, 0.30 mmol) and compound 2-8 (0.50 g, 0.99 mmol) were dissolved in DCM (30 mL), and HOBT (0.17 g, 1.26 mmol), HBTU (0.48 g, 1.26 mmol) and DIPEA (0.5 mL, 3.0 mmol) were added sequentially. The reaction mixture was reacted at 30 °C for 3 h. After the reaction was completed, water (20 mL) was added to quench the reaction, and then extracted with DCM (100 mL × 2). The organic phases were combined and washed sequentially with saturated sodium bicarbonate solution (40 mL) and saturated sodium chloride solution (40 mL), dried over anhydrous sodium sulfate, and the solvent was evaporated under reduced pressure. The resulting residue was separated and purified by silica gel column chromatography (DCM/MeOH (v/v) = 10/1) to obtain compound 2-10 as a white solid (0.23 g, yield 37%).
MS (ESI, pos. ion) m/z: 1046.3 [M/2+H]⁺;
¹H NMR (400 MHz, CD₃OD) δ 7.78 (t, *J =* 5.8 Hz, 2H), 7.38 - 7.36 (m, 3H), 5.36 (d, *J =* 3.4 Hz, 3H), 5.13 (s, 2H), 5.08 (dd, *J =* 11.2, 3.4 Hz, 3H), 4.58 (d, *J =* 8.4 Hz, 3H), 4.21 - 4.08 (m, 9H), 4.04 (t, *J =* 6.7 Hz, 3H), 3.93 - 3.82 (m, 3H), 3.73 - 3.66 (m, 12H), 3.60 - 3.52 (m, 3H), 3.29 - 3.18 (m, 6H), 2.52 (t, *J =* 6.0 Hz, 6H), 2.38 (t, *J =* 7.4 Hz, 2H), 2.20 (t, *J =* 7.7 Hz, 2H), 2.16 (s, 9H), 2.04 (s, 9H), 1.97 (s, 9H), 1.95 (s, 9H), 1.62 - 1.56 (m, 12H), 1.50 - 1.42 (m, 6H), 1.35 - 1.27 (m, 16H), 1.04 - 0.97 (m, 6H).

### Step 7: Synthesis of compound 2-11

Compound 2-10 (0.20 g, 0.094 mmol) was dissolved in methanol (10 mL), and then Pd/C (10 mg, 10%) was added. The atmosphere was replaced three times with hydrogen, and the reaction mixture was stirred at room temperature for 11 h under a hydrogen atmosphere. After the reaction was completed, the reaction mixture was filtered through celite, and the solvent in the filtrate was evaporated under reduced pressure to obtain compound 2-11 (0.19 g, 100%) as a white solid.

MS (ESI, pos. ion) m/z: 1001.1 [M/2+H]⁺.

### Step 8: Synthesis of compound DAW40007-1

Compound 2-11 (0.28 g, 0.14 mmol) was dissolved in DCM (20 mL), and HOBT (0.038 g, 0.28 mmol), HBTU (0.080 g, 0.21 mmol), DIPEA (0.054 g, 0.42 mmol) and compound 13 (0.068 g, 0.16 mmol) were added sequentially. The reaction mixture was stirred at room temperature for 13 h. After completion of the reaction, water (10 mL) was added to quench the reaction, and the mixture was extracted with DCM (20 mL). The organic phase was washed with saturated sodium bicarbonate solution (10 mL), and the solvent was evaporated under reduced pressure. The resulting residue was dissolved in acetonitrile (10 mL) and separated by reverse phase preparative column (acetonitrile/water solution (v/v) = 43% to 60%, 50 min). Sodium chloride was added to the solution containing the product after preparative separation to saturate the solution. The organic phase was separated, and the aqueous phase was extracted with acetonitrile (100 mL × 2), the organic phases were combined, and the combined organic phases were dried over anhydrous sodium sulfate. The solvent was evaporated under reduced pressure, and acetonitrile (30 mL) was added to the residue, which was then dried over sodium sulfate, filtered, and the filtrate was concentrated under reduced pressure to obtain a light yellow solid compound DAW40007-1 (0.080 g, yield 24%).
MS (ESI, neg. ion) m/z: 2400.18 [M-H]⁻;
¹H NMR (400 MHz, DMSO-*d₆*) δ 8.31 (s, 3H), 7.81 (d, *J =* 9.2 Hz, 3H), 7.57 (t, *J =* 6.0 Hz, 3H), 7.35 - 7.26 (m, 4H), 7.20 (td, *J =* 8.9, 3.0 Hz, 5H), 6.95 (s, 1H), 6.88 (ddd, *J =* 8.8, 5.8, 2.2 Hz, 4H), 5.22 (d, *J =* 3.4 Hz, 3H), 4.97 (dd, *J =* 11.2, 3.5 Hz, 4H), 4.49 (d, *J =* 8.5 Hz, 3H), 4.40 (d, *J =* 4.8 Hz, 1H), 4.15 (s, 1H), 4.07 - 4.00 (m, 9H), 3.88 (dt, *J =* 11.2, 8.8 Hz, 3H), 3.74 (s, 9H), 3.59 - 3.48 (m, 12H), 3.17 (dd, *J =* 8.8, 5.0 Hz, 1H), 3.10 - 2.95 (m, 8H), 2.35 (t, *J =* 6.3 Hz, 6H), 2.10 (s, 9H), 2.08 (s, 3H), 2.04 (d, *J =* 4.7 Hz, 2H), 2.00 (s, 9H), 1.89 (s, 9H), 1.78 (s, 9H), 1.40 (d, *J =* 12.2 Hz, 17H), 1.31 - 1.16 (m, 18H), 0.79 (q, *J =* 3.2 Hz, 6H).

### Step 9: Synthesis of compound DAW40007-2

Compound DAW40007-1 (0.080 g, 0.033 mmol) was dissolved in DCM (10 mL), and DIPEA (0.029 mL, 0.17 mmol), succinic anhydride (0.008 g, 0.083 mmol) and DMAP (0.014 g, 0.12 mmol) were added. The mixture was stirred at 40 °C for 5 h, and succinic anhydride (10 mg) was added. After the reaction was stirred for further 16 h, DCM (10 mL), succinic anhydride (10 mg) and DIPEA (0.05 mL) were added. The resulting mixture was stirred for another 9 h, and then succinic anhydride (10 mg) was added. After the reaction was stirred for another 10 h, DCM (20 mL) was added to dilute the mixture, and the mixture was washed with saturated sodium bicarbonate solution (10 mL). The aqueous phase was discarded, and the organic phase was dried over anhydrous sodium sulfate. The solvent was concentrated under reduced pressure to obtain compound DAW40007-2 (0.08 g, 96.07%). MS (ESI, neg. ion) m/z: 2500.12 [M-H]⁻.

### Step 10: Synthesis of compound DAW40007-3

Compound DAW40007-2 (0.08 g, 0.032 mmol), HBTU (0.015 g, 0.04 mmol) and DIPEA (0.011 mL, 0.064 mmol) were dissolved in ACN (5 mL), and the resulting mixture was stirred at room temperature for 5 min, and then transferred to a solid-phase synthesizer containing 0.35 g of H₂N-CPG (purchased from Hebei DNAchem) and shaken for 22.5 h. The mixture was filtered, and the filter cake was rinsed with DCM/MeOH (V/V = 9/1, 10 mL) and DCM (10 mL) and dried under reduced pressure. The resulting filter cake was diluted in 25% Ac₂O/Py solution (5 mL) and stirred for 3 h. The mixture was filtered. The filter cake was rinsed with DCM/MeOH (V/V = 9/1, 10 mL) and DCM (10 mL) in sequence, then dried under reduced pressure to obtain DAW40007-3 (0.357 g) as a white solid with a measured loading of 14.95 µmol/g.

It is well known in the art that DAW40007-4 is the residue after DAW40007-3 undergoes a chemical reaction, and is bound to the nucleic acid through a covalent bond.

### Synthesis of compound L96

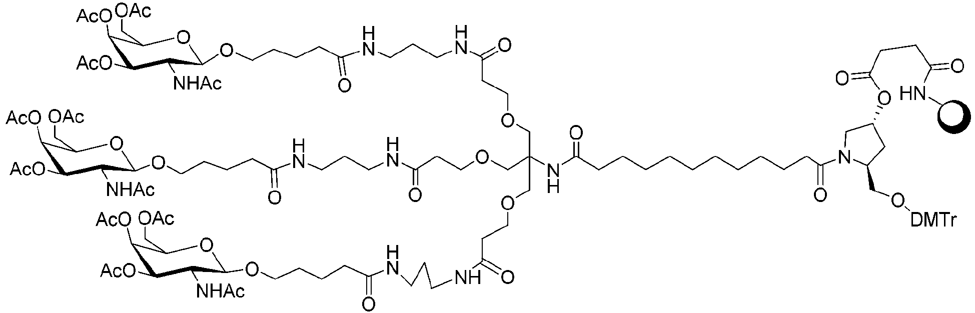

Compound L96 was prepared according to the method described in patent application WO2014025805A1.

The structure of the L96 conjugated group is:

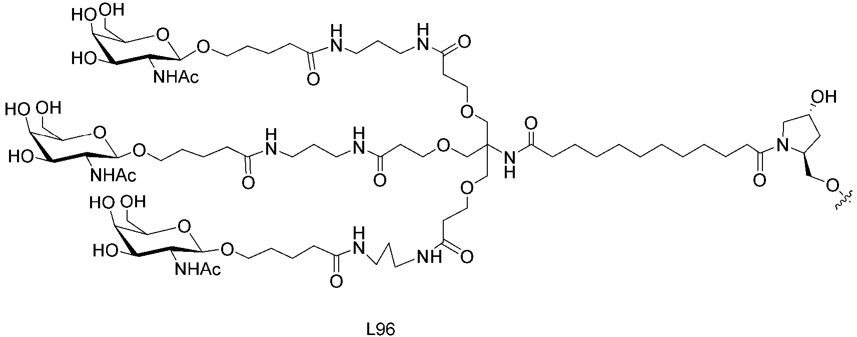

### Example 3: Synthesis of double-stranded siRNA and double-stranded siRNA conjugates

### 1. Synthesis of double-stranded siRNA without conjugated groups

The steps for synthesizing the sense strands and antisense strands of siRNA without conjugated groups are as follows:
The synthesis was completed according to the theoretical yield of 1 µmol. 1 µmol solid support CPG (purchased from Hebei DNAchem) was weighed. All 2'-modified RNA phosphoramidite monomers and auxiliary reagents were commercially available, and all phosphoramidite monomers were provided in 0.1 M anhydrous acetonitrile solution. For oligonucleotides with phosphate backbone thiolation modifications, 0.1 M DDTT solution was used as the thiolation reagent. 5-Ethylthio-1H-tetrazole acetonitrile solution (0.25 M) as an activating agent (purchased from Suzhou Kelema), 0.02 M iodine in pyridine/water solution as an oxidizing agent, and 3% trichloroacetic acid in dichloromethane as a deprotection reagent were placed in the corresponding reagent designated position of the KA-H8 model DNA/RNA automatic synthesizer. The synthesis program was set up and the designated oligonucleotide base sequence was input. After verification, oligonucleotide synthesis was initiated in cycles. The coupling time for each step was 6 min, and the sulfurization time was 6 min. After automated cycling, a CPG-containing oligonucleotide on a solid support was obtained.

The CPG-containing nucleotide obtained above was dried with dry argon and then transferred to a 2 mL EP tube. 28% aqueous ammonia solution (1.8 mL) was added and heated at 55 °C for 5~18 h. The mixture was filtered, the filter cake was washed with 0.5 mL of water, and the filtrates were combined and concentrated under reduced pressure to obtain a white or yellow colloidal solid. After reverse-phase preparative purification, the preparative solution was concentrated and passed through a gel column to remove excess salts to obtain the oligonucleotide. The concentration of the resulting oligonucleotide was determined using a micro-UV spectrophotometer (SPECTRO stat Nano). Analysis was performed using mass spectrometry on an Agilent 6530 LC-MS Q-Tof system. The molecular weight of nucleic acids was calculated after primary scanning and deconvolution.

### Annealing step:

The double-stranded siRNA sense strand synthesized above was mixed with the antisense strand synthesized above in equimolar amounts, heated to 95 °C, maintained at this temperature for 10 min, and then slowly cooled to room temperature. Then, the target double-stranded siRNA was lyophilized.

### 2. Synthesis of siRNA conjugates:

The antisense strand was synthesized using the aforementioned methods for synthesizing the sense and antisense strands without the conjugated group.

Synthesis of the sense strand: The general solid support CPG was replaced with the conjugated group-solid support CPG prepared in the present invention (such as compound DAW40007-3), and the sense strand of the double-stranded siRNA conjugate of the present invention was prepared by referring to the antisense strand synthesis method.

### Annealing step:

The sense strand of the double-stranded siRNA conjugate synthesized above was mixed with the antisense strand synthesized above in equimolar amounts, heated to 95 °C, maintained at this temperature for 10 min, and then slowly cooled to room temperature. Then, the target siRNA conjugate was lyophilized.

The double-stranded siRNA and double-stranded siRNA conjugates synthesized above are shown in Table 1-A.

**Table 1-A: Double-stranded siRNA and double-stranded siRNA conjugates synthesized by the present invention**

| **Numbers of the double-strand ed siRNA** | **Sense strand sequence (5'-3')** | **SEQ ID NO:** | **Antisense strand sequence (5'-3')** | **SEQ ID NO:** |
|---|---|---|---|---|
| S1 | g●Y●guGcACUucgcuucaca | 1 | u●Y●ugaAgCGaaguGcAcac●u●u | 22 |
| S2 | g●u●YuGcACUucgcuucaca | 2 | u●Y●ugaAgCGaaguGcAcac●u●u | 22 |
| S3 | g●u●gYGcACUucgcuucaca | 3 | u●Y●ugaAgCGaaguGcAcac●u●u | 22 |
| S4 | g●u●guYcACUucgcuucaca | 4 | u●Y●ugaAgCGaaguGcAcac●u●u | 22 |
| S5 | g●u●guGYACUucgcuucaca | 5 | u●Y●ugaAgCGaaguGcAcac●u●u | 22 |
| S6 | g●u●guGcYCUucgcuucaca | 6 | u●Y●ugaAgCGaaguGcAcac●u●u | 22 |
| S7 | g●u●guGcAYUucgcuucaca | 7 | u●Y●ugaAgCGaaguGcAcac●u●u | 22 |
| S8 | g●u●guGcACYucgcuucaca | 8 | u●Y●ugaAgCGaaguGcAcac●u●u | 22 |
| S9 | g●u●guGcACUYcgcuucaca | 9 | u●Y●ugaAgCGaaguGcAcac●u●u | 22 |
| S10 | g●u●guGcACUuYgcuucaca | 10 | u●Y●ugaAgCGaaguGcAcac●u●u | 22 |
| S11 | g●u●guGcACUucYcuucaca | 11 | u●Y●ugaAgCGaaguGcAcac●u●u | 22 |
| S12 | g●u●guGcACUucgYuucaca | 12 | u●Y●ugaAgCGaaguGcAcac●u●u | 22 |
| S13 | g●u●gGcACUucgcYucaca | 13 | u●Y●ugaAgCGaaguGcAcac●u●u | 22 |
| S14 | g●u●guGcACUucgcuYcaca | 14 | u●Y●ugaAgCGaaguGcAcac●u●u | 22 |
| S15 | g●u●guGcACUucgcuuYaca | 15 | u●Y●ugaAgCGaaguGcAcac●u●u | 22 |
| S16 | g●u●guGcACUucgcuucYca | 16 | u●Y●ugaAgCGaaguGcAcac●u●u | 22 |
| S17 | g●u●guGcACUucgcuucaYa | 17 | u●Y●ugaAgCGaaguGcAcac●u●u | 22 |
| S18 | g●u●guGcACUucgcuucacY | 18 | u●Y●ugaAgCGaaguGcAcac●u●u | 22 |
| S19 | g●Y●guGcACUucgcuucaca | 1 | u●G●YgaAgCGaaguGcAcac●u●u | 23 |
| S20 | g●u●YuGcACUucgcuucaca | 2 | u●G●YgaAgCGaaguGcAcac●u●u | 23 |
| S21 | g●u●gYGcACUucgcuucaca | 3 | u●G●YgaAgCGaaguGcAcac●u●u | 23 |
| S22 | g●u●guYcACUucgcuucaca | 4 | u●G●YgaAgCGaaguGcAcac●u●u | 23 |
| S23 | g●u●guGYACUucgcuucaca | 5 | u●G●YgaAgCGaaguGcAcac●u●u | 23 |
| S24 | g●u●guGcYCUucgcuucaca | 6 | u●G●YgaAgCGaaguGcAcac●u●u | 23 |
| S25 | g●u●guGcAYUucgcuucaca | 7 | u●G●YgaAgCGaaguGcAcac●u●u | 23 |
| S26 | g●u●guGcACYucgcuucaca | 8 | u●G●YgaAgCGaaguGcAcac●u●u | 23 |
| S27 | g●u●guGcACUYcgcuucaca | 9 | u●G●YgaAgCGaaguGcAcac●u●u | 23 |
| S28 | g●u●guGcACUuYgcuucaca | 10 | u●G●YgaAgCGaaguGcAcac●u●u | 23 |
| S29 | g●u●guGcACUucYcuucaca | 11 | u●G●YgaAgCGaaguGcAcac●u●u | 23 |
| S30 | g●u●guGcACUucgYuucaca | 12 | u●G●YgaAgCGaaguGcAcac●u●u | 23 |
| S31 | g●u●gGcACUucgcYucaca | 13 | u●G●YgaAgCGaaguGcAcac●u●u | 23 |
| S32 | g●u●guGcACUucgcuYcaca | 14 | u●G●YgaAgCGaaguGcAcac●u●u | 23 |
| S33 | g●u●guGcACUucgcuuYaca | 15 | u●G●YgaAgCGaaguGcAcac●u●u | 23 |
| S34 | g●u●guGcACUucgcuucYca | 16 | u●G●YgaAgCGaaguGcAcac●u●u | 23 |
| S35 | g●u●guGcACUucgcuucaYa | 17 | u●G●YgaAgCGaaguGcAcac●u●u | 23 |
| S36 | g●u●guGcACUucgcuucacY | 18 | u●G●YgaAgCGaaguGcAcac●u●u | 23 |
| S37 | g●Y●guGcACUucgcuucaca | 1 | u●G●uYgaAgCGaaguGcAcac●u●u | 24 |
| S38 | g●u●YuGcACUucgcuucaca | 2 | u●G●YgaAgCGaaguGcAcac●u●u | 24 |
| S39 | g●u●gYGcACUucgcuucaca | 3 | u●G●YgaAgCGaaguGcAcac●u●u | 24 |
| S40 | g●u●guYcACUucgcuucaca | 4 | u●G●YgaAgCGaaguGcAcac●u●u | 24 |
| S41 | g●u●guQYACUucgcuucaca | 5 | u●G●YgaAgCGaaguGcAcac●u●u | 24 |
| S42 | g●u●guGcYCUucgcuucaca | 6 | u●G●YgaAgCGaaguGcAcac●u●u | 24 |
| S43 | g●u●guGcAYUucgcuucaca | 7 | u●G●YgaAgCGaaguGcAcac●u●u | 24 |
| S44 | g●u●guGcACYucgcuucaca | 8 | u●G●YgaAgCGaaguGcAcac●u●u | 24 |
| S45 | g●u●guGcACUYcgcuucaca | 9 | u●G●YgaAgCGaaguGcAcac●u●u | 24 |
| S46 | g●u●guGcACUuYgcuucaca | 10 | u●G●YgaAgCGaaguGcAcac●u●u | 24 |
| S47 | g●u●guGcACUucYcuucaca | 11 | u●G●YgaAgCGaaguGcAcac●u●u | 24 |
| S48 | g●u●guGcACUucgYuucaca | 12 | u●G●YgaAgCGaaguGcAcac●u●u | 24 |
| S49 | g●u●gGcACUucgcYucaca | 13 | u●G●YgaAgCGaaguGcAcac●u●u | 24 |
| S50 | g●u●guGcACUucgcuYcaca | 14 | u●G●YgaAgCGaaguGcAcac●u●u | 24 |
| S51 | g●u●guGcACUucgcuucYca | 16 | u●G●YgaAgCGaaguGcAcac●u●u | 24 |
| S52 | g●u●guGcACUucgcuucaYa | 17 | u●G●YgaAgCGaaguGcAcac●u●u | 24 |
| S53 | g●u●guGcACUucgcuucacY | 18 | u●G●YgaAgCGaaguGcAcac●u●u | 24 |
| S54 | g●Y●guGcACUucgcuucaca | 1 | u●G●ugYAgCGaaguGcAcac●u●u | 25 |
| S55 | g●u●YuGcACUucgcuucaca | 2 | u●G●YgaAgCGaaguGcAcac●u●u | 25 |
| S56 | g●u●gYGcACUucgcuucaca | 3 | u●G●YgaAgCGaaguGcAcac●u●u | 25 |
| S57 | g●u●guYcACUucgcuucaca | 4 | u●G●YgaAgCGaaguGcAcac●u●u | 25 |
| S58 | g●u●guQYACUucgcuucaca | 5 | u●G●YgaAgCGaaguGcAcac●u●u | 25 |
| S59 | g●u●guGcYCUucgcuucaca | 6 | u●G●YgaAgCGaaguGcAcac●u●u | 25 |
| S60 | g●u●guGcAYUucgcuucaca | 7 | u●G●YgaAgCGaaguGcAcac●u●u | 25 |
| S61 | g●u●guGcACYucgcuucaca | 8 | u●G●YgaAgCGaaguGcAcac●u●u | 25 |
| S62 | g●u●guGcACUYcgcuucaca | 9 | u●G●YgaAgCGaaguGcAcac●u●u | 25 |
| S63 | g●u●guGcACUuYgcuucaca | 10 | u●G●YgaAgCGaaguGcAcac●u●u | 25 |
| S64 | g●u●guGcACUucYcuucaca | 11 | u●G●YgaAgCGaaguGcAcac●u●u | 25 |
| S65 | g●u●guGcACUucgYuucaca | 12 | u●G●YgaAgCGaaguGcAcac●u●u | 25 |
| S66 | g●u●gGcACUucgcYucaca | 13 | u●G●YgaAgCGaaguGcAcac●u●u | 25 |
| S67 | g●u●guGcACUucgcuuYaca | 15 | u●G●YgaAgCGaaguGcAcac●u●u | 25 |
| S68 | g●u●guGcACUucgcuucYca | 16 | u●G●YgaAgCGaaguGcAcac●u●u | 25 |
| S69 | g●u●guGcACUucgcuucaYa | 17 | u●G●YgaAgCGaaguGcAcac●u●u | 25 |
| S70 | g●u●guGcACUucgcuucacY | 18 | u●G●YgaAgCGaaguGcAcac●u●u | 25 |
| S71 | g●Y●guGcACUucgcuucaca | 1 | u●G●ugaAYCGaaguGcAcac●u●u | 26 |
| S72 | g●u●YuGcACUucgcuucaca | 2 | u●G●YgaAgCGaaguGcAcac●u●u | 26 |
| S73 | g●u●gYGcACUucgcuucaca | 3 | u●G●YgaAgCGaaguGcAcac●u●u | 26 |
| S74 | g●u●guYcACUucgcuucaca | 4 | u●G●YgaAgCGaaguGcAcac●u●u | 26 |
| S75 | g●u●guQYACUucgcuucaca | 5 | u●G●YgaAgCGaaguGcAcac●u●u | 26 |
| S76 | g●u●guGcYCUucgcuucaca | 6 | u●G●YgaAgCGaaguGcAcac●u●u | 26 |
| S77 | g●u●guGcAYUucgcuucaca | 7 | u●G●YgaAgCGaaguGcAcac●u●u | 26 |
| S78 | g●u●guGcACYucgcuucaca | 8 | u●G●YgaAgCGaaguGcAcac●u●u | 26 |
| S79 | g●u●guGcACUYcgcuucaca | 9 | u●G●YgaAgCGaaguGcAcac●u●u | 26 |
| S80 | g●u●guGcACUuYgcuucaca | 10 | u●G●YgaAgCGaaguGcAcac●u●u | 26 |
| S81 | g●u●guGcACUucYcuucaca | 11 | u●G●YgaAgCGaaguGcAcac●u●u | 26 |
| S82 | g●u●guGcACUucgYuucaca | 12 | u●G●YgaAgCGaaguGcAcac●u●u | 26 |
| S83 | g●u●guGcACUucgcYucaca | 13 | u●G●YgaAgCGaaguGcAcac●u●u | 26 |
| S84 | g●u●guGcACUucgcuYcaca | 14 | u●G●YgaAgCGaaguGcAcac●u●u | 26 |
| S85 | g●u●guGcACUucgcuuYaca | 15 | u●G●YgaAgCGaaguGcAcac●u●u | 26 |
| S86 | g●u●guGcACUucgcuucYca | 16 | u●G●YgaAgCGaaguGcAcac●u●u | 26 |
| S87 | g●u●guGcACUucgcuucaYa | 17 | u●G●YgaAgCGaaguGcAcac●u●u | 26 |
| S88 | g●u●guGcACUucgcuucacY | 18 | u●G●YgaAgCGaaguGcAcac●u●u | 26 |
| S89 | g●Y●guGcACUucgcuucaca | 1 | u●G●ugaAYCGaaguGcAcac●u●u | 27 |
| S90 | g●u●YuGcACUucgcuucaca | 2 | u●G●YgaAgCGaaguGcAcac●u●u | 27 |
| S91 | g●u●gYGcACUucgcuucaca | 3 | u●G●ugaAYCGaaguGcAcac●u●u | 27 |
| S92 | g●u●guYcACUucgcuucaca | 4 | u●G●YgaAgCGaaguGcAcac●u●u | 27 |
| S93 | g●u●guQYACUucgcuucaca | 5 | u●G●YgaAgCGaaguGcAcac●u●u | 27 |
| S94 | g●u●guGcYCUucgcuucaca | 6 | u●G●YgaAgCGaaguGcAcac●u●u | 27 |
| S95 | g●u●guGcAYUucgcuucaca | 7 | u●G●YgaAgCGaaguGcAcac●u●u | 27 |
| S96 | g●u●guGcACYucgcuucaca | 8 | u●G●YgaAgCGaaguGcAcac●u●u | 27 |
| S97 | g●u●guGcACUYcgcuucaca | 9 | u●G●YgaAgCGaaguGcAcac●u●u | 27 |
| S98 | g●u●guGcACUuYgcuucaca | 10 | u●G●YgaAgCGaaguGcAcac●u●u | 27 |
| S99 | g●u●guGcACUucYcuucaca | 11 | u●G●YgaAgCGaaguGcAcac●u●u | 27 |
| S100 | g●u●guGcACUucgYuucaca | 12 | u●G●YgaAgCGaaguGcAcac●u●u | 27 |
| S101 | g●u●gGcACUucgcYucaca | 13 | u●G●YgaAgCGaaguGcAcac●u●u | 27 |
| S102 | g●u●guGcACUucgcuYcaca | 14 | u●G●YgaAgCGaaguGcAcac●u●u | 27 |
| S103 | g●u●guGcACUucgcuuYaca | 15 | u●G●YgaAgCGaaguGcAcac●u●u | 27 |
| S104 | g●u●guGcACUucgcuucYca | 16 | u●G●YgaAgCGaaguGcAcac●u●u | 27 |
| S105 | g●u●guGcACUucgcuucaYa | 17 | u●G●ugaAYCGaaguGcAcac●u●u | 27 |
| S106 | g●u●guGcACUucgcuucacY | 18 | u●G●ugaAYCGaaguGcAcac●u●u | 27 |
| S107 | g●Y●guGecACUucgcuucaca | 1 | u●G●ugaAgYGaaguGcAcac●u●u | 28 |
| S108 | g●u●YuGcACUucgcuucaca | 2 | u●G●ugaAYCGaaguGcAcac●u●u | 28 |
| S109 | g●u●gYGcACUucgcuucaca | 3 | u●G●ugaAYCGaaguGcAcac●u●u | 28 |
| S110 | g●u●guYcACUucgcuucaca | 4 | u●G●ugaAgYGaaguGcAcac●u●u | 28 |
| S111 | g●u●guQYACUucgcuucaca | 5 | u●G●ugaAYCGaaguGcAcac●u●u | 28 |
| S112 | g●u●guGcYCUucgcuucaca | 6 | u●G●ugaAYCGaaguGcAcac●u●u | 28 |
| S113 | g●u●guGcAYUucgcuucaca | 7 | u●G●ugaAYCGaaguGcAcac●u●u | 28 |
| S114 | g●u●guGcACYucgcuucaca | 8 | u●G●ugaAYCGaaguGcAcac●u●u | 28 |
| S115 | g●u●guGcACUYcgcuucaca | 9 | u●G●ugaAYCGaaguGcAcac●u●u | 28 |
| S116 | g●u●guGcACUuYgcuucaca | 10 | u●G●ugaAYCGaaguGcAcac●u●u | 28 |
| S117 | g●u●guGcACUucgYuucaca | 12 | u●G●ugaAYCGaaguGcAcac●u●u | 28 |
| S118 | g●u●gGcACUucgcYucaca | 13 | u●G●ugaAYCGaaguGcAcac●u●u | 28 |
| S119 | g●u●guGcACUucgcuYcaca | 14 | u●G●ugaAYCGaaguGcAcac●u●u | 28 |
| S120 | g●u●guGcACUucgcuuYaca | 15 | u●G●ugaAYCGaaguGcAcac●u●u | 28 |
| S121 | g●u●guGcACUucgcuucYca | 16 | u●G●ugaAYCGaaguGcAcac●u●u | 28 |
| S122 | g●u●guGcACUucgcuucaYa | 17 | u●G●ugaAYCGaaguGcAcac●u●u | 28 |
| S123 | g●u●guGcACUucgcuucacY | 18 | u●G●ugaAYCGaaguGcAcac●u●u | 28 |
| S124 | g●u●guGcACUucgcuucaca | 19 | u●Y●ugaAgCGaaguGcAcac●u●u | 22 |
| S125 | g●u●guGcACUucgcuucaca | 19 | u●G●YgaAgCGaaguGcAcac●u●u | 23 |
| S126 | g●u●guGcACUucgcuucaca | 19 | u●G●YgaAgCGaaguGcAcac●u●u | 24 |
| S127 | g●u●guGcACUucgcuucaca | 19 | u●G●YgaAgCGaaguGcAcac●u●u | 25 |
| S128 | g●u●guGcACUucgcuucaca | 19 | u●G●YgaAgCGaaguGcAcac●u●u | 26 |
| S129 | g●u●guGcACUucgcuucaca | 19 | u●G●YgaAgCGaaguGcAcac●u●u | 27 |
| S130 | g●u●guGcACUucgcuucaca | 19 | u●G●ugaAYCGaaguGcAcac●u●u | 28 |
| S131 | g●u●guGcACUucgcuucaca | 19 | u●G●ugaAgCGYaguGcAcac●u●u | 29 |
| S132 | g●u●guGcACUucgcuucaca | 19 | u●G●ugaAgCGaYguGcAcac●u●u | 30 |
| S133 | g●u●guGcACUucgcuucaca | 19 | u●G●ugaAgCGaaguGcAcac●u●u | 31 |
| S134 | g●u●guGcACUucgcuucaca | 19 | u●G●ugaAgCGaaguGcAcac●u●u | 32 |
| S135 | g●u●guGcACUucgcuucaca | 19 | u●G●uga(Agn)gCGYaguGcAcac● u●u | 38 |
| S136 | g●u●guGcACUucgcuucaca | 19 | u●G●uga(Agn)gCGaYguGcAcac● u●u | 39 |
| S137 | g●u●guGcACUucgcuucaca | 19 | u●G●uga(Agn)gCGaaguGcYcac●u ●u | 34 |
| S138 | g●u●guGcACUucgcuucaca | 19 | u●G●uga(Agn)gCGaaguGcAcYc● u●u | 35 |
| S139 | g●u●guGcYCUucgcuucaca | 6 | u●G●ugaAgCGaaguGcAcac●u●u | 36 |
| S140 | g●u●guGcACUucgcuucYca | 16 | u●G●ugaAgCGaaguGcAcac●u●u | 36 |
| S141 | g●u●guGcACUucgcuucacY | 18 | u●G●ugaAgCGaaguGcAcac●u●u | 36 |
| S142 | g●u●guGcACUucgcuucYca | 16 | u●G●YgaAgCGaaguGcAcac●u●u | 27 |
| S143 | g●u●guGcACUucgcuucYca | 16 | u●G●ugaYYCGaaguGcAcac●u●u | 37 |
| S144 | g●u●guGcACUucgcuucaca | 19 | u●G●ugaAgCGaaguGcAcac●u●u | 36 |
| S145 | g●u●guGcACUucgcuucaca | 19 | u•G•uga(Agn)gCGaaguGcAcac•u •u | 33 |

| **Numbers of the double-strand ed siRNA conjugates** | **Sense strand sequence (5'-3')-conjugation group** | **SEQ ID NO** | **Antisense strand sequence (5'-3')** | **SEQ ID NO** |
|---|---|---|---|---|
| S146 | g●u●guGcACUucgcuucYca-DA W40007-4 | 21 | u●G●YgaAgCGaaguGcAcac●u●u | 27 |
| S147 | g●u●guGcACUucgcuuYaca-DA W40007-4 | 41 | u●G●YgaAgCGaaguGcAcac●u●u | 27 |
| S148 | g●u●guGcACUucgcuucaca- DAW40007-4 | 42 | u●G●uga(Agn)gCGaaguGcAcac●u ●u | 33 |
| S149 | g●u●guGcACUucgcuucaca-L96 | 43 | u●G●uga(Agn)gCGaaguGcAcac●u ●u | 33 |
| S150 | g●u●guGcACUucgcuucaYa-DA W40007-4 | 44 | u●G●ugaYgCGaaguGeAcac●u●u | 26 |
| S151 | g●u●guGcACUucgcuucXca-DA W40007-4 | 45 | u●G●ugaAXCGaaguGcAcac●u●u | 46 |

Capital letters C, G, U and A in oligonucleotides or siRNA represent the base composition of natural nucleotides; lowercase letters c, g, u and a respectively represent that the 2-position of the ribose on the nucleotide represented by the corresponding capital letter is modified by methoxy; an underline under a capital letter represents that the 2-position of the ribose on the nucleotide represented by the letter is modified by fluorine; the middle dot "●" represents that there is phosphorothioate linkage between two nucleotide residues adjacent to the left and right sides of the middle dot "●"; Agn is adenosine-glycol nucleic acid (Glycol nucleic acid, GNA,); Y in double-stranded siRNA represents 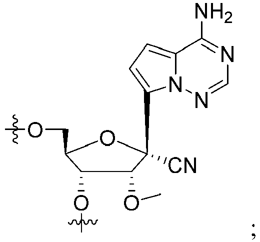 X in double-stranded siRNA represents

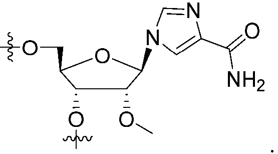

### Example 4 Cellular activity and cytotoxicity testing of the nucleic acid or conjugate of the present invention

Hepatocellular carcinoma cells (Hep AD38) were cultured in DMEM medium containing 10% fetal bovine serum in a 5% CO₂, 37 °C incubator. Hepatoma cells were used for experiments when they were in the logarithmic growth phase and in good condition (70% confluence). 1.5×10⁴ cells were seeded per well in a 96-well plate. After 24 h, the cells were transfected with a series of double-stranded siRNAs at different concentrations listed in Table 1 of Example 3 according to the instructions of RNAiMAX transfection reagent (Thermo Fisher Scientific). After incubation at 37 °C and 5% CO₂ for 72 h, HBsAg expression and cytotoxicity were detected using an HBsAg ELISA kit (Shanghai Kehua) and a CCK8 kit (DOJINDO), respectively. The double-stranded siRNA reagent of the present invention has good inhibitory activity on HBsA, and is significantly better than the HBsA inhibitory activity of the control S144. It can be seen that the introduction of nucleotide residue Y in the double-stranded siRNA described in the present application significantly improves the activity of the double-stranded siRNA, and the double-stranded siRNA described in the present application has low cytotoxicity. The HBsA inhibitory activity and cytotoxicity results of some double-stranded siRNAs or their conjugates are shown in Table 2 .

**Table 2: Inhibitory activity and cytotoxicity results of partially double-stranded siRNA or conjugates of the present invention**

| The double-strand ed siRNA conjugate under test | Sense strand sequence (5'-3') | Antisense strand sequence (5'-3') | EC₅₀/nM | CC₅₀/nM |
|---|---|---|---|---|
| S103 | g●u●guGcACUucgcuuYaca | u●G●ugaAYCGaaguGcAcac●u●u | 0.07003 | >1000 |
| S104 | g●u●guGcACUucgcuucYca | u●G●ugaAYCGaaguGcAcac●u●u | 0.0628 | >1000 |
| S105 | g●u●guGcACUucgcuucaYa | u●G●ugaAYCGaaguGcAcac●u●u | 0.06351 | >1000 |
| S85 | g●u●guGcACUucgcuuYaca | u●G●YgaAgCGaaguGcAcac●u●u | 0.05789 | >1000 |
| S87 | g●u●guGcACUucgcuucaYa | u●G●YgaAgCGaaguGcAcac●u●u | 0.04094 | >1000 |
| S67 | g●u●guGcACUucgcuuYaca | u●G●ugaAgCGaaguGcAcac●u●u | 0.06026 | >1000 |
| S69 | g●u●guGcACUucgcuucaYa | u●G●ugaAgCGaaguGcAcac●u●u | 0.05268 | >1000 |
| S122 | g●u●guGcACUucgcuucaYa | u●G●ugaAYCGaaguGcAcac●u●u | 0.08052 | >1000 |
| S27 | g●u●guGcACUYcgcuucaca | u●G●YgaAgCGaaguGcAcac●u●u | 0.042 | >1000 |
| S66 | g●u●guGcACUucgcYucaca | u●G●ugaAgCGaaguGcAcac●u●u | 0.094 | >1000 |
| S86 | g●u●guGcACUucgcuucYca | u●G●YgaAgCGaaguGcAcac●u●u | 0.043 | >1000 |
| S96 | g●u●guGcACYucgcuucaca | u●G●ugaAYCGaaguGcAcac●u●u | 0.080 | >1000 |
| S144 | g●u●guGcACUucgcuucaca | u●G●ugaAgCGaaguGcAcac●u●u | 0.4388 | >1000 |

Wherein, capital letters C, G, U and A represent the base composition of natural nucleotides; lowercase letters c, g, u and a respectively represent that the 2-position of the ribose on the nucleotide represented by the corresponding capital letter is modified by methoxy; an underline under a capital letter represents that the 2-position of the ribose on the nucleotide represented by the letter is modified by fluorine; the middle dot "●" represents that there is phosphorothioate linkage between two nucleotide residues adjacent to the left and right sides of the middle dot "●".

### Example 6: Evaluation of anti-hepatitis B virus (HBV) activity using the PHH in vitro infection model

### The experimental steps are as follows

Transfection and Drug Dosing: On Day 0, revived PHH were adjusted to an appropriate density (6.67E+05 cells/ml) and seeded into 48-well cell culture plates at 1.32E+05 cells per well. On Day 1, HBV serotype D was added to infect PHH (800 GE/cell). On Day 2, fresh medium was replaced. On Day 4, the cell culture medium was discarded, and the test compound was serially diluted to the required concentration, which were then introduced into the cells via transfection using Lipofectamine^{™} RNAiMAX. The starting concentration of the test compound was 500 pM, and 5-fold dilutions were performed. Seven concentrations were set up in duplicate, with wells containing only the transfection reagent as the medium control. Four to five hours after transfection, fresh medium without compound was replaced. The final DMSO concentration in the medium was 2%. On Day 6, fresh medium without compound was replaced. On Day 8, cell supernatants were harvested, and cell viability was assessed using CCK-8 assay.

Drug Administration via Ad libitum uptake : On day 0, the test compound was diluted to the desired concentration in enzyme-free water, starting at 500 nM. Five-fold dilutions were performed, each with seven concentrations, in duplicate, with 22 µl added to each well of the cell culture plate. Thawed frozen PHHs were plated to an appropriate density (6.67E+05 cells/ml), with 1.32E+05 cells per well. The cells were then plated and allowed to ad libitum uptake of the test compound. On day 1, the medium was changed, and the PHHs were infected with HBV serotype D (800 GE/cell). On day 2, fresh medium was replaced without virus or test compound. The final DMSO concentration in the medium was 2%. On Day 4 and 6, fresh medium without test compound was replaced. On Day 8, cell supernatants were harvested, and cell viability was assessed using CCK-8 assay.

Sample testing: qPCR was used to detect the HBV DNA content in the cell culture supernatant; ELISA was used to detect the HBsAg and HBeAg content in the cell culture supernatant. The experimental results of the double-stranded siRNA conjugate of the present invention reducing the levels of HBsAg, HBeAg and DNA in cells are shown in Table 3.

**Table 3: Experimental results of the double-stranded siRNA conjugates of the present invention in reducing the levels of HBsAg, HBeAg and DNA in cells**

| Treatme nt | The double-strande d siRNA conjugate under test | Experimental results EC50 | | |
|---|---|---|---|---|
| | | HBsAg(nM) | HBeAg(nM) | DNA(nM) |
| Transfec tion | S146 | 0.050 | 0.209 | 0.082 |
| No transfect ion | S148 | 2.902 | 7.417 | 5.720 |
| | S146 | 0.782 | 2.237 | 0.737 |

The experimental results showed that the double-stranded siRNA conjugate S146 had good inhibitory activity against HBsAg, HBeAg and DNA. Without the need for transfection, its inhibitory activity against HBsAg, HBeAg and DNA was significantly better than that of the control S148.

### Example 6: Hepatitis B virus transgenic mouse experiment

### 1. HBV. Tg transgenic mouse experiments

### Test method

Experimental Animals: HBV-Tg transgenic mice, C57B/6N-Tg(1.28HBV)/Vst, SPF-grade males, 6-8 weeks old, weighing 16-20 g, were purchased from Beijing Vitalstar Biotechnology Co.,Ltd., Animal Production Certificate No.: SCXK(Beijing)2019-0002. Animals were housed individually in individual cages by Beijing Vitalstar Biotechnology Co.,Ltd.

Animal Grouping: Based on the quantitative results of serum HBsAg (primary) and HBeAg (secondary), animals were stratified and randomly divided into groups, with 5 animals per group.

Test Sample Preparation and Administration: The required dosage of drug powder was calculated based on the purity of each test sample. At a dosing concentration of 0.6 mg/mL, the corresponding saline solution was added and mixed by vortexing until a colorless, transparent liquid was obtained. The day of the first administration was defined as Day 0. All groups received a single subcutaneous dose of 5 mL/kg on Day 0. If blood sampling was required on the day of dosing, it was performed after blood collection.

Main Outcome Measures: Animals were weighed and blood was collected once on Days 3, 7, 14, 21, 28 and 35. Serum was separated for HBsAg and HBeAg level determination.

Serum HBsAg and HBeAg Level Determination: After blood collection, serum was separated, diluted with PBS, and submitted for analysis. From each sample, 10 µL of serum was removed and diluted to 500 µL (50-fold dilution) with PBS. Serum HBsAg and HBeAg levels were measured using a Hepatitis B e Antigen Assay Kit (Mike Biotech) and a Hepatitis B Surface Antigen Assay Kit (Mike Biotech), respectively.

### Experimental results of HBV-Tg transgenic mice

The inhibitory effect of the siRNA conjugates of the present invention on HBsAg in an HBV-Tg mouse model is shown in Figure 1.

The experimental results show that, compared with the vehicle control group, at a dose of 3 mg/kg, the test compound DAW30227 (i.e., double-stranded siRNA conjugate S146) exhibited significant inhibitory effects on HBsAg and also had a certain inhibitory effect on reducing HBeAg. In addition, the inhibitory activity of double-stranded siRNA conjugate S146 on HBsAg was significantly superior to that of the control group, DAW30025 (i.e., siRNA conjugate S149).

### 2. Hepatitis B virus AAV mouse experiment

### Test method:

Preparation and Grouping of rAAV8-1.3HBV/C57BL/6 Mouse Model: A persistent HBV infection mouse model was established by tail vein injection of rAAV8-1.3HBV (strain C) into C57 mice. The AAV virus injection dose was 1E+11 vg/mouse. Six weeks after injection, blood samples were collected for analysis of serum HBeAg, HBsAg and ALT levels. Based on the quantitative results of serum HBsAg (primary) and HBeAg (secondary), 35 mice were randomly divided into stratified groups, with 5 mice per group.

Test Sample Preparation and Administration: The required dosage of drug powder was calculated based on the purity of each test sample. At a dosing concentration of 0.6 mg/mL, the corresponding saline solution was added and mixed by vortexing until a colorless, transparent liquid was obtained. The day of the first administration was defined as Day 0. All groups received a single subcutaneous dose of 5 mL/kg on Day 0. If blood sampling was required on the day of dosing, it was performed after blood collection.
Main Outcome Measures: Mice were weighed and blood was collected once on Days 3, 7, 14, 21 and 28. Serum was separated for HBsAg and HBeAg level determination.

Serum HBsAg and HBeAg Level Determination: After blood collection, serum was separated, diluted with PBS, and submitted for analysis. From each sample, 10 µL of serum was removed and diluted to 500 µL (50-fold dilution) with PBS. Serum HBsAg and HBeAg levels were measured using a Hepatitis B e Antigen Assay Kit and a Hepatitis B Surface Antigen Assay Kit, respectively.

### Results of Hepatitis B Virus AAV Mouse Experiment

The inhibitory effect of the siRNA conjugates of the present invention on HBsAg in an Tg/HBV mouse model is shown in Figure 2.
The experimental results show that, compared with the vehicle control group, at a dose of 3 mg/kg, the test compound DAW30335 (i.e., siRNA conjugate S147) exhibited significant inhibitory effects on HBsAg and also had a certain inhibitory effect on reducing HBeAg. In addition, the inhibitory activity of siRNA conjugate S147 against HBsAg was significantly better than that of the controls DAW30025 (i.e., siRNA conjugate S149) and DAW30336 (i.e., siRNA conjugate S151).

### Example 7: Toxicity experiment of the conjugate of the present application in rats after subcutaneous injection

### Test method:

Rats were given the drug five times for a total of 29 days for clinical observation. Blood was collected from the abdominal aorta after anesthesia before dissection. The blood biochemistry indicators ALT, AST, ALP, CK, UREA, CREA, TP, ALB, GLU, TBIL, DBIL, CHO, TG, Na, K, Cl, A/G, GLB; hematology indicators WBC, RBC, HB, HCT, MCV, MCH, MCHC, RDW, PLT, MPV, WBC classification and percentage; and coagulation indicators PT, APTT, and FIB were tested. In addition, tissue virology was observed, including weighing of organs, aorta, esophagus, stomach, duodenum, cecum, colon, ileum, jejunum, rectum, lung (with main bronchi), mesenteric lymph nodes, pancreas, pituitary gland, eyeball, thyroid/parathyroid gland, salivary gland, prostate, bladder, seminal vesicle, femur, sternum, skin and muscle at the administration site (marked at the last administration), and grossly observed abnormal tissues.

Statistical method: SPSS16.0 was used for statistics. The mean and standard deviation of body weight, blood biochemistry, hematology, organ weight and coefficients of each group were calculated and statistically analyzed according to the following method: ① First, the data homogeneity was tested using the Levene's test method. If the data were homogeneous (test P>0.05), a one-way analysis of variance (ANOVA) test was performed; if the result of the Levene's test was significant (p≤0.05), a Kruskal-Wallis test was performed. ② If the ANOVA result was significant (p≤0.05), a multiple comparison test was performed using the Dunnett parametric test. If the ANOVA result was not significant (p>0.05), statistical analysis was terminated. ③ If the Kruskal-Wallis test was significant (p≤0.05), a rank-transformed Dunnett test was performed. If the Kruskal-Wallis test was not significant (p>0.05), statistical analysis was terminated. Clinical symptom observations, food intake, and anatomical observation data were not statistically processed.

Experimental conclusion: The experiment showed that clinical observation, blood biochemistry, hematology, organ weight and tissue pathology results overall showed that the siRNA conjugate of the present invention has good safety.

While the present invention has been described in detail above using general explanations, specific embodiments, and experiments, it will be readily apparent to those skilled in the art that modifications and improvements may be made based on the present invention. Accordingly, such modifications and improvements, as long as they do not depart from the spirit of the present invention, are intended to fall within the scope of protection claimed herein.

## Claims

1. A double-stranded siRNA, a conjugate or a salt thereof, wherein, the double-stranded siRNA comprises a sense strand and an antisense strand, and the double-stranded siRNA comprises one or more Y, the Y is 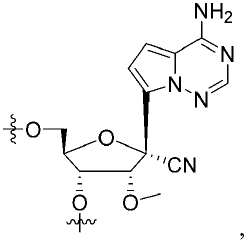 wherein each of nucleotides in the double-stranded siRNA and Y is independently modified or unmodified.

2. The double-stranded siRNA, conjugate or salt thereof according to claim 1, wherein, the antisense strand comprises a sequence of 5'-UGUGAAGCGAAGUGCACACUU-3' (SEQ ID NO:40), or a sequence in which one or more nucleotide residues are replaced by Y in the sequence of SEQ ID NO:40; wherein each of nucleotides and Y in the antisense strand is independently modified or unmodified, and the length of the antisense strand is not more than 23 nucleotides.

3. The double-stranded siRNA, conjugate or salt thereof according to claim 1 or 2, wherein, the sense strand comprises a sequence of 5'-GUGUGCACUUCGCUUCACA-3' (SEQ ID NO:20), or a sequence in which one or more nucleotide residues are replaced by Y in the sequence of SEQ ID NO:20; wherein each of nucleotides and Y in the sense strand is independently modified or unmodified, and the length of the sense strand is not more than 23 nucleotides.

4. The double-stranded siRNA, conjugate or salt thereof according to any one of claims 1-3, wherein, 70%, 75%, 80%, 85%, 90%, 95% or more of the nucleotides and Y in the sense strand or the antisense strand are modified; optionally, all nucleotides and Y in the double-stranded siRNA are modified.

5. The double-stranded siRNA, conjugate or salt thereof according to any one of claims 1-4, wherein, the modification is selected from at least one of the following: 2'-methoxy modification, 2'-methoxyethyl modification, 2'-fluoro modification, phosphorothioate linkage, 2'-deoxy modification, 2'-amino modification, locked nucleic acid modification, unlocked nucleic acid modification, ethylene glycol nucleic acid modification and 5'-vinyl phosphate ester modification.

6. The double-stranded siRNA, conjugate or salt thereof according to any one of claims 1-5, wherein, the sense strand comprises a sequence in which 0, 1, 2, 3, 4 or 5 nucleotide residues are replaced with Y in the sequence of SEQ ID NO:20, wherein each of nucleotides and Y in the sense strand is independently modified or unmodified.

7. The double-stranded siRNA, conjugate or salt thereof according to any one of claims 1-6, wherein, the antisense strand comprises a sequence in which 0, 1, 2, 3, 4 or 5 nucleotide residues are replaced with Y in the sequence of SEQ ID NO:40, wherein each of nucleotides and Y in the antisense strand is independently modified or unmodified.

8. The double-stranded siRNA, conjugate or salt thereof according to any one of claims 1-7, wherein, the Y substitution occurs at positions 1 to 19 from the 5' end of the sequence of SEQ ID NO: 20.

9. The double-stranded siRNA, conjugate or salt thereof according to any one of claims 1-8, wherein, the Y substitution occurs at positions 1 to 21 from the 5' end of the sequence of SEQ ID NO: 40.

10. The double-stranded siRNA, conjugate or salt thereof according to any one of claims 1-9, wherein, the sense strand comprises a sequence in which 0, 1, 2, 3, 4 or 5 nucleotide residues are substituted with Y in the sequence of 5'-g●u●guGcACUucgcuucaca-3' (SEQ ID NO: 19), wherein each of nucleotides and Y in the sense strand is independently modified or unmodified, and the Y substitution occurs at positions 1 to 19 from the 5' end of the sequence of SEQ ID NO: 19, and the length of the sense strand is not more than 23 nucleotides.

11. The double-stranded siRNA, conjugate or salt thereof according to any one of claims 1-10, wherein, the antisense strand comprises a sequence in which 0, 1, 2, 3, 4 or 5 nucleotide residues are substituted with Y in the sequence of 5'-u●G●ugaAgCGaaguGcAcac●u●u-3' (SEQ ID NO: 36), wherein each of nucleotides and Y in the antisense strand is independently modified or unmodified, and the Y substitution occurs at positions 1 to 21 from the 5' end of the sequence of SEQ ID NO: 36, and the length of the antisense strand is not more than 23 nucleotides.

12. The double-stranded siRNA, conjugate or salt thereof according to any one of claims 1-10, wherein, the antisense strand comprises a sequence in which 0, 1, 2, 3, 4 or 5 nucleotide residues are substituted with Y in the sequence of 5'-u●G●uga(Agn)gCGaaguGcAcac●u●u-3' (SEQ ID NO:33), wherein each of nucleotides and Y in the antisense strand is independently modified or unmodified, and the Y substitution occurs at positions 1 to 21 from the 5' end of the sequence of SEQ ID NO: 33, and the length of the antisense strand is not more than 23 nucleotides.

13. The double-stranded siRNA, conjugate or salt thereof according to any one of claims 1-12, the sense strand comprises one of the following nucleotide sequences:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19 and SEQ ID NO:21;
the length of the sense strand is not more than 23 nucleotides.

14. The double-stranded siRNA, conjugate or salt thereof according to any one of claims 1-13, the antisense strand comprises one of the following nucleotide sequences:
SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38 and SEQ ID NO:39;
the length of the sense strand is not more than 23 nucleotides.

15. The double-stranded siRNA, conjugate or salt thereof according to any one of claims 1-14, wherein, the double-stranded siRNA comprises one of the following:
A sense strand comprising the nucleotide sequence of SEQ ID NO:15 and an antisense strand comprising the nucleotide sequence of SEQ ID NO:25, wherein the sense strand and antisense strand are each independently not more than 23 nucleotides;
A sense strand comprising the nucleotide sequence of SEQ ID NO:17 and an antisense strand comprising the nucleotide sequence of SEQ ID NO:25, wherein the sense strand and antisense strand are each independently not more than 23 nucleotides;
A sense strand comprising the nucleotide sequence of SEQ ID NO:15 and an antisense strand comprising the nucleotide sequence of SEQ ID NO:26, wherein the sense strand and antisense strand are each independently not more than 23 nucleotides;
A sense strand comprising the nucleotide sequence of SEQ ID NO:16 and an antisense strand comprising the nucleotide sequence of SEQ ID NO:26, wherein the sense strand and antisense strand are each independently not more than 23 nucleotides;
A sense strand comprising the nucleotide sequence of SEQ ID NO:17 and an antisense strand comprising the nucleotide sequence of SEQ ID NO:26, wherein the sense strand and antisense strand are each independently not more than 23 nucleotides;
A sense strand comprising the nucleotide sequence of SEQ ID NO:15 and an antisense strand comprising the nucleotide sequence of SEQ ID NO:27, wherein the sense strand and antisense strand are each independently not more than 23 nucleotides;
A sense strand comprising the nucleotide sequence of SEQ ID NO:16 and an antisense strand comprising the nucleotide sequence of SEQ ID NO:27, wherein the sense strand and antisense strand are each independently not more than 23 nucleotides;
A sense strand comprising the nucleotide sequence of SEQ ID NO:17 and an antisense strand comprising the nucleotide sequence of SEQ ID NO:27, wherein the sense strand and antisense strand are each independently not more than 23 nucleotides;
A sense strand comprising the nucleotide sequence of SEQ ID NO:16 and an antisense strand comprising the nucleotide sequence of SEQ ID NO:28, wherein the sense strand and antisense strand are each independently not more than 23 nucleotides;
A sense strand comprising the nucleotide sequence of SEQ ID NO:17 and an antisense strand comprising the nucleotide sequence of SEQ ID NO:28, wherein the sense strand and antisense strand are each independently not more than 23 nucleotides; or
A sense strand comprising the nucleotide sequence of SEQ ID NO:18 and an antisense strand comprising the nucleotide sequence of SEQ ID NO:28, wherein the sense strand and antisense strand are each independently not more than 23 nucleotides.

16. The double-stranded siRNA, conjugate or salt thereof according to any one of claims 1-15, wherein, the double-stranded siRNA or conjugate thereof is selected from one of S1 to S143, S146, S147 and S150.

17. The double-stranded siRNA, conjugate or salt thereof according to any one of claims 1-16, wherein, the double-stranded siRNA conjugate is a conjugate of the double-stranded siRNA conjugated with a conjugation group L96 or DAW40007-4, and the structures of the conjugation groups L-96 and DAW40007-4 are respectively:

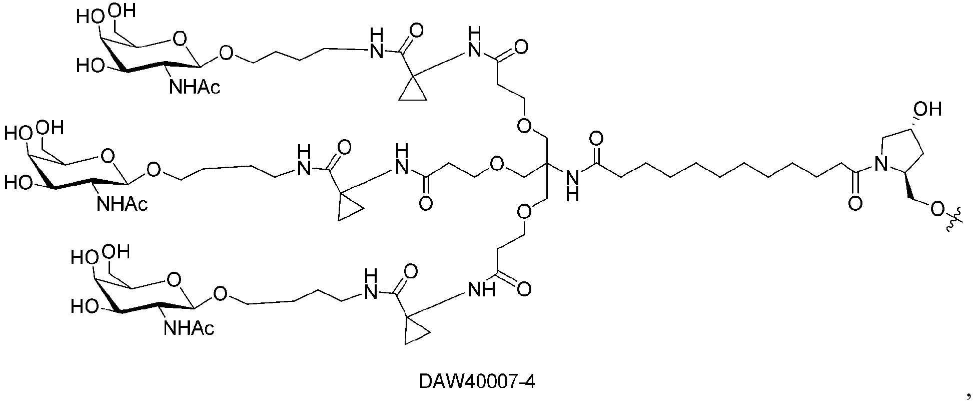

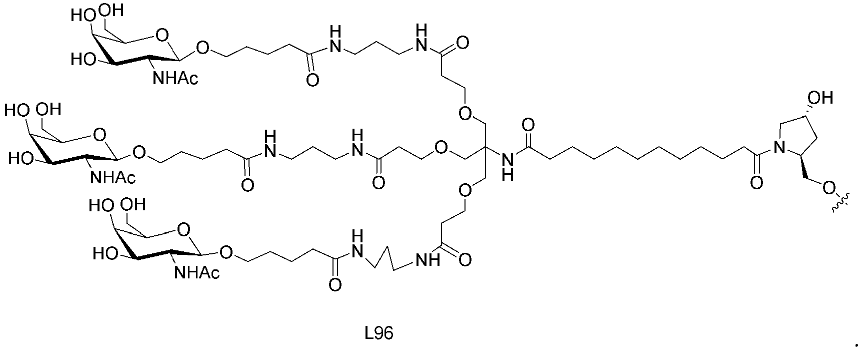

18. The double-stranded siRNA, conjugate or salt thereof according to claim 17, wherein, the conjugated group is conjugated to the 3' end or the 5' end of the sense strand of the double-stranded siRNA, preferably to the 3' end of the sense strand of the double-stranded siRNA.

19. The double-stranded siRNA, conjugate or salt thereof according to claim 17 or 18, wherein, the double-stranded siRNA is linked to the conjugated group through a phosphate eater group, phosphorothioate group or a phosphate group.

20. A nucleotide residue Y having structure:

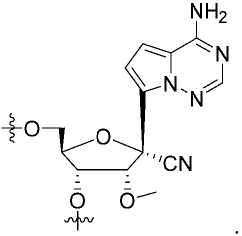

21. A compound having Formula (I) or a stereoisomer, a tautomer or an acceptable salt of the compound having Formula (I), 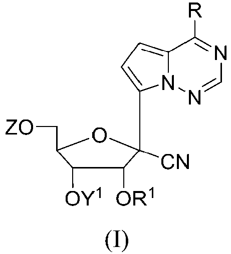
R¹ is methyl, ethyl, n-propyl, i-propyl, n-butyl, i-butyl or tert-butyl, each of the methyl, ethyl, n-propyl, i-propyl, n-butyl, i-butyl or tert-butyl is independently optionally substituted with 1, 2, 3 or 4 substituents selected from deuterium, hydroxyl, cyano, F, Cl, Br, I, methoxy, ethoxy, 1-propoxy and 2-propoxy;
Z and Y¹ are each independently H, deuterium, a hydroxyl protecting group, HOC(=O)(CH₂)ⱼC(=O)-, M-C(=O)(CH₂)ⱼC(=O)-, a phosphate ester group, a phosphorothioate group, a phosphoramidite group or a hydrogen phosphate group;
M is a solid support, preferably a hydroxyl- or amino-functionalized solid support, more preferably a resin or CPG, further preferably a macroporous resin or CPG, further preferably an aminomethyl resin, a hydroxyl resin or -NHCPG;
R is -NHR² or -N=CH-NR^{a}R^{b};
R² is an amino protecting group;
each of R^{a} and R^{b} is independently H or an amino protecting group, or R^{a}, R^{b} and the nitrogen atom to which they are attached form a heterocyclic group consisting of 5-6 ring atoms, each of heterocyclic group consisting of 5-6 ring atoms is independently optionally substituted with 1, 2, 3 or 4 substituents selected from deuterium, hydroxyl, cyano, F, Cl, Br, I, methyl, ethyl, n-propyl, isopropyl, methoxy, ethoxy, 1-propoxy and 2-propoxy;
wherein, each j is independently 1, 2, 3, 4 or 5.

22. The compound according to claim 21, wherein, Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₁₂ alkyl, C₁₋₁₂ alkylC(=O)-, C₁₋₁₂ alkylsilyl, C₆₋₁₀ arylC₁₋₆ alkyl, HOC(=O)(CH₂)ⱼC(=O)-, M-C(=O)(CH₂)ⱼC(=O)-, triphenylmethyl, MMTr, DMTr, 4' ,4' ,4'-trimethoxytrityl, 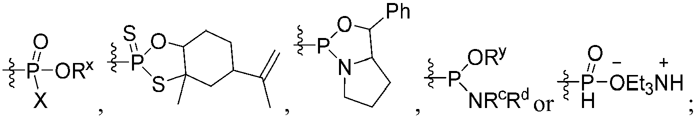
or
Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₁₀ alkyl, C₁₋₁₀ alkylC(=O)-, C₁₋₁₀ alkylsilyl, C₆₋₁₀ arylC₁₋₄ alkyl, HOC(=O)(CH₂)ⱼC(=O)-, M-C(=O)(CH₂)ⱼC(=O)-, triphenylmethyl, MMTr, DMTr, 4',4',4'-trimethoxytrityl, 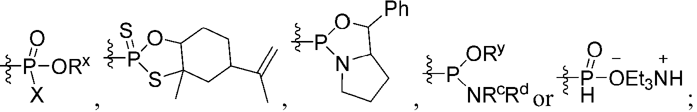 or
Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₈ alkyl, C₁₋₈ alkylC(=O)-, C₁₋₈ alkylsilyl, phenylC₁₋₃ alkyl, HOC(=O)(CH₂)ⱼC(=O)-, M-C(=O)(CH₂)ⱼC(=O)-, triphenylmethyl, MMTr, DMTr, 4',4',4'-trimethoxytrityl, 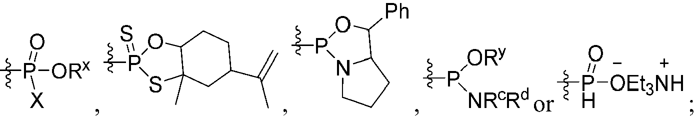 or
Z and Y¹ are each independently H, deuterium, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, C₁₋₆ alkyl, C₁₋₆ alkylC(=O)-, C₁₋₆ alkylsilyl, benzyl, phenylethyl, HOC(=O)(CH₂)ⱼC(=O)-, M-C(=O)(CH₂)ⱼC(=O)-, triphenylmethyl, MMTr, DMTr, 4',4',4'-trimethoxytrityl, 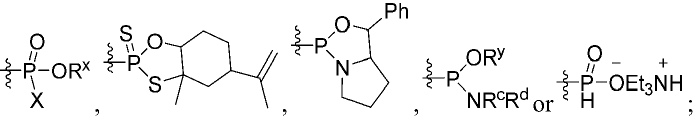
wherein, X is Cl or Br;
each of R^{x} and R^{y} is independently a hydroxy protecting group;
each of R^{c} and R^{d} is independently H or an amino protecting group; or R^{c}, R^{d} and the nitrogen atom to which they are attached form a heterocyclic group consisting of 5-6 ring atoms, the heterocyclic group consisting of 5-6 ring atoms is independently optionally substituted with 1, 2, 3 or 4 substituents selected from deuterium, hydroxyl, cyano, F, Cl, Br, I, methyl, ethyl, propyl, methoxy, ethoxy, 1-propoxy and 2-propoxy.

23. The compound according to claim 21 or 22, wherein, R² is 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, methyl, *tert-butyl,* 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, trimethylsilyl, triethylsilyl, triisopropylsilyl, C₁₋₆ alkoxy or R³C(=O)-;
each of R^{a} and R^{b} is independently H, methyl, ethyl, n-propyl, i-propyl, n-butyl, i-butyl, *tert-butyl,* methoxy, ethoxy, 1-propoxy, 2-propoxy, 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2-trimethylsilylethyl, 1,1-dimethyl-2-propenyl, 3-methyl-3-butenyl, allyl, benzyl, *p*-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl or R⁴C(=O)-, or R^{a}, R^{b} and the N atom to which they are attached form a pyrrolidinyl, morpholinyl, piperidinyl or piperazinyl, each of pyrrolidinyl, morpholinyl, piperidinyl and piperazinyl is independently optionally substituted with 1, 2, 3 or 4 substituents selected from deuterium, hydroxyl, cyano, F, Cl, Br, I, methoxy, ethoxy, 1-propoxy and 2-propoxy;
wherein, each of R³ and R⁴ is independently C₁₋₆ alkyl, halo C₁₋₆ alkyl, C₁₋₆ alkoxy, phenyl, halo-substituted phenyl, C₁₋₆ alkylphenyl or benzyl; or
each of R³ and R⁴ is independently C₁₋₄ alkyl, halo C₁₋₄ alkyl, C₁₋₄ alkoxy, phenyl, halo-substituted phenyl, C₁₋₄ alkylphenyl or benzyl; or
each of R³ and R⁴ is independently methyl, ethyl, n-propyl, isopropyl, trifluoromethyl, difluoromethyl, monofluoromethyl, trichloromethyl, dichloromethyl, monochloromethyl, 2,2,2-trichloroethyl, methoxy, ethoxy, 1-propoxy, 2-propoxy, 1-butoxy, 2-methyl-1-propoxy, 2-butoxy, *tert*-butoxy, phenyl, halogen-substituted phenyl, C₁₋₃ alkylphenyl or benzyl.

24. The compound according to any one of claims 22-23, each of R^{x} and R^{y} is independently benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, cyano C₁₋₆ alkyl, C₁₋₆ alkyl, C₁₋₆ alkyl C(=O)-, C₁₋₆ alkylsilyl, phenyl, halogen-substituted phenyl, benzyl or phenethyl; or
each of R^{x} and R^{y} is independently benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2,2,2-trichloroethyl, 2-trimethylsilylethyl, 1,1-dimethyl-2-propenyl, 3-methyl-3-butenyl, allyl, p-methoxybenzyldiphenylmethyl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, p-toluenesulfonyl, CNCH₂-, CN(CH₂)₂-, CNCH(CH₃)₂CH₂-, CN(CH₂)₃-, CNCH(CH₃)₂CH₂CH₂-, methyl, ethyl, *n*-propyl, *i*-propyl, *n*-butyl, *i*-butyl, *tert*-butyl, C₁₋₄ alkyl C(=O)-, C₁₋₄ alkylsilyl, phenyl, halogen-substituted phenyl, benzyl or phenethyl;
each of R^{c} and R^{d} is independently H, methyl, ethyl, *n*-propyl, *i*-propyl, *n*-butyl, *i*-butyl, *tert*-butyl*,* methoxy, ethoxy, 1-propoxy, 2-propoxy, 9-fluorenylmethoxycarbonyl, benzyloxycarbonyl, 4-nitrobenzyloxycarbonyl, 4-bromobenzyloxycarbonyl, 4-methoxybenzyloxycarbonyl, biphenylmethoxycarbonyl, 2,2,2-trichloroethoxycarbonyl, 2-(trimethylsilyl)ethoxycarbonyl, allyloxycarbonyl, chloroacetyl, trifluoroacetyl, methoxyacetyl, phenoxyacetyl, benzoyl, 2-trimethylsilylethyl, 1,1-dimethyl-2-propenyl, 3-methyl-3-butenyl, allyl, benzyl, p-methoxybenzyldiphenylmethyl, trityl, tetrahydrofuranyl, methoxymethyl, methylthiomethyl, benzyloxymethyl, 2,2,2-trichloroethoxymethyl, 2-(trimethylsilyl)ethoxymethyl, methanesulfonyl, *p*-toluenesulfonyl, trimethylsilyl, triethylsilyl, triisopropylsilyl or R⁴C(=O)-, or R^{c}, R^{d} and the N atom to which they are attached form a pyrrolidinyl, morpholinyl, piperidinyl or piperazinyl, each of pyrrolidinyl, morpholinyl, piperidinyl and piperazinyl is independently optionally substituted with 1, 2, 3 or 4 substituents selected from deuterium, hydroxyl, cyano, F, Cl, Br, I, methoxy, ethoxy, 1-propoxy and 2-propoxy.

25. The compound according to any one of claims 21-24 having a structure as shown in formula (II),

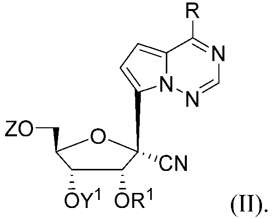

26. A pharmaceutical composition comprising the double-stranded siRNA, conjugate or salt thereof according to any one of claims 1-19, and a pharmaceutically acceptable carrier.

27. Use of the double-stranded siRNA conjugate, conjugate and salt thereof according to any one of claims 1-19, or the pharmaceutical composition according to claim 26 in the preparation of a medicament for treating and/or preventing hepatitis B.

28. Use of the compound according to any one of claims 21-25 as a raw material for solid-phase synthesis of DNA nucleotides, a raw material for the synthesis of oligonucleotide drugs, a raw material for the synthesis of siRNA drugs, siRNA drug research, gene function research, and/or screening of whole gene libraries.

29. Use of the nucleotide residue according to claim 20 as an oligonucleotide embedding group, wherein, the oligonucleotide is a nucleotide sequence containing 10~50 nucleotides or nucleotide base pairs, and the oligonucleotide can inhibit or block gene expression.

30. The use according to claim 29, wherein, the gene is an HBV gene.

31. The use according to claim 29 or 30, wherein, the oligonucleotide is siRNA.

32. The use according to claim 31, wherein, the siRNA comprises the sense strand and the antisense strand.

33. The use according to claim 32, wherein, the nucleotide residues are embedded only into the sense strand of the siRNA.

34. The use according to claim 32 or 33, wherein, the nucleotide residues are embedded only into the antisense strand of the siRNA.

35. The use according to any one of claims 32-34, wherein, the nucleotide residues are embedded into the sense strand and the antisense strand of the siRNA.

36. The use according to any one of claims 32-35, wherein, the sense strand of the siRNA comprises the sequence of SEQ ID NO: 19.

37. The use according to any one of claims 32-36, wherein, the antisense strand of the siRNA comprises the sequence of SEQ ID NO:36 or SEQ ID NO:33.

38. The double-stranded siRNA conjugate, conjugate and salt thereof according to any one of claims 1-19, or the pharmaceutical composition according to claim 26 for use in treating and/or preventing hepatitis B.

39. The compound according to any one of claims 21-25 is used as a raw material for solid-phase synthesis of DNA nucleotides, a raw material for the synthesis of oligonucleotide drugs, a raw material for the synthesis of siRNA drugs, siRNA drug research, gene function research, and/or screening of whole gene libraries.

40. The nucleotide residue according to claim 20 is used as an oligonucleotide embedding group, wherein, the oligonucleotide is a nucleotide sequence containing 10 to 50 nucleotides or nucleotide base pairs, and the oligonucleotide can inhibit or block gene expression.

41. The nucleotide residue according to claim 40, wherein, the gene is an HBV gene.

42. The nucleotide residue according to claim 40 or 41, wherein, the oligonucleotide is siRNA.

43. The nucleotide residue according to claim 42, wherein, the siRNA comprises the sense strand and the antisense strand.

44. The nucleotide residue according to claim 43, wherein, the nucleotide residues are embedded only into the sense strand of the siRNA.

45. The nucleotide residue according to claim 43 or 44, wherein, the nucleotide residues are embedded only into the antisense strand of the siRNA.

46. The nucleotide residue according to any one of claims 43-45, wherein, the nucleotide residues are embedded into the sense strand and the antisense strand of the siRNA.

47. The nucleotide residue according to any one of claims 43-46, wherein, the sense strand of the siRNA comprises the sequence of SEQ ID NO:19.

48. The nucleotide residue according to any one of claims 43-47, wherein, the antisense strand of the siRNA comprises the sequence of SEQ ID NO:36 or SEQ ID NO:33.

49. A method for treating and/or preventing hepatitis B, comprising using the double-stranded siRNA conjugate, conjugate and salt thereof according to any one of claims 1-19, or the pharmaceutical composition according to claim 26.

50. A method for solid-phase synthesis of DNA nucleotides, synthesis of oligonucleotide drugs, synthesis of siRNA drugs, siRNA drug research, gene function research and/or screening of a whole gene library, the method comprises using the compound according to any one of claims 21-25.

51. A method for inhibiting or blocking gene expression, comprising using a nucleotide sequence comprising 10 to 50 nucleotides or nucleotide base pairs, wherein the nucleotide sequence has embedded the nucleotide residues according to claim 20.

52. The method according to claim 51, wherein, the gene is an HBV gene.

53. The method according to claim 51 or 52, wherein, the nucleotide sequence is siRNA.

54. The method according to claim 53, wherein, the siRNA comprises the sense strand and the antisense strand.

55. The method according to claim 54, wherein, the nucleotide residues are embedded only into the sense strand of the siRNA.

56. The method according to claim 54 or 55, wherein, the nucleotide residues are embedded only into the antisense strand of the siRNA.

57. The method according to any one of claims 54-56, wherein, the nucleotide residues are embedded into the sense strand and the antisense strand of the siRNA.

58. The method according to any one of claims 54-57, wherein, the sense strand of the siRNA comprises the sequence of SEQ ID NO: 19.

59. The method according to any one of claims 54-58, wherein, the antisense strand of the siRNA comprises the sequence of SEQ ID NO:36 or SEQ ID NO:33.
